# EUROPEAN PATENT SPECIFICATION

(11) **EP 3 694 489 B1**
(45) Date of publication and mention of the grant of the patent: **02.12.2026**
(21) Application number: 18865817.3
(22) Date of filing: 11.10.2018
(51) Int. Cl.: A61K 9/06, A61K 9/70, A61K 38/00, A61K 38/39, A61P 1/02, A61P 1/04, A61P 13/02, A61K 9/00, A61K 47/36, A61K 47/42, A61K 8/368, A61K 8/42, A61K 8/44, A61K 8/64, A61K 31/167, A61K 31/195, A61K 31/573, A61Q 15/00, A61Q 19/08

(54) **METHODS AND COMPOSITIONS FOR TOPICAL DELIVERY**
VERFAHREN UND ZUSAMMENSETZUNG ZUR TOPISCHEN VERABREICHUNG
PROCÉDÉS ET COMPOSITIONS À ADMINISTRATION TOPIQUE

(30) Priority: 11.10.2017 US 201762571025 P; 11.10.2017 US 201762571038 P; 11.10.2017 US 201762571049 P; 14.12.2017 US 201762598786 P; 14.12.2017 US 201762598796 P; 14.12.2017 US 201762598828 P
(43) Date of publication of application: 19.08.2020
(73) Proprietor: Illustris Pharmaceuticals, Inc., Palo Alto, California 94030 (US)
(72) Inventor: WAUGH, Jacob M., Corona Del Mar California 92625 (US); DAKE, Michael D., Palo Alto California 94301 (US)
(74) Representative: HGF
(86) International application number: PCT/US2018/055499
(87) International publication number: WO 2019/075263

(56) References cited:
- WO-A1-2017/180788
- WO-A2-2014/044808
- WO-A2-2014/044808
- US-A- 5 646 129
- US-A1- 2009 220 415
- US-A1- 2011 014 241
- US-A1- 2016 000 834
- US-A1- 2016 324 934
- US-A1- 2017 100 523
- US-A1- 2017 266 267
- US-A1- 2017 290 778
- US-B2- 8 916 539
- ESSENDOUBI M. ET AL: "Human skin penetration of hyaluronic acid of different molecular weights as probed by Raman spectroscopy", SKIN RESEARCH AND TECHNOLOGY, vol. 22, no. 1, 1 February 2016 (2016-02-01), DK, pages 55 - 62, XP055804719, ISSN: 0909-752X, DOI: 10.1111/srt.12228

## Description

### CROSS REFERENCE

This application claims priority to U.S. Provisional Application No. 62/571,025, filed on October 11, 2017; U.S. Provisional Application No. 62/571,038, filed on October 11, 2017; U.S. Provisional Application No. 62/598,796, filed on December 14, 2017; U.S. Provisional Application No. 62/571,049, filed on October 11, 2017; U.S. Provisional Application No. 62/598,786, filed on December 14, 2017; and U.S. Provisional Application No. 62/598,828, filed on December 14, 2017.

### BACKGROUND

US8916539B2 discloses a method of preventing or treating a dermatologic condition including, inter alia, psoriasis, contact dermatitis, and atopic dermatitis, in a subject, the method includes the step of administering to said subject a compound comprising a lipid or phospholipid moiety bound to a physiologically acceptable monomer, dimer, oligomer, or polymer, and/or a pharmaceutically acceptable salt or a pharmaceutical product thereof, in an amount effective to treat the subject suffering from a dermatologic condition.

### BRIEF SUMMARY

A topical route of drug administration is desirable because the risks and inconvenience of parenteral treatment can be avoided; the variable absorption and metabolism associated with oral treatment can be circumvented; drug administration can be continuous, thereby permitting the use of pharmacologically active agents with short biological half-lives; the gastrointestinal irritation associated with many compounds can be avoided; and cutaneous manifestations of diseases can be treated more effectively than by systemic approaches. Additionally, higher tissue concentrations can be achieved in the effected areas with a topical verses a systemic administration of an active ingredient if desired. Most transdermal and transmucosal delivery systems achieve penetration by using a penetration-enhancing vehicle or agents. Such compounds or mixtures of compounds are known in the art as "penetration enhancers" or "skin enhancers." Many of the penetration enhancers in the literature enhance transdermal absorption, yet they also possess certain drawbacks in that some are regarded as toxic; some irritate the skin; some have a thinning effect on the skin on prolonged use; and most are incapable of delivering high molecular weight pharmaceuticals and cosmetic agents. Clearly, there remains a need for safe and effective transdermal delivery compositions and systems that can administer a wide-range of pharmaceuticals and cosmetic agents to and through the skin, mucosa, hair, nails, teeth, bone, and various other surfaces without the use of standard penetration enhancers known in the art.

Various embodiments of the invention are directed to compositions comprising a mixture of two components dissolved, suspended, dispersed or otherwise mixed in a carrier or vehicle, the first component consisting of a decoy molecule consisting of an extracellular matrix (ECM) component and/or fragment thereof, and the second component comprised of one or more active agents, wherein the extracellular matrix component or fragment thereof has an average molecular weight of about 2,000 daltons to about 30,000 daltons and is present in the composition at between about 0.001 wt. % to about 10 wt. %.

Further embodiments include compositions for use in therapy comprising a mixture of two components dissolved, suspended, dispersed or otherwise mixed in a carrier or vehicle, the first component consisting of a decoy molecule consisting of an extracellular matrix (ECM) component and/or fragment thereof, and the second component comprised of one or more active agents, wherein the extracellular matrix component or fragment thereof has an average molecular weight of about 2,000 daltons to about 30,000 daltons and is present in the composition at between about 0.001 wt. % to about 10 wt. %..

In one embodiment, a composition a plurality of therapeutic cells expressing a tumor antigen; and a decoy molecule consisting of an ECM component and/or a fragment thereof present in the composition at between about 0.1 wt. % to about 10 wt. %, wherein said composition is for separate or simultaneous use in the treatment of cancer, and wherein the ECM component and/or fragment thereof has an average molecular weight of about 2,000 daltons to about 30,000 daltons is provided.

In another embodiment, the composition is for use in a method of delivering therapeutic cells to a tissue surface comprises topically administering a composition comprising a plurality of therapeutic cells and a decoy molecule selected from an extracellular matrix component, fragments thereof and combinations thereof is provided. In some embodiments, a composition comprising therapeutic cells and a composition comprising decoy molecules are administered separately.

In additional embodiment, the composition is for use in a method of eliciting an immune response in a subject comprises topically administering to a tissue surface a composition comprising a plurality of cells expressing an antigen and a decoy molecule selected from an extracellular matrix component, fragments thereof and combinations thereof is provided. In some embodiments, a composition comprising cells expressing an antigen and a composition comprising decoy molecules are administered separately.

Some embodiments are directed to compositions for use in a method of treating scars and wrinkles on a skin surface comprising topically administering a composition comprising a plurality of therapeutic cells and a decoy molecule selected from an extracellular matrix component, fragments thereof and combinations thereof. In some embodiments, a composition comprising therapeutic cells and a composition comprising decoy molecules are administered separately.

Also disclosed herein are compositions for use and compositions that may be used to deliver an active agent via a transdermal patch. In one embodiment, a transdermal patch comprises a composition comprising one or more active agents and a decoy molecule selected from an extracellular matrix component, fragments thereof and combinations thereof.

In another embodiment, a composition for use in a method of detecting an antigen noninvasively involves applying to a tissue surface a transdermal patch, wherein the transdermal patch comprises a composition comprising one or more active agents and a decoy molecule selected from an extracellular matrix component, fragments thereof and combinations thereof is provided.

In another embodiment, a composition for use in a method of detecting an analyte in a body fluid comprises applying a transdermal patch to a tissue surface to capture the body fluid, wherein the transdermal patch comprises a composition comprising one or more active agents and a decoy molecule selected from an extracellular matrix component, fragments thereof and combinations thereof is provided.

Embodiments of the invention are directed to compositions comprising a mixture of two components dissolved, suspended, dispersed or otherwise mixed in a carrier or vehicle, the first component consisting of a decoy molecule consisting of an ECM component and/or a fragment thereof and the second component comprising a neurotoxic agent, wherein the decoy molecule is present in the composition at between about 0.001 wt.% to about 10 wt.%, and wherein the composition is for use as claimed in 13, and wherein said neurotoxin agent is selected from the group consisting of botulinum toxin type A, botulinum toxin type B, botulinum toxin type C, botulinum toxin type D, botulinum toxin type E, botulinum toxin type F, abobotulinumtoxinA, BTX-A, daxibotulinumtoxinA, incobotulinumtoxinA, onabotulinumtoxinA, rimabotulinumtoxinB, syntaxin derivatives thereof, pharmaceutically acceptable salts thereof, and combinations thereof.

Embodiments of the invention are directed to compositions for use in methods of topically administering a neurotoxin agent to a subject in need thereof a composition comprising an effective amount of a neurotoxin agent and a decoy molecule selected from an extracellular matrix component, fragments thereof and combinations thereof. In some embodiments, a composition comprising neurotoxin agent and a composition comprising decoy molecules are administered separately.

Embodiments of the invention are also directed to compositions for use in methods of treating, reducing or improving the look of frown lines (e.g., glabellar lines), wrinkles or crow's feet lines in a subject in need thereof comprising topically administering to a surface tissue of the subject a composition comprising an effective amount of a neurotoxin agent and a decoy molecule selected from an extracellular matrix component, fragments thereof and combinations thereof. In some embodiments, a composition comprising neurotoxin agent and a composition comprising decoy molecules are administered separately.

Embodiments of the invention are directed to compositions for use in methods of treating the symptoms of sweating in a subject in need thereof comprising topically administering to a surface tissue of the subject a composition comprising an effective amount of a neurotoxin agent and a decoy molecule selected from an extracellular matrix component, fragments thereof and combinations thereof. In some embodiments, a composition comprising neurotoxin agent and a composition comprising decoy molecules are administered separately.

Embodiments of the invention are directed to compositions for use in methods of treating migraines in a subject in need thereof comprising topically administering to a surface tissue of the subject a composition comprising an effective amount of a neurotoxin agent and a decoy molecule selected from an extracellular matrix component, fragments thereof and combinations thereof. In some embodiments, a composition comprising neurotoxin agent and a composition comprising decoy molecules are administered separately.

Embodiments of the invention are directed to compositions for use in a method of treating a condition in a subject in need thereof by topically administering to the subject an effective amount of one or more active agents and an enzyme selected from hyaluronidase, elastase, or a combination thereof. In some embodiments, the composition further comprises a decoy molecule selected from an extracellular matrix component, fragments thereof and combinations thereof. In some embodiments, a composition comprising active agents and a composition comprising hyaluronidase/elastase are administered separately.

Embodiments of the invention are directed to a composition for use in method of treating or reducing scars and wrinkles on a skin surface in a subject in need thereof by topically administering to the skin surface a composition that comprises an effective amount of one or more active agents and an enzyme selected from hyaluronidase, elastase, or a combination thereof. In some embodiments, the composition further comprises a decoy molecule selected from an extracellular matrix component, fragments thereof and combinations thereof. In some embodiments, a composition comprising active agents and a composition comprising hyaluronidase/elastase are administered separately.

Embodiments of the invention are directed to a composition for use in method of treating hair loss in a subject in need thereof by topically administering to the subject a composition that comprises an effective amount of one or more active agents and an enzyme selected from hyaluronidase, elastase, or a combination thereof. In some embodiments, the composition further comprises a decoy molecule selected from an extracellular matrix component, fragments thereof and combinations thereof. In some embodiments, a composition comprising active agents and a composition comprising hyaluronidase/elastase are administered separately.

Embodiments of the invention are directed to a composition for use in method of treating a skin condition in a subject in need thereof by topically administering to the subject a composition that comprises an effective amount of one or more active agents and an enzyme selected from hyaluronidase, elastase, or a combination thereof. In some embodiments, the composition further comprises a decoy molecule selected from an extracellular matrix component, fragments thereof and combinations thereof. In some embodiments, a composition comprising active agents and a composition comprising hyaluronidase/elastase are administered separately.

### BRIEF DESCRIPTION OF THE DRAWINGS

For a fuller understanding of the nature and advantages of the present invention, reference should be made to the following detailed description taken in connection with the accompanying drawings, in which:
**FIGS. 1A-1B** are graphs showing the percent of peptide flux relative to flux of peptide from the composition of peptide alone, for peptide compositions comprising a decoy molecule of hyaluronic acid with a molecular weight of 10,000 Da, 20,000 Da, 40,000 Da, 60,000 Da, or 100,000 Da, where flux was measured in skin with stratum corneum intact (FIG. 1A) and in skin with stratum corneum stripped (FIG. 1B) and each composition was measured in duplicate (solid line, dashed line).
**FIG. 2** is a bar graph showing the percent increase of salicylate flux from compositions of salicylate and a decoy molecule of hyaluronic acid with molecular weights designated as small (5,000 Da to 10,000 Da), small to mid (10,000 Da to 20,000 Da), low to mid (20,000 Da to 30,000 Da), and mid (30,000 Da to 40,000 Da) over a composition with no decoy molecule.
**FIG. 3** is a bar graph showing the percent increase of hydrocortisone flux from compositions of hydrocortisone and a decoy molecule of hyaluronic acid with molecular weights designated as very small (5,000 Da to 10,000 Da), small (10,000 Da to 20,000 Da), mid (30,000 Da to 40,000 Da), and large (40,000 Da to 60,000 Da) over a composition with no decoy molecule.
**FIG. 4** is a bar graph showing the percent of lidocaine in porcine skin from topically applied compositions of lidocaine and an elastin decoy molecule with a molecular weight designated as very very small (2,000 Da to 5,000 Da), very small (5,000 Da to 10,000 Da), and small (10,000 Da to 20,000 Da) and no decoy molecule.
**FIG. 5** is a bar graph showing the percent of topically applied minocycline in porcine skin from compositions containing of minocycline and a decoy molecule of hyaluronic acid with molecular weights designated as 3,000 Da, 5,000 Da, and 10,000 Da compared with a composition with no decoy.
**FIG. 6** is a bar graph showing the absorption of UVA and UVB in skin (4.0 corresponds to 100%), where the bars correspond with a sunscreen composition with a decoy molecule added to the commercially available sunscreen (Anthelios 60) and the commercially available sunscreen (Anthelios 60).
**FIGS. 7A-7B** are graphs of UV absorption as a function of wavelength, in nm, for commercially available sunscreen (Anthelios 60) alone (FIG. 7A) and for the commercially available sunscreen (Anthelios 60) with a decoy molecule (FIG. 7B).
**FIG. 8** is a graph showing the percent UV absorbance through skin as a function of wavelength, in nm, for commercially available sunscreen (Anthelios 60) (solid line) and for the commercially available sunscreen (Anthelios 60) with a decoy molecule (dashed line).
**FIG. 9** is a bar graph showing the amount of gabapentin in tissue (µg gabapentin/g tissue) delivered into porcine skin grafts in vitro from a topical formulation of gabapentin and sodium hyaluronate and from a topical formulation of gabapentin alone.
**FIG. 10** is a bar graph showing the amount of palmitoyl-lysine-threonine-threonine-lysine-serine (pal-KTTKS) in tissue (µg pal-KTTKS/50 mg tissue) delivered into porcine skin grafts in vitro from a topical formulation of pal-KTTKS and sodium hyaluronate and from a topical formulation of pal-KTTKS alone.
**FIG. 11** is a bar graph showing the percent increase in salicylate delivery across porcine mucosal tissue when a decoy molecule of elastin is included in the composition compared with a composition of salicylate and saline.
**FIG. 12** is bar graph showing the percentage increase of antibody flux from compositions comprised of antibody and a decoy molecule of hyaluronic acid with molecular weights designated as vvlow, vlow and low compared with antibody alone.
**FIG. 13** shows confocal imaging of FITC-dextran applied to glabellar region in the presence of a decoy molecule.
**FIG. 14** shows penetration of FITC-dextran over time in the glabellar region by confocal imaging.
**FIG. 15** shows penetration of FITC-dextran in the presence of various decoy molecules ILS-20 (A) and ILS-3 (B) in the lateral canthus region after 3 hrs.
**FIG. 16** depicts the topical application of botox at baseline.
**FIG. 17** depicts the effect at week 1 and week 8 on armpit sweat of topically applied botox + ILS-20 in accordance with embodiments described herein.
**FIG. 18** depicts the effect at week 1 and week 8 on armpit sweat of topically applied botox + ILS-3 in accordance with embodiments described herein.

### DETAILED DESCRIPTION

Various aspects now will be described more fully hereinafter. Such aspects may, however, be embodied in many different forms and should not be construed as limited to the embodiments set forth herein; rather, these embodiments are provided so that this disclosure will be thorough and complete, and will fully convey its scope to those skilled in the art.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also explicitly disclosed, as well as the range of values greater than or equal to 1 µm and the range of values less than or equal to 8 µm.

The singular forms "a," "an," and "the" include plural referents unless the context clearly dictates otherwise. Thus, for example, reference to a "polymer" includes a single polymer as well as two or more of the same or different polymers; reference to an "excipient" includes a single excipient as well as two or more of the same or different excipients, and the like.

All percentages, parts and ratios are based upon the total weight of the topical compositions and all measurements made are at about 25 °C, unless otherwise specified.

The word "about" when immediately preceding a numerical value means a range of plus or minus 10% of that value, e.g, "about 50" means 45 to 55, "about 25,000" means 22,500 to 27,500, etc, unless the context of the disclosure indicates otherwise, or is inconsistent with such an interpretation. For example in a list of numerical values such as "about 49, about 50, about 55, "about 50" means a range extending to less than half the interval(s) between the preceding and subsequent values, e.g, more than 49.5 to less than 52.5. Furthermore, the phrases "less than about" a value or "greater than about" a value should be understood in view of the definition of the term "about" provided herein.

The terms "administer," "administering" or "administration" as used herein refer to either directly administering a compound (also referred to as an agent of interest) or pharmaceutically acceptable salt of the compound (agent of interest) or a composition to a subject.

The term "antigen" can be a peptide, a polypeptide, a protein, a glycoprotein, a lipoprotein, a lipid, a phospholipid, a carbohydrate, a glycolipid, a mixture or a conjugate thereof, or any other material known to induce an immune response. The molecular weight of the antigen may be greater than 1 kilodalton (kDa), 10 kDa or 100 kDa (including intermediate ranges thereof). An antigen can be conjugated to a carrier. An antigen can be provided as a whole organism such as, for example, a bacterium or virion; an antigen can be obtained from an extract or lysate of organisms, e.g., from whole cells or from membranes; an antigen can be provided as live organisms such as, for example, live viruses or bacteria, attenuated live organisms such as, for example, attenuated live viruses or bacteria, or organisms that have been inactivated by chemical or genetic techniques; and an antigen can be chemically synthesized, produced by recombinant technology or purified from natural sources.

The term "antibody fragments" can include any derivative of an antibody which is less than full-length. In exemplary embodiments, the antibody fragment retains at least a significant portion of the full-length antibody's specific binding ability. Examples of antibody fragments include, but are not limited to, Fab, Fab', F(ab') , scFv, Fv, diabody, tribody, tetrabody, Fd fragments, or mixtures thereof. The antibody fragment may be produced by any means. For instance, the antibody fragment may be enzymatically or chemically produced by fragmentation of an intact antibody, it may be recombinantly produced from a gene encoding the partial antibody sequence, or it may be wholly or partially synthetically produced. The antibody fragment may optionally be a single chain antibody fragment. Alternatively, the fragment may comprise multiple chains which are linked together, for instance, by disulfide linkages. The fragment may also optionally be a multimolecular complex.

The transitional term "comprising," which is synonymous with "including," "containing," or "characterized by," is inclusive or open-ended and does not exclude additional, unrecited elements or method steps. By contrast, the transitional phrase "consisting of" excludes any element, step, or ingredient not specified in the claim. The transitional phrase "consisting essentially of" limits the scope of a claim to the specified materials or steps "and those that do not materially affect the basic and novel characteristic(s)" of the claimed invention. In embodiments or claims where the term comprising is used as the transition phrase, such embodiments can also be envisioned with replacement of the term "comprising" with the terms "consisting of" or "consisting essentially of." The compositions and methods of the present disclosure can comprise, consist essentially of, or consist of, the components disclosed.

The term "carrier" as used herein encompasses carriers, excipients, and diluents, meaning a material, composition or vehicle, such as a liquid or solid filler, diluent, excipient, solvent or encapsulating material involved in carrying or transporting a pharmaceutical, cosmetic or other agent across a tissue layer such as the stratum corneum or stratum spinosum.

The term "disorder" is used in this disclosure to mean, and is used interchangeably with, the terms disease, condition, or illness, unless otherwise indicated.

The term "decoy molecule" as used herein is interchangeable with an extracellular matrix component, fragments thereof and combinations thereof as described herein.

The terms "effective amount" and "therapeutically effective amount" are used interchangeably in this disclosure and refer to an amount of a composition, compound, or an active agent that, when administered to a subject, is capable of reducing a symptom of a disorder in a subject or enhance the texture, appearance, color, sensation, or hydration of the intended tissue treatment area. The actual amount which comprises the "effective amount" or "therapeutically effective amount" will vary depending on a number of conditions including, but not limited to, the severity of the disorder, the size and health of the patient, and the route of administration. A skilled medical practitioner can readily determine the appropriate amount using methods known in the medical arts.

The phrase "pharmaceutically acceptable" is employed herein to refer to those agents of interest/compounds, salts, compositions, dosage forms, etc, which are--within the scope of sound medical judgment--suitable for use in contact with the tissues of human beings and/or other mammals without excessive toxicity, irritation, allergic response, or other problem or complication, commensurate with a reasonable benefit/risk ratio. In some aspects, "pharmaceutically acceptable" means approved by a regulatory agency of the federal or a state government, or listed in the U.S. Pharmacopeia or other generally recognized pharmacopeia for use in mammals (e.g, animals), and more particularly, in humans.

The term "salts" as used herein embraces pharmaceutically acceptable salts commonly used to form alkali metal salts of free acids and to form addition salts of free bases. The nature of the salt is not critical, provided that it is pharmaceutically acceptable. The term "salts" also includes solvates of addition salts, such as hydrates, as well as polymorphs of addition salts. Suitable pharmaceutically acceptable acid addition salts can be prepared from an inorganic acid or from an organic acid. Non-limiting examples of such inorganic acids are hydrochloric, hydrobromic, hydroiodic, nitric, carbonic, sulfuric, and phosphoric acid. Appropriate organic acids can be selected from aliphatic, cycloaliphatic, aromatic, arylaliphatic, and heterocyclyl containing carboxylic acids and sulfonic acids, for example formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, stearic, salicylic, p-hydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, cyclohexylaminosulfonic, algenic, 3-hydroxybutyric, galactaric and galacturonic acid.

The term "patient" and "subject" are interchangeable and may be taken to mean any living organism which may be treated with compounds of the present invention. As such, the terms "patient" and "subject" may include, but is not limited to, any non-human mammal, primate or human. In some embodiments, the "patient" or "subject" is a mammal, such as mice, rats, other rodents, rabbits, dogs, cats, swine, cattle, sheep, horses, primates, or humans. In some embodiments, the patient or subject is an adult, child or infant. In some embodiments, the patient or subject is a human.

The term "treating" is used herein, for instance, in reference to methods of treating a skin disorder or a systemic condition, and generally includes the administration of a compound or composition which reduces the frequency of, or delays the onset of, symptoms of a medical condition or enhance the texture, appearance, color, sensation, or hydration of the intended tissue treatment area of the tissue surface in a subject relative to a subject not receiving the compound or composition. This can include reversing, reducing, or arresting the symptoms, clinical signs, and underlying pathology of a condition in a manner to improve or stabilize a subject's condition.

By reserving the right to proviso out or exclude any individual members of any such group, including any sub-ranges or combinations of sub-ranges within the group, that can be claimed according to a range or in any similar manner, less than the full measure of this disclosure can be claimed for any reason. Further, by reserving the right to proviso out or exclude any individual substituents, analogs, compounds, ligands, structures, or groups thereof, or any members of a claimed group, less than the full measure of this disclosure can be claimed for any reason. Throughout this disclosure, various patents, patent applications and publications are referenced. The disclosures of these patents, patent applications and publications more fully describe the state of the art as known to those skilled therein as of the date of this disclosure. This disclosure will govern in the instance that there is any inconsistency between the patents, patent applications and publications cited and this disclosure.

For convenience, certain terms employed in the specification, examples and claims are collected here. Unless defined otherwise, all technical and scientific terms used in this disclosure have the same meanings as commonly understood by one of ordinary skill in the art to which this disclosure belongs.

Various embodiments of the invention are directed to compositions for topical delivery of active agents. The compositions comprise an active agent and a decoy molecule that is capable of causing rearrangement of tissues that the composition contacts by temporarily disrupting cell-cell (*i.e.* intercellular) and cell-scaffold attachment, thereby allowing the active agent to pass through cell layers and distribute throughout the tissue passively. The compositions and methods described herein can be used for administering any active agent including small molecule drugs, macromolecular drugs, biologics, antibodies, chimeric antibodies, antigens, peptides, antioxidants, cosmetic ingredients, therapeutic cells, diagnostic agents, radioactive tracers, contrast agents, neurotoxins, and the like and combinations thereof. The compositions and methods can also be used for diagnostic purposes and mediating the flow of diagnostic molecules through various tissues. In some embodiments, the compositions can be applied to any surface tissue, including skin, mucosa, eyes, ears, inside the nose, inside the mouth, lips, urethral openings, vaginal, anus, tongue, frenulum of tongue, hair, teeth, bone, lacrimal glands, sinus mucosa, respiratory tract, gums, and the like and combinations thereof.

### Decoy Molecules

The decoy molecule is an extracellular matrix component or a fragment thereof having an average molecular weight of about 2,000 daltons to about 30,000 daltons. Extracellular matrix component can be divided into several classes biomolecules based upon their structure and function within the extracellular matrix. The most prominent class is the structural class of extracellular matrix proteins. These consist primarily of the collagen and elastin families of proteins. Collagen fibers strengthen and organize the matrix; elastin fibers provide flexibility and resilience. Another class is of specialized proteins, such as fibrillin, fibronectin, laminin, merosin, tenascin, and vitronectin serve less of a structural role and more of an adhesive or integral role within the extracellular matrix; these proteins allow for cell attachment and form crosslinks within the matrix gel. Finally, numerous proteoglycans and heparan sulfate containing proteins form the highly hydrated gel-like mixture that helps stabilize the matrix within its aqueous environment. Proteoglycans are comprised of a protein core to which is attached long chains of glycosaminoglycans (GAGs) forming extremely complex high molecular weight components of the ECM. Another GAG which is a component of extracellular matrix is hyaluronic acid, a non-sulfate GAG.

For example, in some embodiments, the decoy molecule may be proteins, peptides, or a receptor associated with the extracellular matrix, hyaluronic acid, elastin, collagen, fibronectin, lectin, and fragments thereof and combinations thereof. Such fragments include, without limitation, hyaluronic acid fragments, collagen fragments, fibronectin fragments, elastin fragments, lectin fragments, and combinations thereof.

In particular embodiments, the decoy molecule may be hyaluronic acid. Hyaluronic acid is known to interact with, for example, CD44, receptor for hyaluronic acid - mediated motility (RHAMM), and intercellular adhesion molecule-1 (ICAM-1). CD44 is widely distributed throughout the body and mediates cell interaction with hyaluronic acid. ICAM-1 is a metabolic cell surface receptor for hyaluronic acid, and binding of hyaluronic acid to ICAM-1 may contribute to the control of ICAM-1-mediated inflammatory activation. Hyaluronic acid is polymer of disaccharides. Without wishing to be bound by theory, low molecular weight fragments of hyaluronic acid may disrupt cell-cell and cell-scaffold attachments by interrupting intercellular interactions and/or by triggering cellular injury response, which may disrupt intercellular interactions between cells that do not directly contact the hyaluronic acid decoy molecule. Hyaluronic acid may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da. In some embodiments, hyaluronic acid fragments are not cross-linked.

In some embodiments, the decoy molecule may be collagen. Collagen can be isolated in a various forms and from a number of sources. Example collagens include collagen type I, collagen type II, collagen type III, collagen type IV, or collagen type V. The collagen can also be fibrillary collagen or non-fibrillar collagen. Low molecular weight collagens can be made, for example, by hydrolysis, and like hyaluronic acid, low molecular weight collagen may disrupt cell-cell and cell-scaffold attachments by interrupting intercellular interactions and/or by triggering cellular injury response, which may disrupt intercellular interactions between cells deeper in the tissue. Collagen fragments may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

In certain embodiments, the decoy molecule may be fibronectin. Fibronectin is a protein dimer, consisting of two nearly identical monomers linked by a pair of disulfide bonds. Fibronectin binds to membrane-spanning receptor proteins called integrins and extracellular matrix components such as collagen, fibrin, and heparin sulfate proteoglycans. Like hyaluronic acid and collagen, fibronectin fragments may disrupt cell-cell and cell-scaffold attachments by interrupting intercellular interactions and/or by triggering cellular injury response, which may disrupt intercellular interactions between cells deeper in the tissue. Fibronectin fragments may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

In some embodiments, the decoy molecule may be elastin. Elastin is a protein found in connective tissue and allows many tissues in the body to resume their shape after stretching or contracting. Like hyaluronic acid, collagen, and fibronectin, elastin fragments may disrupt cell-cell and cell-scaffold attachments by interrupting intercellular interactions and/or by triggering cellular injury response, which may disrupt intercellular interactions between cells deeper in the tissue. Elastin fragments may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

Elastin fragments may be obtained commercially or may be generated by protease digestion, such as using proteinase K or thermolysin. For example, commercially available Elastin E91 preparation from Protein Preparations, Inc., St. Louis, MO, is a suitable elastin product to subject to digestion, having about 1,000 to about 60,000 Dalton molecular weight. Additionally, a series of digests available under the trade name ProK, and specifically ProK-60 and ProK-60P, which are elastin peptide mixtures derived from the proteolytic digestion of insoluble elastin derived from bovine neck ligaments, can also be used.

In some embodiments, the elastin peptide fragments may comprise an amino acid sequence selected from the group consisting of: GAAPG, GVVPG, GGGPG, GLLPG, GIIPG, GSSPG, GTTPG, GCCPG, GMMPG, GFFPG, GYYPG, GWWPG, GDDPG, GNNPG, GEEPG, GQQPG, GRRPG, GHHPG, GKKPG, GPPPG, G3Hyp3HypPG (Glycine-3-hydroxyproline-3-hydroxyproline-Proline-Glycine), G4Hyp4HypPG (Glycine-4-hydroxyproline-4-hydroxyproline-Proline-Glycine), RRPEV, QPSQPGGV, PGGV, GPGV, KPGV, GPGL, EGSA, PGGF, GGGA, KPGKV, PGGV, KPKA, GPGGV, GPQA, GGPGI, PGPGA, GPGGV, GQPF, GGKPPKPF, GGQQPGL, GGPGI, VGVAPG, IGVAPG, PGGVLPG, VGVVPG, IGLGPGGV, VGAMPG, VGLSPG, IGAMPG, IGLSPG, GVAPGV, VAPGVG, APGVGV, PGVGVA, and GVGVAP.

In some embodiments, the decoy molecule may be laminin. The protein laminin is a complex, consisting of three different polypeptide chains (α, β, γ) that are bound to each other by disulfide bonds into a cross-shaped molecule comprising one long and three short arms with globules at each end. The α-2 chain is a subunit of laminin-2 (merosin) and laminin-4 (S-merosin). Its cell binding ability (via membrane bound integrin receptors) makes laminin an effective substrate coating for stimulating and enhancing cell migration and neurite outgrowth. In laminin from placenta, the A chain is replaced with merosin, and in laminin found near the neuromuscular junction, the B1 chain is replaced by s-laminin (synapse laminin). Laminin fragments may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

In some embodiments, the decoy molecule may be lectin. Lectins are often complex, multi-domain, multimeric proteins. However, the carbohydrate-binding activity of mammalian lectins is normally the property of a carbohydrate recognition domain or CRD. The CRDs of mammalian lectins fall into three phylogenetically conserved classes: C-type, S-type and P-type. C-type lectins require Ca⁺⁺for ligand binding, are extracellular membrane and soluble proteins and, as a class, bind a variety of carbohydrates. S-type lectins are most active under reducing conditions, occur both intra- and extracellularly, bind β-galactosides and do not require Ca⁺⁺. P-type lectins bind mannose 6-phosphate as their primary ligand. Lectin fragments may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

In some embodiments, the decoy molecule may be at least one of: heparin sulfate, chondroitin sulfate, keratan sulfate, laminin, merosin, tenascin, vitronectin, and fibrillin, and fragments thereof and combinations thereof. These decoy molecules may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

In some embodiments, the decoy molecule may be extracellular matrix receptors, such as integrins, discoidin domain receptors and syndecans, and fragments thereof and combination thereof. These decoy molecules may have an average molecular weight of less than about 60,000 Da, about 2,000 Da to about 60,000 Da, about 2,000 Da to about 40,000 Da, about 5,000 Da to about 40,000 Da, about 2,000 Da to about 30,000 Da, about 2,000 Da to about 20,000 Da, or about 2,000 Da to about 10,000 Da.

The size of the decoy molecule may impact the cell-cell and cell-scaffold disruption, and in various embodiments, the decoy molecule may have an average molecular weight of about 2000 daltons to about 30,000 daltons ("Da"). In other embodiments, the decoy molecule may have an average molecular weight of about 2,000 Da to about 5,000 Da ("very small" size), about 5,000 Da to about 10,000 Da ("small" size), about 10,000 Da to about 20,000 Da ("small-to-mid" size), about 20,000 Da to about 30,000 Da ("low-to-mid" size). Because the decoy molecule generally includes fragments of extracellular matrix components, the compositions of embodiments may include decoy molecules falling within any of the ranges identified above and outside the "average molecular weight." For example, the decoy molecule may include individual molecules that are large and extra-large or very small and small when the average molecular weight is small-to-mid.

In embodiments, the extracellular matrix component, fragments thereof and combinations thereof (the decoy molecule) present in the composition have specified average molecular weight. In embodiments, the decoy molecule may have an average molecular weight of about 2,000 Da to about 30,000 Da, about 2,000 to about 20,000 Da, about 2,000 to about 15,000 Da, or any range or individual number falling within these example ranges and numbers.

Disclosed herein, compositions may include substantially no extracellular matrix component, fragments thereof and combinations thereof (the decoy molecule) having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In other embodiments, the composition may contain no detectable decoy molecule having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In further embodiments, the composition may contain less than 0.05% of decoy molecule (that is, less than 0.05 wt. % of the total decoy molecules present in the composition) having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In further embodiments, the composition may contain less than 0.1% of decoy molecule (that is, less than 0.1 wt. % of the total decoy molecules present in the composition) having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In some embodiments, the composition may contain less than 1% decoy molecule (that is, less than 1 wt. % of the total decoy molecules present in the composition) having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da.

In embodiments, the decoy molecule is hyaluronic acid or fragments thereof, wherein substantially all of the hyaluronic acid or fragments thereof do not have a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In other embodiments, the decoy molecule is hyaluronic acid or fragments thereof, wherein there is no detectable hyaluronic acid or fragments having a molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In other embodiments, the decoy molecule is hyaluronic acid or fragments thereof, wherein less than 0.1% of the hyaluronic acid or fragments thereof have a molecular weight molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In other embodiments, the decoy molecule is hyaluronic acid or fragments thereof, wherein less than 1% of the hyaluronic acid or fragments thereof have a molecular weight molecular weight above about 150,000 Da, above about 125,000 Da, above about 100,000 Da, above about 90,000 Da, above about 80,000 Da, above about 70,000 Da, above 60,000 Da, above about 55,000 Da, above about 50,000 Da, above about 45,000 Da, above about 40,000 Da or above about 35,000 Da. In embodiments, the decoy molecule may further comprise collagen, fibronectin, elastin, lectin, collagen fragments, fibronectin fragments, elastin fragments, lectin fragments, and combinations thereof.

The amount of decoy in the composition may impact the cell-cell and cell-scaffold disruption by modulating the depth of the disruption, thereby modulating the depth of penetration of the active agent. The amount of decoy present in the compositions of various embodiments is from about 0.001 wt. % to about 10 wt. %, and in particular embodiments, the amount of decoy in such compositions may be from about 0.1 wt. % to about 2.0 wt. %, about 0.25 wt. % to about 3.0 wt. %, about 0.5 wt. % to about 5.0 wt. %, about 0.75 wt. % to about 7.5 wt. %, or any range or individual concentration encompassing these example ranges. As indicated above, the amount of decoy molecule can modulate the depth of penetration of the active agent. For example, a relatively low concentration of decoy molecule, e.g. about 0.1 wt. % to about 2.0 wt. % or about 0.25 wt. % to about 1.0 wt. %, may allow for transport of an active agent partially across the epidermis, for example, through the stratum granulosum and into the stratum spinosum, when the composition is administered topically. A higher concentration of decoy molecule, e.g. about 0.5 wt. % to about 5.0 wt. % or about 0.5 wt. % to about 3.0 wt. %, may allow for transport of an active agent fully across the epidermis to the basement membrane underlying tissues layers, for example, dermis, subcutis, and blood stream, when the composition is administered topically. The weight percentages disclosed herein may be weight-to-weight or weight-to-volume percentages with respect to the total amount of the composition.

In some embodiments, one or more decoy molecules may be present from about 0.001 wt. % to about 10 wt. %, about 0.001 wt. % to about 9 wt. %, about 0.001 wt. % to about 8 wt. %, about 0.001 wt. % to about 7 wt. %, about 0.001 wt. % to about 6 wt. %, about 0.001 wt. % to about 5 wt. %, about 0.001 wt. % to about 4 wt. %, about 0.001 wt. % to about 3 wt. %, about 0.001 wt. % to about 3 wt. %, or about 0.001 wt. % to about 1 wt. % of the total composition. Specific examples include about 0.001 wt. %, about 0.01 wt. %, about 0.1 wt. %, about 0.5 wt. %, about 1 wt. %, about 2 wt. %, about 5 wt. %, about 10 wt. %, and ranges between any two of these values. The weight percentages disclosed herein may be weight-to-weight or weight-to-volume percentages with respect to the total amount of the composition.

In some embodiments, one or more decoy molecules may be present from about 1 microgram to about 100 milligrams per mL of the composition, about 1 microgram to about 10 milligrams per mL of the composition, about 1 microgram to about 5 milligrams per mL of the composition, about 1 microgram to about 1 milligram per mL of the composition, or about 1 microgram to about 100 micrograms per mL of the composition.

Because the concentration of decoy molecule can modulate the depth of penetration of the active agent, active agents that target, for example, the epidermis may be included in compositions containing lower concentrations of decoy molecule, e.g. about 0.1 wt. % to about 2.0 wt. % or about 0.25 wt. % to about 1.5 wt. %, and active agents that target, for example, dermis or subcutis may be included in compositions containing higher concentrations of decoy molecule, e.g. about 1.0 wt. % to about 5.0 wt. % or about 1.0 wt. % to about 3.0 wt. %. Similarly, the size of the active agent may impact the formulation of the composition. For example, a large active agent, such as a macromolecule therapeutic or biologic/therapeutic peptide may require higher concentrations of decoy molecule, e.g. about 0.5 wt. % to about 5.0 wt. % or about 0.5 wt. % to about 3.0 wt. %, to allow administration to the epidermis even though similar concentrations may allow administration of smaller therapeutics to the dermis or systemic administration through the blood stream.

### Hyaluronidase/elastase

In some embodiments, enzymes such as hyaluronidase and elastase may be used in combination with decoy molecules to facilitate delivery of active agents. Without wishing to be bound by theory, the presence of the enzymes hyaluronidase and/or elastase may cause rearrangement of tissues by degrading the extracellular matrix and temporarily disrupting cell-cell (i.e. intercellular) and cell-scaffold attachment, and allow the active agent to pass through the cell layers into the tissue effectively. Hyaluronidases and elastases, when administered, can spread and diffuse rapidly through tissues, can modify the permeability and viscosity of the intercellular cement by hydrolyzing hyaluronic acid and elastin.

In some embodiments, hyaluronidases and elastases may be used in combination with decoy molecules. The presence of the decoy molecules may also disrupt cell-cell (i.e. intercellular) and cell-scaffold attachment thereby enhancing the effect of the hyaluronidases and elastases synergistically, and allowing the active agent to penetrate and pass through the tissue. The decoy molecules may provide synergistic effect along with hyaluronidase/elastase, and deliver the active agent more effectively.

In embodiments, the hyaluronidases and elastases may cause the active agent to penetrate or diffuse into the underlying tissue after a lag period of administering the active agent and the decoy molecule. The presence of high molecular weight decoy molecule may not effectively cause the active agent to penetrate and pass through the tissue. During the lag period, the hyaluronidases and elastases in the composition may degrade the high molecular weight decoy molecules and generate smaller fragments, which may help in the penetration of the active agent. Thus, the enzymes hyaluronidases and elastases, the selection of the decoy molecule(s), the average molecular weight, the presence (or absence) of high molecular weight decoy molecule, the amount of decoy molecule, the active agent being delivered and the target surface tissue will affect the ability of the active agent to be delivered topically to the desired site of action.

In embodiments, the compositions may include an enzyme selected from hyaluronidase, elastase, or a combination thereof. The hyaluronidase enzyme family consists of enzymes capable of hydrolyzing or "breaking down" the polysaccharide hyaluronic acid. Hyaluronic acid is an important constituent of connective tissue. Hyaluronidases can be broadly classified into three groups: mammalian-type hyaluronidases (EC 3.2.1.35) are endo-beta-N-acetylhexosaminidases that produce tetrasaccharides and hexasaccharides as the major end products. They have both hydrolytic and transglycosidase activities, and can degrade hyaluronan and chondroitin sulfates (CS), specifically C4-S and C6-S. Bacterial hyaluronidases (EC 4.2.99.1) degrade hyaluronan and, and to various extents, CS and DS. They are endo-beta-N-acetylhexosaminidases that operate by a beta elimination reaction that yields primarily disaccharide end products. Hyaluronidases (EC 3.2.1.36) from leeches, other parasites, and crustaceans are endo-beta-glucuronidases that generate tetrasaccharide and hexasaccharide end products through hydrolysis of the beta 1-3 linkage.

The hyaluronidase disclosed herein can be derived from any source whatsoever and, for instance, may be recovered from bovine protein (bovine type), leeches or bacteria (e.g. in the form of hyaluronate lyase). The hyaluronidase can also be of vegetable origin. Genetic engineering techniques in the art can likewise be used to produce hyaluronidase. Various types of hyaluronidase can be also obtained commercially, e.g.. from Wyeth-Ayerst (Wydase^{®}), Abbot (Hyazyme), Bristol-Myers Squibb (Enzodase), and Ortho Pharmaceuticals (Diffusin). Non-limiting examples of hyaluronidases that can be used in the compositions are human hyaluronidase-1 (SEQ ID NO: 1), human hyaluronidase-2 (SEQ ID NO: 2), human hyaluronidase-3 (SEQ ID NO: 3), human hyaluronidase-4 (SEQ ID NO: 4), and human PH20 (SEQ ID NO: 5).

Elastase (EC 3.4.21.36.) is a member of a group of enzymes termed "serine proteases" which are characterized by the reactivity of a serine residue in the active site of the enzyme. Elastase breaks down elastin, the specific protein of elastic fibers, and digests other proteins such as fibrin, haemoglobin and albumin. Three structurally related types of elastase, named elastases I, II and III (or protease E), have been identified, with several isoforms being secreted by the mammalian exocrine pancreas. Elastase has been confirmed to exist in the pancreas of most mammals, including humans, monkeys, cats, rabbits, etc. Elastase disclosed herein can be derived from any source, and can be produced by genetic engineering techniques. Non-limiting examples of elastases that can be used are human elastase I (SEQ ID NO: 6), human elastase II, and human elastase III.

In some embodiments, an effective amount of hyaluronidase, elastase, or a combination thereof in the composition is 1.0 unit, 2 units, 3, units, 4 units, 5 units, 6 units, 7 units, 8 units, 9 units, 10 units, 11 units, 12 units, 13 units, 14 units, 15 units, 16 units, 17 units, 18 units, 19 units, 20 units, 25 units, 30 units, 35 units, 40 units, 45 units, 50 units, 100 units, 125 units, 150 units, 175 units, 200 units, 225 units, 250 units, 275 units, 300 units, 325 units, 350 units, 375 units, 400 units, 425 units, 450 units, 475 units, 500 units, 750 units, 1000 units, 1500 units, 2000 units, and any individual amount or any ranges between any two of these values.

In embodiments, an effective amount of hyaluronidase, elastase, or a combination thereof in the composition is selected from about 0.1 wt. % to about 25 wt. %, about 0.1 wt. % to about 20 wt. %, about 0.1 wt. % to about 15 wt. %, about 0.1 wt. % to about 10 wt. %, about 0.1 wt. % to about 8 wt. %, about 0.1 wt. % to about 5 wt. %, about 0.1 wt. % to about 4 wt. %, about 0.1 wt. % to about 3 wt. %, about 0.1 wt. % to about 3 wt. %, or about 0.1 wt. % to about 1 wt. %. Specific examples include about 0.1 wt. %, about 0.5 wt. %, about 1 wt. %, about 2 wt. %, about 5 wt. %, about 10 wt. %, about 25 wt. %, and any individual amount or any ranges between any two of these values. In embodiments, the weight percentages disclosed herein may be weight-to-weight or weight -to-volume percentages with respect to the total amount of the composition.

In embodiments, the effective amount of hyaluronidase, elastase, or a combination thereof in the composition is selected from about 1 microgram to about 100 milligrams per mL of the composition, about 1 microgram to about 10 milligrams per mL of the composition, about 1 microgram to about 5 milligrams per mL of the composition, about 1 microgram to about 1 milligram per mL of the composition, or about 1 microgram to about 100 micrograms per mL of the composition.

### Active Agents

The compositions of various embodiments may include nearly any active agent, including agents for topical, or local delivery. Non-limiting examples of active agents include a biologic, therapeutic peptides, biomimetic peptide, small molecule and macromolecular analgesic agents, antifungal agents, antibacterial agents, anesthetic agents, proteins, prostaglandins, enzyme inhibitors, steroids, small molecule drugs, macromolecular drugs, biologics, antibodies, chimeric antibodies, antibody fragments, diagnostic antibodies, antigens, peptides, adjuvants, antioxidants, cosmetic ingredients, therapeutic cells, diagnostic agents, radioactive tracers, contrast agents, neurotoxins, sensation modifying agents, and the like and combinations thereof.

Biologic, therapeutic peptides, and biomimetic peptide encompassed by embodiments include, but are not limited to, botulinum toxin and chimeras or derivatives thereof, antibodies, antibody fragments, derivatives of antibodies, Rejuline, CG-Purilux, CG-Dermaheal, CGKeramin2, Prohairin-β4, CG-TGP2, CG-EDP3, CG-IDP, and the like and combinations thereof.

Non-limiting examples of analgesic agents, antifungal agents, antibacterial agents, and anesthetic agents, and steroids include gabapentin, pregabalin, minocycline, acetyl salicylic acid, cyclosporine, tacrolimus (FK506), bimatoprost and other PGE2 inhibitors, tadalafil, clindamycin, cortisone, minoxidil, minoxidil sulfate, niacinamide, methylsalicylate, gabapentin, hydrocortisone, palmitoyl-KTTKS peptide, phenytoin, vitamin B12, cyclobenzaprine, anastrozole, lidocaine, retinoic acid, retinyl propionate, minocycline, gentamicin sulfate, bimatoprost, minoxidil sulfate, clobetasol propionate, ascorbic acid, tranexamic acid, salicylic acid (sodium salicylate), hydroquinone, Renokin^{®}, tolfnaftate, clotrimazole, terbinafine, isotretinoin, trentinoin, kojic acid, prednisone, a sunscreen actives such as homosalate, octisalate, octocrylene, or avobenzone, hydrocortisone, lidocaine, ixekizumab taltz, aminolevulinic acid (ALA), baricitinib, tofacitinib, adalimumab, citronella oil, 3(N-butyl-N-acetyl)aminopropionic acid ethyl ester, sarecycline, D3 analogs, calcineurin inhibitors, meclorethamine, immunization antigens, imiquimod, ibuprofen, celecoxib, diclofenac, sildenafil, cyclopyrox, sarecycline, estrogen, conjugated estrogens (PREMARIN^{®}), and the like and combinations thereof.

In various embodiments, the active agent may be one or more of the following: α-Tocopherol, β-Carotene, 2-Mercaptobenzothiazole, Abacavir, Abatacept, Abciximab, Abrotanum, Absinthium, Acacia, Acamprosate, Acarbose, Acebutolol, Acepromazine Maleate, Acetagesic, Acetaminophen, Acetazolamide, Acetic Acid, Acetohydroxamic Acid, Acetylcysteine, Acetyl-Tyrosine, Acidulated Phosphate Fluoride, Acitretin, Aclidinium, Aconite, Aconitum Napellus, Acremonium Cephalosporium, Actaea Spicata, Acyclovir, Adalimumab, Adapalene, Adenine, Adenosine, Adonis Vernalis, Adrenalinum, Aesculus Hip, Aethusa Cynapium, Afatinib, Afoxolaner, Agaricus Muscarius, Agnus Castus, Ailanthus Glandulosus, Aklomide, Alanine, Albendazole, Albiglutide, Albumin Human, Albuterol, Alcaftadine, Alclometasone, Aldesleukin, Alendronate, Aletris Farinosa, Alfalfa, Alfaxalone, Alfentanil, Alfuzosin, Alirocumab, Aliskiren Hemifumarate, Alitretinoin, Allantion, Allopurinol, Almotriptan, Alnus Glutinosa, Aloe, Alosetron, Alprazolam, Alprostadil, Alstonia Constricta, Alternaria Tenuis, Altrenogest, Aluminum, Amantadine, Ambrergris, Ambrosia Artemisiaefolia, Amikacin, Amiloride, Aminocaproic Acid, Aminohippurate, Aminolevulinic Acid, Aminopentamide, Aminophylline, Aminopropazine, Amiodarone, Amitriptyline, Amlodipine, Ammonia, Amobarbital, Amoxapine, Amoxicillin, Amphetamine, Amphomycin, Amphotericin B, Ampicillin, Amprolium, Amyl Nitrosum, Anagallis Arvensis, Anagrelide, Anastrozole, Anhydrous, Anidulafungin, Anthralin, Apomorphine, Apraclonidine, Apramycin, Argatroban, Argentum Metallicum, Arginine, Aripiprazole, Armodafinil, Arnica, Arsenamide, Arsenic, Arsenicum, Artemether, Articaine, Asafoetida, Asarum Europaeum, Asclepias Tuberose, Ascorbic Acid, Asenapine Maleate, Aspartic Acid, Aspirin, Atracurium Besylate, Atriplex Lentiformis, Atropa Belladonna, Atropine, Attapulgite, Aureobasidium Pullularia Pullulans, Aurum Bromatum, Aurum Iodatum, Aurum Metallicum, Aurum Muriaticum, Avena Sativa, Avibactam, Avilamycin, Avobenzoe, Avovenzone, Axitinib, Azacitidine, Azaperone, Azathioprine, Azelaic Acid, Azelastine, Azithromycin, Aztreonam, Bacitracin, Baclofen, Badiaga, Balsalazide, Balsamum Peruvianum, Bambermycins, Baptisia Tinctoria, Barium, Baryta, Basiliximab, Beclomethasone, Belatacept, Benazepril, Bendroflumethiazide, Bentoquatam, Benzalkonium, Benzocaine, Benzonatate, Benzophenone, Benzoyl peroxide, Benzphetamine, Benztropine, Benzyl Alcohol, Beractant, Beta Carotene, Beta-Aminopropionitrile, Betamethasone, Betaxolol, Bethanechol, Bexarotene, Bezlotoxumab, Bicalutamide, Bicisate, Bimatoprost, Biotin, Bisacodyl, Bismuthum Metallicum, Bisoprolol Fumarate, Bivalirudin, Bleomycin, Boceprevir, Boldenone, Borax, Boricum Acidum, Bosutinib, Botrytis Cinerea, Botulinum Toxin Type A, Bovine Somatotropin (Sometribove Zinc), Brimonidine, Brinzolamide, Brodalumab, Bromfenac, Bromine, Bromocriptine, Budesonide, Bultabital, Bumetanide, Bupivacaine, Buprenorphine, Buquinolate, Buspirone, Butabarbital, Butacaine, Butalbital, Butamben, Butamisole, Butenafine, Butorphanol, Butyl, Cabazitaxel, Cabergoline, Caladium Seguinum, Calamine, Calcarea Acetica, Calcarea Arsenicica, Calcarea Carbonica, Calcarea Caustica, Calcarea Flour, Calcarea Fluorica, Calcarea Iodata, Calcarea Muriatica, Calcarea Oxalica, Calcarea Phosphorica, Calcarea Silicate, Calcarea Suflruica, Calcarea Sulphurica, Calceria Carbonica, Calceria Phosphorica, Calcipotriene, Calcipotriene, Calcitriol, Calcium, Calgest, Cambendazole, Camphor, Canakinumab, Candesartan Cilexetil, Cantharidinum, Cantharis, Capecitabine, Capromorelin, Capsaicin, Capsicum, Captopril, Caramiphen Edisylate, Carbachol, Carbadox, Carbamazepine, Carbamide, Carbidopa, Carbo Animalis, Carbo Vegetabilis, Carbolicum Acidum, Carbomycin, Carbon, Carbonate Lime, Carbonate of Barium, Carbonate Of Potassium, Carbonate Of Sodium, Carboneum, Carboplatin, Carboprost Tromethamine, Carboxymethylcellulose, Carduus Marianus, Carfentanil Citrate, Carisoprodol, Carnidazole, Carprofen, Carum Carvi, Carvedilol, Cascarilla, Casein, Caspofungin, Castanea Vesca, Castoreum, Caulophyllum, Causticum, Cedron, Cefaclor, Cefadroxil, Cefazolin, Cefdinir, Cefepime, Cefotaxime, Cefotetan, Cefovecin, Cefoxitin, Cefpodoxime Proxetil, Cefprozil, Ceftaroline Fosamil, Ceftazidime, Ceftiofur, Ceftriaxone, Cefuroxime, Celecoxib, Cenchris Contortrix, Cephalanthus Occidentalis, Cephalexin, Cephapirin B, Ceritinib, Cetirizine, Cetylpyridinium, Cevimeline, Chaetomium Globosum, Chelidonium Majus, Chenodiol, Chenopodium Anthelminticum, Chimaphila Umbellata, China Sulphuricum, Chinchona Officinalis, Chininum, Chlophedianol, Chloral, Chloramine, Chloramphenicol, Chlorcyclizine, Chlordiazepoxide, Chlorhexidine, Chlorine, Chlorinum, Chlorobutanol, Chloroprocaine, Chloroquine, Chlorothiazide, Chloroxylenol, Chlorphenesin, Chlorpheniramine, Chlorpromazine, Chlorpropamide, Chlortetracycline, Chlorthalidone, Chlorzoxazone, Cholecalciferol, Cholesterinum, Cholestyramine, Choriogonadotropin Alfa, Chorionic Gonadotropin, Chromic, Chromium, Chymotrypsin, Ciclopirox, Cicuta Virosa, Cilastatin, Cilostazol, Cimetidine, Cimex Lectularius, Cimicifuga Racemosa, Cina, Cineraria Maritima, Ciprofloxacin, Cisatracurium, Cisplatin, Cistus Canadensis, Citalopram, Citric Acid, Citroma Magnesium, Cladosporium Cladosporioides, Cladribine, Clarithromycin, Clavulanate, Clemastine, Clematis Erecta, Clenbuterol, Clidinium, Clindamycin, Clioquinol, Clobetasol, Clocortolone, Clodronate, Clofarabine, Clomiphene, Clomipramine, Clonazepam, Clonidine, Clopidogrel, Clopidol, Cloprostenol, Clorazepate, Clorsulon, Clotrimazole, Cloxacillin, Clozapine, Cobalamin, Cobaltum, Cobicistat, Cocaine, Codeine, Colchicine, Colchicinum, Colestipol, Colistimethate, Collagenase Santyl, Colloidal Ferric Oxide, Colloidal Sulfur, Colocynthis, Compound Benzoin, Condurango, Conium, Conjugated Estrogens, Convallaria Majalis, Copaiva Officinalis, Copper, Corticotropin, Cortisone, Cosyntropin, Coumaphos, Cratageus Oxycantha, Cresol, Crizotinib, Crocus Sativus, Cromolyn, Crotalus Horridus, Crotamiton, Croton Tiglium, Crypthecodinium Cohnii DHA Oil, Cubeba Officinalis, Cucurbita Citrullus, Culex Pipiens, Cupric, Cuprum, Curvularia Inaequalis, Cuttlefish Ink, Cyanocobalamin, Cyclamen Europaeum, Cyclizine, Cyclobenzaprine, Cyclophosphamide, Cycloserine, Cyclosporine, Cyproheptadine, Cysteine, Cytarabine, Cythioate, Dabigatran, Dabrafenib, Dacarbazine, Daclatasvir, Daclizumab, Daikon, Dalfampridine, Dalteparin, Danazol, Danofloxacin, Dantrolene, Dapagliflozin, Dapsone, Daptomycin, Darifenacin, Dasabuvir, Dasatinib, Datura Stramonium, Daunorubicin, Decitabine, Decoquinate, Deferasirox, Deferoxamine, Delavirdine, Delphinium Staphisagria Seed, Delsym, Demeclocycline, Deracoxib, Desflurane, Desipramine, Desirudin, Desloratadine, Deslorelin, Desmopressin, Desogestrel, Desonide, Desoximetasone, Desoxycorticosterone, Desvenlafaxine, Dexamethasone, Dexmedetomidine, Dexrazoxane, Dextran, Dextrmethorphan, Dextroamphetamine, Dextrose, Diatrizoate, Diazepam, Diazoxide, Dibasic, Dibucaine, Dichlorophene, Dichlorvos, Dichromate, Diclazuril, Diclofenac, Dicloxacillin, Dicyclomine, Didanosine, Diethylcarbamazine, Diethylpropion, Diflorasone, Difloxacin, Diflunisal, Digitalis Purpurea, Digoxin, Dihydroergotamine, Dihydrostreptomycin, Diltiazem, Dimenhydrinate, Dimethicone, Dimethyl, Dinoprostone, Dioscorea Villosa, Dioscoreinum, Diphenhydramine, Dipiperazine, Diprenorphine, Dipyridamole, Dirlotapide, Disopyramide, Disulfiram, Dithiazanine, Divalproex, Dobutamine, Docetaxel, Docone, Doconexant, Docosanol, Dofetilide, Dog Epithelia, Dog Fennel, Dolasetron, Dolichos Pruriens, Domperidone, Donepezil, Dopamine, Doramectin, Dorzolamide, Doxapram, Doxazosin, Doxepin, Doxercalciferol, Doxorubicin, Doxycycline, Drechslera Helminthosporium, Dronabinol, Dronedarone, Droperidol, Drosera Rotundifolia, Drospirenone, D-Thiamine, Dulaglutide, Dulcamara, Duloxetine, Durezol, Dutasteride, Dyclonine, Ecamsule, Echinacea Purpurea, Echothiophate, Econazole, Efavirenz, Efinaconazole, Eflornithine, Efrotomycin, Elaps Corallinus, Elbasvir, Eletriptan, Elm Chinese, Eltrombopag Olamine, Embutramid, Emedastine, Emodepside, Empagliflozin, Emtricitabine, Enalaprilat, Enfuvirtide, Enoxaparin, Enrofloxacin, Ensulizole, Entacapone, Entecavir, Enzalutamide, Ephedrine, Ephedrine, Ephedrine, Epicoccum Nigrum, Epigaea Repens, Epinastine, Epinephrine, Epiphegus Virginiana, Epirubicin Hydrochloride, Eplerenone, Epoprostenol, Eprinomectin, Eprosartan, Epsiprantel, Eptifibatide, Equine Thymocyte Immune Globulin, Equisetum Arvense, Equisetum Hyemale, Ergocalciferol, Ergotamine Tartrate, Erigeron Canadensis, Ertapenem, Erysimum Cheiri, Erythromycin, Escitalopram, Esium, Esmolol, Esomeprazole, Estazolam, Esterified, Estradiol, Estrogens, Estrone, Estropipate, Eszopiclone, Ethacrynic Acid, Ethambutol, Ethinyl Estradiol, Ethionamide, Ethopabate, Ethosuximide, Ethyl Alcohol, Ethylisobutrazin, Ethynodiol, Etidronate, Etodolac, Etomidate, Etonogestrel, Etoposide, Etorphine, Eugenia Jambosa, Eugenol, Euphorbia Lathyris, Everolimus, Exemestane, Exenatide, Extract Arisaema Triphyllum Root, Extract Aristolochia Clematitis, Extract Arizona Ash, Extract Arizona Cypress, Extract of Baptisia Tinctoria Root, Extract of Corallium Rubrum, Extract of Abies Canadensis, Extract of Abrus Precatorius Seed, Extract of Anacardium Occidentale, Extract of Anacardium Orientale, Extract of Anamirta Cocculus Seed, Extract of Artemisia Cina Pre-Flowering Top, Extract of Artemisia Vulgaris, Extract of Arum Triphyllum, Extract of Ash Arizona, Extract of Ash White, Extract of Asparagus, Extract of Aspen, Extract of Aspergillus Fumigatus, Extract of Aspergillus Niger, Extract of Australian Pine Beefwood, Extract of Avocado, Extract of Azadirachta Indica, Extract of Bahia Grass, Extract of Bald Cypress, Extract of Barberry, Extract of Barley Food, Extract of Bayberry Wax Myrtle, Extract of Bee Venom, Extract of Beech, Extract of Beef, Extract of Belladonna Leaf, Extract of Bellis, Extract of Berberis Aquarius, Extract of Berberis Aquifolium, Extract of Berberis Vulg, Extract of Berberis Vulgaris, Extract of Bermuda Grass, Extract of Betula Alba, Extract of Birch Black, Extract of Birch River Red, Extract of Birch White, Extract of Bitter Cucumber, Extract of Black Cohosh, Extract of Black Lead, Extract of Black Locust, Extract of Black Pepper, Extract of Black Pollen Walnut, Extract of Black Willow, Extract of Blatta Americana, Extract of Blatta Orientalis, Extract of Blattella Germanica, Extract of Bluegrass Annual, Extract of Box Elder Ash Leaf Maple, Extract of Brazil Nut, Extract of Broccoli, Extract of Brome Grass, Extract of Bryonia, Extract of Buckwheat, Extract of Bushmaster Snake Venom, Extract of Buttercup, Extract of Cabbage, Extract of Cactus Grandiflorus, Extract of Cadmium Sulphuricum, Extract of Caffeine, Extract of Calcitonin Salmon, Extract of Calendula, Extract of California Live Oak Coast, Extract of California Pepper Tree, Extract of California Walnut Black Pollen, Extract of Calomel, Extract of Calotropis Gigantea, Extract of Candida Albicans, Extract of Candida Parapsilosis, Extract of Cantaloupe, Extract of Carelessweed, Extract of Carrot, Extract of Castor Birch, Extract of Castor Equi, Extract of Castor Oil, Extract of Cat Hair, Extract of Cat Pelt, Extract of Cattle Epithelium, Extract of Ceanothus Americanus, Extract of Cedar Elm, Extract of Cedar Mountain, Extract of Cedar Red, Extract of Celery, Extract of Chamomile Plant, Extract of Chastetree, Extract of Cherry, Extract of Chicken Meat, Extract of Chinese Elm, Extract of Chionanthus Virginica, Extract of Cinchona, Extract of Cinnamon, Extract of Citrullus Colocynthis Fruit, Extract of Clam, Extract of Club Moss, Extract of Coal Tar, Extract of Cocculus Cacti, Extract of Cocculus Indicus, Extract of Cocklebur, Extract of Cocoa Bean Whole Bean Chocolate, Extract of Cocoa Butter, Extract of Coconut, Extract of Codfish, Extract of Coffea, Extract of Collinsonia Canadensis, Extract of Collinsonia Canadensis Root, Extract of Colloidal Oatmeal, Extract of Comfrey Plant, Extract of Comfrey Root, Extract of Common Mugwort, Extract of Common Sagebrush, Extract of Common Wormwood Annual, Extract of Conium Maculatum, Extract of Coral Snake (Micrurus Fulvius) Immune Globulin Antivenin (Equine), Extract of Corn, Extract of Cotton Linters, Extract of Cottonseed, Extract of Cottonwood Eastern Common, Extract of Cottonwood Fremont, Extract of Cottonwood Western, Extract of Crab, Extract of Cramp Bark, Extract of Cucumber, Extract of Cuttlefish, Extract of Cypress Arizona, Extract of Cypress Bald, Extract of Daisy, Extract of Dandelion, Extract of Daphne Indica, Extract of Daphne Mezereum Bark, Extract of Deadly Nightshade, Extract of Dock Sour Sheep Sorrel, Extract of Dock Yellow, Extract of Eastern Cottonwood Common, Extract of Echinacea Angustafolia, Extract of Echinacea Angustifolia, Extract of Egg White, Extract of Egg Yolk, Extract of Elm American, Extract of Elm Cedar, Extract of Elotuzumab, Extract of English Plantain, Extract of English Walnut, Extract of English Walnut Pollen, Extract of Eucalyptus, Extract of Eucalyptus Oil, Extract of Eupatorium Perfoliatum, Extract of Eupatorium Perfoliatum Flowering Top, Extract of Eupatorium Purpureum, Extract of Euphrasia, Extract of European Elder, Extract of False Ragweed Bur, Extract of Flounder, Extract of Fragrant Sumac, Extract of Fraxinus Americana, Extract of Fremont Cottonwood, Extract of Freshwater Sponge, Daisy, Extract of Fucus Vesiculosus, Extract of Galphimia Glauca Flowering Top, Extract of Garden Rue, Extract of Garlic, Extract of Gelsemium Sempervirens, Extract of Gelsemium Sempervirens Root, Extract of Geranium Maculatum, Extract of German Cockroach, Extract of Ginger, Extract of Ginkgo Biloba, Extract of Goat Milk, Extract of Goldenrod, Extract of Goldenseal, Extract of Gopher plant, Extract of Grapefruit, Extract of Graphite, Extract of Green Coffee, Extract of Green Pea English, Extract of Guinea Pig Epithelia, Extract of Hackberry, Extract of Hazelnut Pollen, Extract of Heloderma Horridum Venom, Extract of Hemoglobin Glutamer-200 (bovine), Extract of Honey Bee, Extract of Honeydew, Extract of Hops, Extract of Horse Chestnut, Extract of Horse Epithelia, Extract of Horsetail, Extract of Indian Cockle, Extract of Ipecac, Extract of Ipecac Root, Extract of irginia Live Oak, Extract of Iris Germanica Root, Extract of Italian Rye Grass, Extract of Jalapa, Extract of Johnson Grass, Extract of Juglans Regia, Extract of Juniper Western, Extract of Juniperus Communis, Extract of Juniperus Sabina Leafy Twig, Extract of Juniperus Virginiana, Extract of Lachesis Muta, Extract of Lamb, Extract of Lima Bean, Extract of Lobster, Extract of Locust Black Non Stock, Extract of Loose Wheat Smut, Extract of Magnolia Grandiflora, Extract of Maple Red, Extract of Maple Sugar, Extract of Marking Nut, Extract of Marshelder Burweed, Extract of Marshelder Rough, Extract of Matricaria Recutita, Extract of Meadow Fescue Grass Standardized, Extract of Melaleuca Pollen, Extract of Melilotus Officinalis, Extract of Melissa Officinalis, Extract of Mexican Tea, Extract of Milk of Magnesia, Extract of Milk Thistle, Extract of Milk Whole Cows, Extract of Mountain Arnica, Extract of Mountain Cedar, Extract of Mountain Tobacco, Extract of Mouse Epithelia, Extract of Mouse Epithelium, Extract of Mucor Circinelloides F. Lusitanicus, Extract of Mucor Plumbeus, Extract of Mucor Racemosus, Extract of Mugwort Common, Extract of Mulberry Red, Extract of Mulberry White, Extract of Mustard Seed, Extract of Oak California Live Coast, Extract of Oak Red, Extract of Oak Virginia Live, Extract of Oak White, Extract of Oat Grain, Extract of Oat Straw, Extract of Oat Wild Pollen, Extract of Oatmeal, Extract of Oatstraw, Extract of Oil of Mustard Seed, Extract of Old Balsam, Extract of Oleander, Extract of Olive, Extract of Olive Pollen, Extract of Onion, Extract of Orange, Extract of Orchard Grass, Extract of Orris Root, Extract of Oyster, Extract of Palm Queen Coco Palm, Extract of panish Fly, Extract of Parsley, Extract of Passiflora Incarnata, Extract of Passiflora Incarnata Top, Extract of Passion Flower, Extract of Peach, Extract of Peanut, Extract of Pear, Extract of Pecan, Extract of Pecan Pollen, Extract of Pectin, Extract of Pepper Tree California, Extract of Periplaneta Americana, Extract of Picea Mariana Resin, Extract of Pigweed Rough Redroot, Extract of Pigweed Spiny, Extract of Pine Australian Beefwood, Extract of Pine White, Extract of Pine Yellow, Extract of Pineapple, Extract of Pinto Bean Kidney Bean, Extract of Pinus Lambertiana, Extract of Pinus Sylvestris, Extract of Pistachio Nut, Extract of Plantago Major, Extract of Plantago Seed, Extract of Plantain English, Extract of Plum, Extract of Poison Hemlock, Extract of Poison Ivy, Extract of Poison Nut, Extract of Poison oak, Extract of Pongia Officinalis Skeleton, Extract of Poplar White, Extract of Pork, Extract of Pot Marigold, Extract of Prairie Sage, Extract of Psyllium, Extract of Pure Flint, Extract of Purple Cone Flower, Extract of Quack Grass, Extract of Quebracho, Extract of Queen Palm Coco Palm, Extract of Quercus Glandium Spiritus, Extract of Rabbit, Extract of Rabbit Epithelium, Extract of Ragweed False Bur, Extract of Ragweed Short, Extract of Ragweed Slender, Extract of Ragweed Southern, Extract of Ragweed Tall Giant, Extract of Ragweed Western, Extract of Rancid Beef, Extract of Ranunculus Bulbosus, Extract of Raw Opium Gum, Extract of Red Cedar, Extract of Red Maple, Extract of Red Mulberry, Extract of Red Oak, Extract of Red Onion, Extract of Redtop Grass, Extract of Rhamnus Frangula, Extract of Rhododendron Aureum Leaf, of Rhododendron Chrysanthum, Extract of Rhodotorula Rubra, Extract of Rhubarb, Extract of River Birch Red, Extract of Robinia Pseudoacacia, Extract of Rough Marshelder, Extract of Rough Pigweed, Extract of Rough Pigweed Redroot, Extract of Rumex, Extract of Russian Thistle, Extract of Ruta, Extract of Rye, Extract of Rye Grass, Extract of Rye Grass Italian, Extract of Sage Prairie, Extract of Sagebrush Common, Extract of Salmon, Extract of Salt Grass, Extract of Salvia Officinalis, Extract of Sambucus, Extract of Sanguinaria Canadensis, Extract of Saponaria Officinalis Root, Extract of Schoenocaulon Officinale Seed, Extract of Senecio, Extract of Senna, Extract of Sepia, Extract of Serum Gonadotropin, Extract of Sesame Seed, Extract of Shagbark Hickory, Extract of Short Ragweed Pollen Allergen Extract, Extract of Shrimp, Extract of Slender Ragweed, Extract of Solanum, Extract of Solidago Virgaurea, Extract of Solidago Virgaurea Flowering Top, Extract of Sour Dock Sheep Sorrel, Extract of Southern Ragweed, of Soybean, Extract of Soybean Oil, Extract of Spinach, Extract of Spiny Pigweed, Extract of Spongia Officinalis Skeleton, Extract of Squash, Extract of St Ignatius Bean, Extract of St Johns Wort, Extract of Stemphylium Solani, Extract of Stinging Nettle, Extract of Strawberry, Extract of String Bean Green Bean, Extract of Strychnos Ignatii Seed, Extract of Strychnos Nux-Vomica Seed, Extract of Sugar Maple, Extract of Sweet Corn, Extract of Sweet Potato, Extract of Sweet Vernal Grass Standardized, Extract of Sweetgum, Extract of Sweetgum Non Stock, Extract of Sycamore American, Extract of Symphytum, Extract of Tarentula Cubensis, Extract of Tarentula Hispana, Extract of Thuja OCC, Extract of Tobacco Leaf, Extract of Tomato, Extract of Tuna, Extract of Turkey Meat, Extract of Turpentine, Extract of Turpentine Oil, Extract of Uva Ursi, Extract of Valerian, Extract of Vanilla, Extract of Vegetable Charcoal, Extract of Velvet Grass, Extract of Veratrum Album, Extract of Veratrum Album Root, Extract of Veratrum Viride, Extract of Verbascum Thapsus, Extract of Verbena Hastata, Extract of Viburnum Opulus, Extract of Viburnum Opulus Root, Extract of Viola Odorata, Extract of Viola Tricolor, Extract of Walnut Black Pollen, Extract of Walnut California Black Pollen, Extract of Walnut English Pollen, Extract of Water Hemp, Extract of Watermelon, Extract of Western Cottonwood, Extract of Western Juniper, Extract of Western Ragweed, Extract of Wheat Pollen, Extract of Wheat Smut, Extract of White Alder, Extract of White Ash, Extract of White Birch, Extract of White Cedar, Extract of White Mulberry, Extract of White Oak, Extract of White Oxide Of Arsenic, Extract of White Petrolatum, Extract of White Petrolatum Mineral Oil, Extract of White Pine, Extract of White Poplar, Extract of White Potato, Extract of White Seedless Grape, Extract of Whole Arnica Plant, Extract of Whole Egg, Extract of Whole Wheat Wheat Grain, Extract of Wild Hops, Extract of Wild Lavender, Extract of Wild Pollen Oat, Extract of Willow Black, Extract of Wind Flower, Extract of Witch Hazel, Extract of Wood Creosote, Extract of Woody Nightshade, Extract of Wormseed, Extract of Wormwood Common Annual, Extract of Wyethia Helenioides, Extract of Wyethia Helenioides Root, Extract of Yeast Saccharomyces Cerevisiae, Extract of Yellow Dock, Extract of Yellow Jasmine, Extract of Yellow Pine, Extract of Protortonia Cacti, Ezogabine, Fagopyrum Esculentum, Famciclovir, Famotidine, Famphur, Febantel, Fel Tauri, Felbamate, Felodipine, Fenbendazole, Fenofibrate, Fenofibric Acid, Fenoldopam, Fenoprofen, Fenprostalene, Fentanyl, Ferric, Ferrous Fumarate Fire Ant, Ferrous Fumarate Fish Berry, Fesoterodine, Fexofenadine, Fibrinogen, Ficus Religiosa, Filix Mas, Finasteride, Fingolimod, Firocoxib, Flavone, Flecainide, Florbetapir, Florfenicol, Fluconazole, Flucytosine, Fludarabine, Fludeoxyglucose, Fludrocortisone, Flumazenil, Flumethasone, Flunisolide, Flunixin, Fluocinonide, Fluorescein, Fluoride, Fluorometholone, Fluorouracil, Fluoxetine, Fluoxymesterone, Fluphenazine, Fluprostenol, Fluralaner, Flurandrenolide, Flurazepam, Flurbiprofen, Flutamide, Fluticasone, Fluvastatin, Fluvoxamine, Foeniculum Vulgare, Folic Acid, Follitropin, Fomepizole, Formaldehyde, Formalin, Formic Acid, Formica Rufa, Formoterol, Fosaprepitant Dimeglumine, Foscarnet, Fosfomycin Tromethamine, Fosinopril, Fosphenytoin, Frovatriptan, Fulvestrant, Furazolidone, Furosemide, Fusarium, Gabapentin, Gadobenate, Gadodiamide, Gadoteridol, Gadoversetamide, Galantamine, Galanthus Nivalis, Gallicum Acidum, Gallium, Gambogia, Gamithromycin, Ganciclovir, Ganirelix, Gatifloxacin, Gauifenesin, Gaultheria Procumbens, Gefitinib, Gelatin, Gemcitabine, Gemfibrozil, Gentamicin, Gentiana Quinqueflora, Glatiramer, Gleptoferron, Glimepiride, Glipizide, Glonoinum, Glucagon, Gluconolactone, Glutamic Acid, Glutathione, Glyburide, Glycerin, Glycine, Glycopyrrolate, Glycyrrhiza Glabra, Gnaphalium, Goldenseal Root, Gonadorelin, Gonadorelin Acetate, Gonadotropin Releasing Factor - Diphtheria Toxoid Conjugate, Goserelin, Gossypium Herbaceum, Gramicidin, Granisetron, Grapiprant, Gratiola Officinalis, Grazoprevir, Griseofulvin, Guaco, Guafenesin, Guaiacol, Guaiacum, Guaifenesin, Guaifensin, Guanfacine, Haemophilus b Conjugate Vaccine (Meningococcal Protein Conjugate), Hahnemanns Causticum, Halcinonide, Halobetasol, Halofuginone, Haloperidol, Halothane, Haloxon, Hamamelis, Hedeoma Pulegioides, Hekla Lava, Helianthus Annuus, Heliox, Helium, Helleborus Foetidus, Helleborus Niger, Helminthmucor, Helonias Dioica, Heme Iron Polypeptide, Henbane, Hepar, Heparin, Heptahydrate, Hetacillin, Hetastarch, Hexachlorophene, Hexaminolevulinate, Histamine, Histidine, Homatropine, Homosalate, Human Insulin, Human Papillomavirus 9-Valent Vaccine, Human Papillomavirus Quadrivalent (Types 6, 11, 16, 18) Vaccine, Human Recombinant, Human Rho(D) Immune Globulin, Humulus Lupulus, Hyaluronate, Hyaluronidase, Hydorcortisone, Hydralazine, Hydrangea Arborescens, Hydrastis Canadensis, Hydrochloride, Hydrochlorothiazide, Hydrocodone, Hydrocortisone, Hydrocotyle Asiatica, Hydrofluoric Acid, Hydrogen, Hydrogenate Palm Kernel Oil, Hydromorphone, Hydroquinone, Hydrous, Hydroxocobalamin, Hydroxychloroquine, Hydroxyethyl, Hydroxyurea, Hydroxyzine, Hygromycin B, Hyoscyamine, Hyoscyamus Niger, Hypericum, Hypromellose, Ibandronate, Iberis amara, Ibuprofen, Ibutilide, Ichthyolum, Icodextrin, Icosapent, Idarubicin, Idarucizumab, Ifosfamide, Ignatia Amara, Ignatius Bean, Iiris Versicolor, Iloperidone, Imatinib, Imidacloprid, Imidocarb, Imipenem, Imipramine, Imiquimod, Immune Globulin (Human), Impure Calcium, Incobotulinumtoxina, Indacaterol, Indapamide, Indigo, Indinavir, Indium, Indomethacin, Infliximab, Influenza Virus Vaccine, Influenzinum, Ingenol, Insulin, Interferon, Iodides Tincture, Iodinated Casein, Iodine, Iodipamide Meglumine, Iodium, Iodixanol, Iodochlorhydroxyquin, Iohexol, Iopamidol, Iothalamate, Ioversol, Ipecacuanha, Ipilimumab, Ipratropium, Irbesartan, Iridium, Irinotecan, Tenax, Iris Versicolor, Iron, Isavuconazonium, Isodium, Isoflupredone, Isoflurane, Isoleucine, Isometheptene, Isoniazid, Isopropamide, Isopropyl Alcohol, Isoproterenol, Isosorbide, Isotretinoin, Isradipine, Itraconazole, Ivermectin, Ixabepilone, Ixekizumab, Jacaranda Caroba, Jacobaea Maritima, Justicia Adhatoda, Kali Arsenicosum, Kali Arsenicum, Kali Bechromate, Kali Bechromate Karaya Gum Bassora, Kali Bechromate Kentucky Bluegrass (June) Standardized, Kali Bechromate Kochia Firebush, Kali Bechromate Krameria Lappacea Root, Bechromate Lemon, Kali Bechromate Leopards Bane, Kali Bechromate Lettuce, Kali Bichromicum, Kali Bromatum, Kali Carbonate, Kali Carbonicum, Kali Iodatum, Kali Muriaticum, Kali Muriaticum, Silicea, Kali Nitricum, Kali Phosphoricum, Kali Phosphoricum, Kali Sulphuricum, Kali Phosphoricum, Magnesia Phosphorica, Natrum Phosphoricum, Kali Sulph, Kali Sulphuricum, Kalmia Latifolia, Kanamycin Sulfate, Kapok, Ketamine, Ketamine, Ketoconazole, Ketoprofen, Ketorolac, Ketotifen, Ketotifen, Kreosotum, Labetalol, Lac Caninum, Lac Defloratum, Lac Felinum, Lac Vaccinum, Lachnanthes Tinctoria, Lacosamide, Lactic Acid, Lacticum Acidum, Lactuca Virosa, Lactulose, Laidlomycin, Lamium Album, Lamivudine, Lamotrigine, Lanolin, Lanreotide, Lansoprazole, Lapatinib, Lapis Albus, Lappa Major, Lasalocid, Latanoprost, Lathyrus Sativus, Latrodectus Mactans, Lauric Acid, Laurocerasus, Laxative, L-Cysteine, Lead, Lecithin, Ledum, Ledum Palustre, Ledum Palustre Twig, Leflunomide, Lemna Minor, Leptandra Virginica, Lesinurad, Letrozole, Leucine, Leucovorin, Leuprolide, Levalbuterol, Levamisole, Levetiracetam, Levobunolol, Levocarnitine, Levodopa, Levofloxacin, Levoleucovorin, Levomefolate, Levomilnacipran, Levonordefrin, Levonorgestrel, Levorphanol, Levothyroxine, Levulose, Lidocaine, Lilium Tigrinum, Linaclotide, Linagliptin, Lincomycin, Lindane, Linezolid, Linolenic Acid, Liothyronine, Liraglutide, Lisinopril, Lithium, Lixisenatide, Lobaria Pulmonaria, Lobelia Inflata, Lodoxamide Tromethamine, Loperamide, Lopinavir, Loratadine, Lorazepam, Losartan, Loteprednol, Lovastatin, Loxapine, Lubiprostone, Lufenuron, Luffa Operculata, Lugols, Luliconazole, Lumefantrine, Luprostiol, Lutein, Lycopodium, Lycopus Virginicus, Lysine, Lytta Vesicatoria, Macrocrystalline, Maduramicin Ammonium, Mag Phos, Magnesium, Malathion, Manganese, Manganum, Mannitol, Maprotiline Hydrochloride, Maraviroc, Marbofloxacin, Maropitant, Maxzide, Mebendazole, Mebrofenin, Mecamylamine, Mecasermin, Mechlorethamine, Meclizine, Meclofenamate, Medetomidine, Medroxyprogesterone, Mefenamic Acid, Mefloquine, Megestrol Acetate, Melarsomine, Melatonin, Melengestrol Acetate, Meloxicam, Melphalan, Memantine, Mentha Piperita, Menthol, Menyanthes Trifoliata, Mepenzolate, Meperidine, Mephitis Mephitica, Mepivacaine H, Mepolizumab, Meprobamate, Meradimate, Mercaptopurine, Mercurius Corrosivus, Mercurius Dulcis, Mercurius Iodatus Flavus, Mercurius Iodatus Ruber, Mercurius Solubilis, Mercurous, Mercury, Meropenem, Mertiatide, Mesalamine, Mesna, Mesquite, Metaxalone, Metformin, Methadone, Methamphetamine, Methazolamide, Methenamine, Methimazole, Methionine, Methocarbamol, Methotrexate, Methoxsalen, Methoxy Polyethylene Glycol-Epoetin Beta, Methscopolamine, Methsuximide, Methyclothiazide, Methyl Salicylate, Methyldopa, Methylene Blue, Methylergonovine Maleate, Methylphenidate, Methylprednisole, Methylprednisolone, Methylsalicylate, Methyltestosterone, Metoclopramide, Metolazone, Metoprolol, Metoserpate, Metronidazole, Mexiletine, Mezereum, Mibolerone, Micafungin, Miconazole, Midazolam, Miglitol, Miglustat, Milbemycin Oxime, Millefolium, Milnacipran, Milrinone, Minocycline, Minoxidil, Mirabegron, Mirtazapine, Misoprostol, Mitomycin, Mitotane, Mitoxantrone, Mivacurium, Modafinil, Moexipril, Molybdenum, Mometasone Furoate, Monensin, Monobasic, Monohydrate, Montelukast, Morantel, Morphine, Morrhuate, Moschus, Moxidectin, Moxifloxacin, Mupirocin, Murex Purpurea, Muriaticum Acidum, Mycophenilic, Mygale, Myrica Cerifera, Myristica Sebifer, Myristyl, Nabilone, Nabumentone, Nadolol, Nafarelin, Nafcillin, Naftifine, Naja Tripudians, Nalbuphine, Nalorphine, Naloxegol, Naloxone, Naltrexone, Nandrolone, Naphazoline, Naphthalinum, Naproxen, Narasin, Naratriptan, Phos Nutmeg, Natamycin, Nateglinide, Natrum, Nebivolol, Necitumumab, Nedocromil, Nefazodone, Nelarabine, Neomycin, Neostigmine, Nepafenac, Nequinate, Neurospora Intermedia, Neutral Sodium Fluoride, Nevirapine, Niacin, Nicarbazin, Nicardipine, Niccolum, Nicotine, Nifedipine, Nigrospora, Nilotinib, Nilutamide, Nimodipine, Nintedanib, Nisoldipine, Nitenpyram, Nitric Acid, Nitrofurantoin, Nitrofurazone, Nitrogen, Nitroglycerin, Nitromide, Nitrous Oxide, Nivolumab, Nizatidine, Norelgestromin, Norepinephrine, Norethindrone, Norgestimate, Norgestomet, Norgestrel, Nortriptyline, Novobiocin, Nux Moschata, Nux vomica, Nystatin, Ocimum Sanctum, Ocitnoxate, Ocitsalate, Oclacitinib, Octinoxate, Octisalate, Octobenzone, Octocrylene, Octreotide, Oenanthe Crocata, Ofatumumab, Ofloxacin, Olanzapine, Olaparib, Olaratumab, Oleate Sodium, Olmesartan Medoxmil, Olodaterol, Olopatadine, Olsalazine, Ombitasvir, Omeprazole, Onabotulinumtoxina, Ondansetron, Onosmodium Virginianum, Oophorinum, Opium, Opuntia Vulgaris, Orbifloxacin, Orgotein, Orlistat, Ormetoprim, Orphenadrine Citrate, Oseltamivir Phosphate, Osimertinib, Osmium Metallicum, Ova Tosta, Ovine Digoxin Immune Fab, Oxacillin, Oxalicum Acidum, Oxaliplatin, Oxandrolone, Oxaprozin, Oxazepam, Oxcarbazepine, Oxctinoxate, Oxibendazole, Oxiconazole, Oxide of Aluminum, Oxybenzone, Oxybutynin, Oxycodone, Oxygen, Oxymetazoline, Oxymorphone, Oxyquinoline, Oxytetracycline, Oxytocin, Paclitaxel, Padimate O, Paeonia Officinalis, Palbociclib, Paliperidone, Palladium Metallicum, Pamabrom, Pamidronate, Pancrelipase, Pancuronium, Panobinostat, Pantoprazole, Pantothenic Acid, Papaverine, Paraffinum, Pareira Brava, Paricalcitol, Paris Quadrifolia, Paritaprevir, Paroxetine, Pasireotide, Pazopanib, Peg-3350, Pegaspargase, Pegbovigrastim, Peginterferon Alfa-2a, Peginterferon Alfa2b, Pegvisomant, Pembrolizumab, Pemetrexed, Penciclovir, Penicillamine, Penicillin G, Penicillin V, Penicillium Chrysogenum, Penicillium Glabrum, Penicillium Roqueforti, Pentavalent, Pentazocine, Pentobarbital, Pentostatin, Pentoxifylline, Perflutren, Pergolide Mesylate, Perindopril Erbumine, Permethrin, Perphenazine, Petrolatum, Petroleum, Petroselinum, Phellandrium Aquaticum, Phenazopyridine, Phendimetrazine, Phenelzine, Pheniramine Maleate, Phenobarbital, Phenol, Phenothiazine, Phenoxybenzamine, Phenozapyridine, Phentermine, Phentolamine, Phenykephrine, Phenyl Salicylate, Phenylalanine, Phenylbenzimidazole Sulfonic Acid, Phenylbutazone, Phenylephrine, Phenylpropanolamine, Phenyltoloxamine, Phenytoin, Phoma Herbarum, Phophorus, Phosmet, Phosphate of Iron, Phosphorated Carbohydrate, Phosphoric Acid, Phosphorus, Physalis Alkekengi, Physostigma Venenosum, Phytolacca Americana Root, Phytolacca Decandra, Phytonadione, Picric Acid, Picricum Acidum, Pilocarpine Hydrochloride, Pilocarpus, Pimecrolimus, Pimobendan, Pindolol, Pioglitazone, Piperacillin, Piperazine, Piperonyl, Pirlimycin, Piroxicam, Pituitary Luteinizing Hormone, Pix Liquida, Platinum, Plerixafor, Plumbum, Podofilox, Podophyllum, Podophyllum Resin, Poloxalene, Polyehtylene, Polymyxin, Polyoxyethylene, Polyporus Pinicola, Polysorbate 80, Polysulfated Glycosaminoglycan, Polyvinyl Alcohol, Ponazuril, Poractant Alfa, Porcine, Posaconazole, Potassium, Pothos Foetidus, Povidone, Pradofloxacin, Pralidoxime Chloride, Pramipexole, Pramlintide, Pramoxine, Prasugrel, Pravastain, Praziquantel, Prazosin, Prednicarbate, Prednisolone, Prednisone, Pregabalin, Prilocaine, Primaquine, Primidone, Privet, Probenecid, Procainamide, Prochlorperazine, Progesterone, Proguanil, Proline, Promazine, Promethazine, Propafenone, Propiopromazine, Propofol, Propoxyphene, Propranolol, Propylene, Propylhexedrine, Propylthiouracil, Prostalene, Protriptyline, Providone Iodine, Prunus Spinosa, Pseudoephedrine, Pullularia Pullulans, Pulsatilla, Pyrantel, Pyrazinamide, Pyrethrum, Pyridostigmine, Pyridoxine, Pyrilamine, Pyrimethamine, Pyrithione Zinc, Pyrogenium, Quassia Amara, Quetiapine, Quinapril, Quinidine, Rabacfosadine, Rabeprazole, Racepinephrine, Ractopamine, Radium, Raloxifene, Raltegravir, Ramipril, Ramucirumab, Ranitidine, Raphanus Sativus, Rasagiline, Rasburicase, Ratanhia, RauwolfiaSerpentina, Recombinant, Regadenoson, Repaglinide, Reserpine, Resorcinol, Retapamulin, Rheum Officinale, Rhodium, RhusGlabra, Rice, Ribavirin, Ribociclib, Riboflavin, RicinusCommunis, Rifabutin, Rifampin, Rifapentine, Riluzole, Rimabotulinumtoxinb, Rimantadine, Rimexolone, Risedronate, Risperidone, Ritonavir, Rivastigmine, Rizatriptan, Robenacoxib, Robenidine, Robinul, Rocuronium, Roflumilast, Romifidine, Ropinirole, Ropivacaine, Rosiglitazone Maleate, Rosuvastatin Calcium, Roxarsone, Rubella, Rubidium, Rue, Sabadilla, Sabal Serrulata, Sabina, Saccharomyces Cerevisiae, Saccharum Lactis, Sacubitril, Salicyclic Acid, Salicylamide, Saline, Salinomycin, Salix Nigra, Salmeterol, Salsalate, Samarium SM 153 Lexidronam, Santoninum, Saquinavir Mesylate, Sarcolacticum AcidumSargramostim, Sarocladium Strictum, Sarolaner, Sarracenia Purpurea, Sarsaparilla, Saxagliptin, Schizochytrium Dha Oil, Scopalamine, Scopolamine, Scrophularia Nodosa, Scutellaria Lateriflora, Secale Cornutum, Secobarbital, Secukinumab, Selamectin, Selan, Selegiline, Selenium, Selenomethionine, Semduramicin, Sennosides, Serine, Sertaconazole, Sertraline, Sevelamer Carbonate, Sevoflurane, Shark Liver Oil, Sildenafil, Silica, Silicon, Silver, Simethicone, Simvastatin, Sinapis Nigra, Sincalide, Sinecatechins, Sirolimus, Sitagliptin, Skatolum, Sodium, Solenopsis Invicta, Somatropin, Sonidegib, Sorbitol, Sotalol, Spectinomycin, Spigelia, Spinosad, Spironolactone, Spongia Tosta, Stannous, Stanozolol, Staphysagria, Starch, Stavudine, Stellaria Media, Sticta Pulmonaria, Stigmata Maidis, Stramonium, Streptomycin, Streptozocin, Strontium, Strophanthus Hispidus, Succinylcholine, Sucralfate, Sufentanil, Sugammadex, Sulbactam, Sulconazole, Sulfabromomethazine, Sulfacetamide, Sulfachlorpyridazine, Sulfadiazine, Sulfadimethoxine, Sulfaethoxypyridazine, Sulfamerazine, Sulfamethazine, Sulfamethizole, Sulfamethoxazole, Sulfanilamide, Sulfanitran, Sulfaquinoxaline, Sulfasalazine, Sulfisoxazole, Sulfomyxin, Sulfur, Sulindac, Sulphide Of Antimony, Sulphur, Sumatriptan, Sumatriptan, Succinate, Sumbul, Sunitinib Malate, Suvorexant, Syzygium Jambolanum, Tabaccum, Tabaccum Tall Ragweed Giant, Tacrolimus, Tadalafil, Talc, Taliglucerase Alfa, Tamoxifen Citrate, Tamsulosin Hydrochloride, Tanacetum Vulgare, Tannic Acid, Tapentadol, Taraxacum Officinalis, Tartaremetic, Tartaricum Acidum, Taurine, Tavaborole, Tazarotene, Tazobactam, Tazobactam, Technetium, Telbivudine, Telithromycin, Tellurium Metallicum, Telmisartan, Temazepam, Temozolomide, Temsirolimus, Tenofovir Disoproxil Fumarate, Tepoxalin, Terazosin, Terbinafine, Terbutaline, Terconazole, Terebinthina, Teriparatide, Testosterone, Tetanus, Tetracaine, Tetracycline, Tetrafluoroborate, Tetrahydrozoline, Tetrakis, Teucrium Marum, Thallium, Thallous, Thaspium Aureum, Thea Sinensis, Thenium Closylate, Theophylline, Theridion, Thiabendazole, Thialbarbitone, Thiamin, Thiamine, Thiamylal, Thiopental, Thioridazine, Thiosinaminum, Thiostrepton, Thiotepa, Thiothixene, Thlaspi Bursa-Pastoris, Threonine, Thrombin Human, Thymol, Thymus Serpyllum, Thyroidinum, Tiagabine, Tiamulin, Ticagrelor, Ticarcillin, Ticlopidine, Tigecycline, Tildipirosin, Tiletamine, Tilia Europaea, Tilmicosin, Tiludronate, Timolol Maleate, Tincture Of Benzoin, Tinidazole, Tioconazole, Tiopronin, Tioxidazole, Tipranavir, Titanium, Tizanidine, Tl 201, Tobramycin, Toceranib, Tocopheryl Acid, Succinate, Tofacitinib, Tolazamide, Tolazoline, Tolbutamide, Tolcapone, Tolmetin, Tolnaftate, Tolterodine, Toluene, Topiramate, Topotecan, Toremifene, Torsemide, ToxicodendronPubescensLeaf, Tramadol, Trametinib, Trandolapril, TranexamicAcid, Tranylcypromine, Travoprost, Trazodone, Trenbolone, Tretinoin, Triamcinolone, Triamterene, Triazolam, Tribasic, TricaieTrichlorfon, Trichlormethiazie, TrichloroaceticAcid, Trichophyton, Triclocarban, Triclosan, Trientine, Trifluoperazine, Trifolium, Pratense, Trifolium, Repens, Trihexyphenidyl, Trilostane, Trimeprazine, Trimethadione, Trimethoprim, Tripelennamine, Tripolidine, Trolamine, Tromethamine, Tropicamid, Trospium, Trypsin, Tryptophan, Tulathromycin, Tylosin, Tylvalosin, Tyrosine, Umeclidinium, Undecylenic Acid, Uranium Nitricum, Urea, Ursodiol, Urtica Urens, Ustilago Maidis, Valacyclovir, Valganciclovir H, Valine, Valproate, Valproic Acid, Valsartan, Vancomycin, Vandetanib, Vardenafil, Varenicline, Vasopressin, Vecuronium B, Venetoclax, Venlafaxine, Vilanterol, Vilazodone, Vinca Minor, Vincristine, Vinorelbine, Virginiamycin, Viscum Album, Vitamin A, Vitamin B6, Vitamin C, Vitamin D, Vitamin D3, Vitamin E, Vorapaxar, Voriconazole, Vorinostat, Wal-Zan, Wal-Zyr, Warfarin, Xanthoxylum Fraxineum, Xray, Xylazine, Yohimbine, Yohimbinum, Zafirlukast, Zaleplon, Zanamivir, Zavara, Zeranol, Zidovudine, Zileuton, Zilpaterol, Zinc, Zingiber Officinale, Ziprasidone, Ziv-Aflibercept, Zoalene, Zolazepam, Zoledronic Acid, Zolmitriptan, Zolpidem, Zonisamide, and combinations thereof.

In some embodiments, the active agents may be lisinopril, atorvastatin, levothryroxine, amlodipine, omeprazole, metformin, gabapentin, simvastatin, amoxicillin, hydrochlorothiazide, sertraline, losartian, alprazolam, furosemide, azithromycin, ibuprofen, metoprolol, prednisone, tramadol, fluticasone, montelukast, pantoprazole, escitalopram, and combinations thereof.

In some embodiments, the active agent is selected from analgesic agents, antifungal agents, antibacterial agents, anesthetic agents, anti-inflammatory agents, anti-rosacea agents, vasoconstrictors, anti-acne agents, anti-claudication agents, skin retexturing agents and steroids including, but not limited to, retioinds (retinol, retinal, retinoic acid, retinyl propionate), salicylates (acetyl salicylic acid, methylsalicylate, salicyclic acid), benzoyl peroxide, minocycline, clindamycin hydrochloride, clindamycin phosphate, erythromycin, tetracycline, dicloxacilin, doxycycline, tretinoin, isoretinoin, adapalene, gabapentin, pregabalin, cyclosporine, tacrolimus (FK506), oxymetazoline, brimonidine, tetrahydrozoline, phenylephrine, clopidogrel, prasugrel, ticagrelor, ticlopidine, bimatoprost and other PGE2 inhibitors, tadalafil, clindamycin, cortisone, minoxidil, minoxidil sulfate, niacinamide, , gabapentin, hydrocortisone, palmitoyl-KTTKS peptide, phenytoin, vitamin B12, cyclobenzaprine, anastrozole, lidocaine, minocycline, gentamicin sulfate, bimatoprost, minoxidil sulfate, clobetasol propionate, ascorbic acid, tranexamic acid, salicylic acid (sodium salicylate), hydroquinone, Renokin^{®}, tolfnaftate, clotrimazole, terbinafine, isotretinoin, trentinoin, kojic acid, prednisone, a sunscreen actives such as homosalate, octisalate, octocrylene, or avobenzone, hydrocortisone, lidocaine, ixekizumab taltz, aminolevulinic acid (ALA), baricitinib, tofacitinib, adalimumab, citronella oil, 3(N-butyl-N-acetyl)aminopropionic acid ethyl ester, sarecycline, D3 analogs, calcineurin inhibitors, meclorethamine, immunization antigens, imiquimod, ibuprofen, celecoxib, diclofenac, sildenafil, cyclopyrox, sarecycline, estrogen, conjugated estrogens (PREMARIN^{®}), potassium hydroxide, podophyllin, cantharidin, imiquimod, nitric acid, oral cimetidine, 5-fluorouracil, bleomycin, DNCB, imiquimod, and trichloroacetic acid, bleomycin, 2,4-dinitrochlorobenzene, fluorouracil, silver nitrate, zinc sulfate, zinc oxide, bacitracin, chlortetracycline, neomycin, mupirocin, polymyxin B, cuprimyxin, furazolidone, gentamycin, lincomycin, cephalosporins, betalactam antibiotics, Iincomycin hydrochloride, tazarotene, vitamin A, acitretin, bexarotene, oxybutynin; vitamin D, vitamin C, vitamin B, vitamin E; sulfur; glucocorticosteroids, corticosteroids, triamcinolone, triamcinolone acetonide, betamethasone, betamethasone 1 7-valerate, betamethasone dipropionate, halcinonide, isoflupredone acetate, flumethasone, fluocinonide, mometasone, fluticasone, fluticasone propionate, prednisolone, becIomet(h)asone, hydrocortisone, cyproterone, drospirenone, estrogen, progestogen, tacrolimus, pimecrolimus, ursolic acid, betulinic acid, moronic acid, oleanolic acid, acyclovir, valaciclovir, famciclovir, penciclovir, docosanol, perillyl alcohol, and combinations thereof.

In some embodiments, the active agent may be a for veterinary use. Such agents include, but are not limited to, 2-mercaptobenzothiazole, acepromazine maleate, acetazolamide sodium, acetylsalicylic acid, afoxolaner, aklomide, albendazole, albuterol sulfate, alfaxalone, altrenogest, amikacin sulfate, aminopentamide hydrogen sulfate, aminopropazine fumarate, amitraz, ammonium chloride, amoxicillin trihydrate, amphomycin calcium, ampicillin anhydrous, ampicillin sodium, ampicillin trihydrate, amprolium, apramycin sulfate, arsenamide sodium, atipamezole hydrochloride, atropine, attapulgite, avilamycin, azaperone, bacitracin methylene disalicylate, bacitracin zinc, balsam peru oil, bambermycins, beta-aminopropionitrile, betamethason valerate, betamethasone acetate, betamethasone dipropionate, betamethasone sodium phosphate, betamethasone valerate, bismuth subcarbonate, boldenone undecylenate, bovine somatotropin (sometribove zinc), bunamidine hydrochloride, bupivacaine, buprenorphine, buquinolate, butacaine sulfate, butamisole hydrochloride, butorphanol tartrate, cambendazole, capromorelin, caramiphen edisylate, carbadox, carbomycin, carbon dioxide, carfentanil citrate, carnidazole, carprofen, castor oil, cefadroxil, cefovecin sodium, cefpodoxime proxetil, ceftiofur crystalline free acid, ceftiofur hydrochloride, ceftiofur sodium, cephalexin, cephapirin benzathine, cephapirin sodium, chloral hydrate, chloramine-t trihydrate, chloramphenicol, chloramphenicol palmitate, chlorhexidine acetate, chlorhexidine hydrochloride, chlorobutanol, chloroquine phosphate, chlorothiazide, chlorphenesin carbamate, chlortetracycline, chlortetracycline bisulfate, chlortetracycline calcium complex, chlortetracycline hydrochloride, chorionic gonadotropin, chymotrypsin, citric acid, clavulanate potassium, clenbuterol hydrochloride, clindamycin hydrochloride, clodronate, clomipramine hydrochloride, clopidol, cloprostenol sodium, clorsulon, clotrimazole, cloxacillin benzathine, cloxacillin sodium, colistimethate sodium, colloidal ferric oxide, copper naphthenate, corticotropin, coumaphos, cupric glycinate, cyclosporine, cyclosporine oral solution, cythioate, danofloxacin, decoquinate, deracoxib, deslorelin acetate, desoxycorticosterone pivalate, detomidine hydrochloride, dexamethasone, dexamethasone sodium phosphate, dexamethasone-21-isonicotinate, dexmedetomidine, dexmedetomidine hydrochloride, dextrose, diatrizoate meglumine, diatrizoate sodium, dibucaine hydrochloride, dichlorophene, dichlorvos, diclazuril, diclofenac sodium, dicloxacillin sodium monohydrate, diethylcarbamazine citrate, difloxacin hydrochloride, dihydrostreptomycin sulfate, dimethyl sulfoxide, dinoprost tromethamine, dipiperazine sulfate, diprenorphine hydrochloride, dirlotapide, dithiazanine iodide, domperidone, doramectin, doxapram hydrochloride, doxycycline hyclate, doxylamine succinate, droperidol, efrotomycin, embutramid, emodepside, enalapril maleate, enrofloxacin, eprinomectin, epsiprantel, erythromycin, erythromycin phosphate, erythromycin thiocyanate, estradiol, estradiol benzoate, estradiol valerate, estriol, ethopabate, ethylisobutrazine hydrochloride, etodolac, etorphine hydrochloride, famphur, febantel, fenbendazole, fenprostalene, fentanyl, fentanyl citrate, fenthion, firocoxib, florfenicol, flumethasone, flumethasone acetate, flunixin meglumine, fluocinolone acetonide, fluoxetine hydrochloride, fluprostenol sodium, fluralaner, follicle stimulating hormone, formalin, furazolidone, furosemide, gamithromycin, gelatin, gentamicin sulfate, gentamicin sulfate usp, gleptoferron, glycine, glycopyrrolate, gonadorelin acetate, gonadorelin diacetate tetrahydrate, gonadorelin hydrochloride, gonadotropin releasing factor - diphtheria toxoid conjugate, grapiprant, griseofulvin, guaifenesin, halofuginone hydrobromide, halothane, haloxon, helium, hemoglobin glutamer-200 (bovine), hetacillin potassium, hyaluronate sodium, hydrochloride, hydrochlorothiazide, hydrocortisone, hydrocortisone aceponate, hydrocortisone acetate, hydrogen peroxide, hygromycin b, imidacloprid, imidocarb dipropionate, iodinated casein, iodochlorhydroxyquin, iron dextran complex, isoflupredone acetate, isoflurane, isopropamide iodide, itraconazole, ivermectin, kanamycin sulfate, ketamine, ketamine hydrochloride, ketoprofen, laidlomycin propionate potassium, lasalocid, lasalocid sodium, levamisole, levamisole hydrochloride, levamisole phosphate, levamisole resinate, levothyroxine sodium, lidocaine, lincomycin, lincomycin hydrochloride, lincomycin hydrochloride monohydrate, liothyronine sodium, lufenuron, luprostiol, maduramicin ammonium, magnesium sulfate, marbofloxacin, maropitant, mebendazole, meclofenamic acid, medetomidine, medical air, megestrol acetate, melarsomine dihydrochloride, melatonin, melengestrol acetate, meloxicam, mepivacaine hydrochloride, methenamine mandelate, methocarbamol, methylprednisolone, methylprednisolone acetate, metoserpate hydrochloride, mibolerone, miconazole nitrate, milbemycin oxime, mometasone furoate, mometasone furoate anhydrous, mometasone furoate monohydrate, monensin, monensin sodium, monensin usp, morantel tartrate, moxidectin, mupirocin, myristyl-gamma- picolinium chloride, nalorphine hydrochloride, naltrexone hydrochloride, naproxen, narasin, n-butyl chloride, n-butylscopolammonium bromide, neomycin, neomycin palmitate, neomycin sulfate, neostigmine methylsulfate, nequinate, nf, nicarbazin, nitenpyram, nitrofurazone, nitrogen, nitromide, nitrous oxide, norgestomet, novobiocin, novobiocin sodium, nystatin, oclacitinib, oleate sodium, omeprazole, opafp-ghc2 rdna construct, orbifloxacin, orgotein, ormetoprim, oxfendazole, oxibendazole, oxygen, oxytetracycline, oxytetracycline (monoalkyl trimethyl ammonium salt), oxytetracycline dihydrate, oxytetracycline hydrochloride, oxytocin, pegbovigrastim, penicillin g benzathine, penicillin g potassium, penicillin g procaine, penicillin v potassium, pentazocine lactate, pentobarbital, pentobarbital sodium, pergolide mesylate, phenothiazine, phenylbutazone, phenylpropanolamine hydrochloride, phenytoin sodium, phosmet, pimobendan, piperazine citrate, piperazine dihydrochloride, piperazine hydrochloride, piperazine monohydrochloride, piperazine phosphate, piperazine-carbon disulfide complex, pirlimycin hydrochloride, pituitary luteinizing hormone, poloxalene, polymyxin b sulfate, polyoxyethylene 23 lauryl ether, polysulfated glycosaminoglycan, ponazuril, porcine insulin zinc, porcine pituitary-derived follicle stimulating hormone, posaconazole, potassium, potassium citrate, potassium phosphate, pradofloxacin, pralidoxime chloride, praziquantel, prednisolone, prednisolone acetate, prednisolone sodium phosphate, prednisolone sodium succinate, prednisolone tertiary butylacetate, prednisone, primidone, prochlorperazine dimaleate, prochlorperazine edisylate, prochlorperazine maleate, progesterone, promazine hydrochloride, proparacaine hydrochloride, propiopromazine hydrochloride, propofol, prostalene, pyrantel pamoate, pyrantel tartrate, pyrilamine maleate, pyrimethamine, rabacfosadine, ractopamine hydrochloride, robenacoxib, robenidine hydrochloride, romifidine hydrochloride, roxarsone, salinomycin sodium, sarolaner, secobarbital sodium, selamectin, selegiline hydrochloride, selenium disulfide, semduramicin sodium, semduramicin sodium biomass, serum gonadotropin, sevoflurane, silver sulfadiazine, sodium chloride, sodium selenite, sodium sulfachloropyrazine monohydrate, sodium sulfachlorpyridazine, sodium sulfamethazine, spectinomycin, spectinomycin dihydrochloride pentahydrate, spectinomycin hydrochloride pentahydrate, spectinomycin sulfate tetrahydrate, spinosad, stanozolol, streptomycin sulfate, sulfabromomethazine sodium, sulfachlorpyridazine, sulfadiazine, sulfadiazine sodium, sulfadimethoxine, sulfaethoxypyridazine, sulfamerazine, sulfamethazine, sulfamethazine bisulfate, sulfamethizole, sulfanitran, sulfaquinoxaline, sulfaquinoxaline sodium, sulfisoxazole, sulfomyxin, tepoxalin, terbinafine, testosterone propionate, tetracaine hydrochloride, tetracycline, tetracycline hydrochloride, tetracycline phosphate, thenium closylate, thiabendazole, thialbarbitone sodium, thiamylal sodium, thiopental sodium, thiostrepton, thyroid stimulating hormone, tiamulin, tiamulin hydrogen fumarate, ticarcillin disodium, tildipirosin, tiletamine hydrochloride, tilmicosin phosphate, tiludronate disodium, tioxidazole, toceranib phosphate, tolazoline hydrochloride, tolnaftate, toluene, trenbolone acetate, triamcinolone acetonide, tricaine methanesulfonate, trichlorfon, trichlormethiazide, triflupromazine hydrochloride, trilostane, trimeprazine tartrate, trimethoprim, tripelennamine hydrochloride, triptorelin acetate, trypsin, tulathromycin, tylosin, tylosin phosphate, tylosin tartrate, tylvalosin, tylvalosin tartrate, clotrimazole, virginiamycin, vitamin E, xylazine, xylazine hydrochloride, yohimbine hydrochloride, zeranol, zilpaterol, zilpaterol hydrochloride, zinc gluconate, zoalene, and zolazepam hydrochloride.

In some embodiments, the active agents may be antigens used for immunizations against malaria, rabies, anthrax, tuberculosis, hepatitis B, diptheria, pertussis, tetanus, Haemophilus influenza type b, poliovirus, measles, mumps, rubella, varicella, pneumococcus, hepatitis A, influenza, and combination thereof.

In some embodiments, the active agents may be protein, a peptide, an antigen, an antibody, an antibody fragment, a nucleic acid, a dye, a radioactive tracer, a contrast agent, an organic compound, or an inorganic compound.

### Antigens/Antibody Fragments

In some embodiments, the active agent may be an antigen. An antigen can be derived from a pathogen that can infect a subject. Thus, antigens can be derived from, for example, bacteria, viruses, fungi, or parasites. The antigen can be a tumor antigen. The antigen can be an allergen including, but not limited to, pollen, animal dander, mold, dust mite, flea allergen, salivary allergen, grass, or food (e.g., peanuts and other nuts). The antigen can be an autoantigen. The autoantigen can be associated with an autoimmune disease such as, for example, the pancreatic islet antigen.

Non-limiting examples of bacterial antigens include antigens derived from anthrax, Campylobacter, Vibrio cholera, clostridia including Clostridium difficile, Diphtheria, enterohemorrhagic E. coli, enterotoxgenic E. coli, Giardia, gonococcus, Helicobacter pylori, Hemophilus influenza B, Hemophilus influenza non-typeable, Legionella, meningococcus, Mycobacteria including those organisms responsible for tuberculosis, pertussis, pneumococcus, salmonella, shigella, staphylococcus, Group A beta-hemolytic streptococcus, Streptococcus B, tetanus, Borrelia burgdorfi, Yersinia, and the like. According to the present invention, bacterial antigens include, for example, toxins, toxoids (i.e., chemically inactivated toxins, which are less toxic but retain immunogenicity), subunits or combinations thereof, and virulence or colonization factors. Bacterial constituents, products, lysates and/or extracts can be used as a source for bacterial antigens.

Antigens can be derived from viruses. Viral antigens include, but are not limited to, antigens derived from adenovirus, dengue serotypes 1 to 4 virus, ebola virus, enterovirus, hanta virus, hepatitis virus serotypes A to E, herpes simplex virus 1 or 2, human immunodeficiency virus, human papilloma virus, influenza virus, measles (rubeola) virus, Japanese equine encephalitis virus, papilloma virus, parvovirus B19, poliovirus, rabies virus, respiratory syncytial virus, rotavirus, St. Louis encephalitis virus, vaccinia virus, yellow fever virus, rubella virus, chickenpox virus, varicella virus, and mumps virus. Viral constituents, products, lysates and/or extracts can be used as a source for the viral antigens.

In some embodiments, the antigen may be a tumor antigen. The tumor antigen, without limitation, includes stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor.

In some embodiments, the active agent may be an adjuvant. The term "adjuvant" refers to a substance that is used to specifically or nonspecifically potentiate an antigen-specific immune response. The term "adjuvant activity" is the ability to increase the immune response to an antigen (i.e., an antigen which is a separate chemical structure from the adjuvant) by inclusion of the adjuvant in a composition or as part of a method. Adjuvants include, but are not limited to, an oil emulsion (e.g., complete or incomplete Freud's adjuvant), chemokines (e.g., defensins, HCC-1, HCC-4, MCP-1, MCP-3, MCP-4, MIP-1α, MIP-1β, MIP-1δ, MIP-3 α, and MIP-2); other ligands of chemokine receptors (e.g., CCR-1, CCR-2, CCR-5, CCR-6, CXCR-1); cytokines (e.g., IL-1, IL-2, IL-6, IL-8, IL-10, IL-12, IFN-γ; TNF- α, GM-CSF); other ligands of receptors for these cytokines, immunostimulatory CpG motifs of bacterial DNA or oligonucleotides; muramyl dipeptide (MDP) and derivatives thereof (e.g., murabutide, threonyl-MDP, muramyl tripeptide); heat shock proteins and derivatives thereof; Leishmania homologs and derivatives thereof; bacterial ADP-ribosylating exotoxins, chemical conjugates and derivatives thereof (e.g., genetic mutants, A and/or B subunit-containing fragments, chemically toxoid versions); or salts (e.g., aluminum hydroxide or phosphate, calcium phosphate).

In some embodiments, the active agent may be one or more antibodies, and antibody fragments. For example, the antibody may be a therapeutic antibody or a diagnostic antibody. Therapeutic antibodies include, but not limited to antibodies recognizing antigens of stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor. Examples include, but not limited to Abciximab; Abatacept; Adalimumab; Abrilumab; Afutuzumab; Aflibercept; Alemtuzumab; Alefacept; Alacizumab pegol; Anakinra; Arcitumomab; Atacicept; Atlizumab; Atorolimumab; Basiliximab; Baminercept; Bectumomab; Belimumab; Besilesomab; Bevacizumab; Biciromab; Belatacept; Brentuximab vedotin; Brodalumab; Canakinumab; Capromab pendetide; Catumaxomab; Certolizumab pegol; Cetuximab; Clivatuzumab tetraxetan; Daclizumab; Denosumab; Eculizumab; Edrecolomab; Efalizumab; Efungumab; Eloctate; Ertumaxomab; Etanercept; Etaracizumab; Fanolesomab; Farletuzumab; Fontolizumab; Gemtuzumab ozogamicin; Girentuximab; Golimumab; Ibritumomab tiuxetan; Igovomab; Imciromab; Infliximab; Ipilimumab; Labetuzumab; Mepolizumab; Motavizumab; Muromonab-CD3; Natalizumab; Nimotuzumab; Nofetumomab merpentan; Obinutuzumab; Ofatumumab; Omalizumab; Oregovomab; Palivizumab; Panitumumab; Pemtumomab; Pertuzumab; Ramucirumab; Ranibizumab; Raxibacumab; Rituximab; Rilonacept; Rovelizumab; Ruplizumab; Sulesomab; Tacatuzumab tetraxetan; Tefibazumab; Tocilizumab; Trastuzumab; Ado-Trastuzumab Emtansine; Tositumomab; TRBS07; Ustekinumab; Vedolizumab; Visilizumab; Votumumab; Zalutumumab; Zanolimumab, and fragments thereof.

In some embodiments, the active agent may be a diagnostic antibody. Diagnostic antibody includes, but not limited to antibodies against a tumor antigen, a cancer antigen, an allergen, a bacterial antigen, a viral antigen, a drug, a hormone, a plant lectin, an endotoxin, and combinations thereof. In some embodiments, the disclosed antibody and its fragments can also be coupled to toxins, chemotherapeutic drugs, a radiolabel, using methods known in the art, including but not limited to carbodiimide conjugation, esterification, sodium periodate oxidation followed by reductive alkylation, and glutaraldehyde crosslinking.

The diagnostic antibodies and its fragments may include a detectable moiety for detection. The term "detectable moiety" refers to incorporation of another molecule in the antibody, such as but not limited to chromophores, fluorescent moieties, enzymes, antigens, groups with specific reactivity, chemiluminescent moieties, and electrochemically detectable moieties, etc. In some embodiments, the antibodies are biotinylated.

In some embodiments, the antibodies and its fragments disclosed herein can be labeled with a detectable moiety. The detectable moiety can be any one that is capable of producing, either directly or indirectly, a detectable signal. Examples of detectable moieties for antibodies include, but are not limited to, the following: radioisotopes or radionuclides (e.g., 3H, 14C, 15N, 35S, 90Y, 99Tc, 111In, 125I, 131I), fluorescent labels (e.g., FITC, rhodamine, lanthanide phosphors), enzymatic labels (e.g., horseradish peroxidase, beta-galactosidase, luciferase, alkaline phosphatase), chemiluminescent markers, biotinyl groups, predetermined polypeptide epitopes recognized by a secondary reporter (e.g., leucine zipper pair sequences, binding sites for secondary antibodies, metal binding domains, epitope tags), magnetic agents, such as gadolinium chelates, toxins such as pertussis toxin, taxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. In some embodiments, labels are attached by spacer arms of various lengths to reduce potential steric hindrance. Such antibodies and their fragments may be used for diagnostic applications, including but not limited to detection applications and imaging applications.

In some embodiments, a detectable moiety comprises a fluorophore. Any fluorophore can be employed with any antibody disclosed herein, provided that the conjugation of fluorophore results in a composition that is detectable either in vivo (e.g., after administration to a subject) and/or in vitro, and further does not negatively impact the ability of the antibody fragment to bind to its epitope. Representative fluorophores include, but are not limited to 7-dimethylaminocoumarin-3-carboxylic acid, dansyl chloride, nitrobenzodiazolamine (NBD), dabsyl chloride, cinnamic acid, fluorescein carboxylic acid, Nile Blue, tetramethylcarboxyrhodamine, tetraethylsulfohodamine, 5-carboxy-X-rhodamine (5-ROX), and 6-carboxy-X-rhodamine (6-ROX). It is understood that these representative fluorophores are exemplary only, and additional fluorophores can also be employed. For example, there the ALEXA FLUOR^{®} dye series includes at least 19 different dyes that are characterized by different emission spectra. These dyes include ALEXA FLUOR^{®} 350, 405, 430, 488, 500, 514, 532, 546, 555, 568, 594, 610, 633, 635, 647, 660, 680, 700, and 750 (available from Invitrogen Corp., Carlsbad, California, United States of America), and the choice of which dye to employ can be made by the skilled artisan after consideration of the instant specification based on criteria including, but not limited to the chemical compositions of the specific ALEXA FLUOR^{©}, whether multiple detectable moieties are to be employed and the emission spectra of each, the detection technique to be employed, etc.

In some embodiments, a detectable moiety comprises a cyanine dye. Non-limiting examples of cyanine dyes that can be conjugated to the antibody fragments of the presently disclosed subject matter include the succinimide esters CyS, CyS.5, and Cy7, supplied by Amersham Biosciences (Piscataway, New Jersey, United States of America).

In some embodiments, a detectable moiety comprises a near infrared (NIR) dye. Non- limiting examples of near infrared dyes that can be conjugated to the antibody fragment of the presently disclosed subject matter include NIR641 , NIR664, NIT7000, and NIT782.

In some embodiments, the biotinylated antibodies are detected using a secondary antibody that comprises an avidin or streptavidin group and is also conjugated to a fluorescent label including, but not limited to Cy3, Cy5, Cy7, and any of the ALEXA FLUOR^{®}1^{®} series of fluorescent labels available from INVITROGEN^{™} (Carlsbad, California, United States of America). In some embodiments, the antibody fragment is directly labeled with a fluorescent label and cells that bind to the antibody fragment are separated by fluorescence-activated cell sorting. Additional detection strategies are known to the skilled artisan.

### Tracer/Contrast Agent

In some embodiments, the active agent may be a radioactive tracers, such as but not limited to ²⁴¹Am, ⁵¹Cr, ⁶⁰Co, ⁶⁴Cu, ¹⁸F, ⁶⁷Ga, ¹⁹⁸Au, ¹¹³In, ¹¹¹In, ¹³¹I, ¹²⁵I, ⁵⁹Fe, ⁸⁵Kr, ¹⁹⁷Hg, ³²P, ⁴²K, ⁷⁵Se, ²⁴Na, ⁹⁹Tc, ¹³³Xe, ⁹⁰Y, ³H, ¹⁴C, ³⁵S, ¹⁵N, and ¹⁶⁹Y.

In some embodiments, the active agent may be a contrast agent used in imaging. Non-limiting examples of contrast agents include radiopaque contrast agents, paramagnetic contrast agents, superparamagnetic contrast agents, optical imaging moieties, CT contrast agents and other contrast agents. For example, radiopaque contrast agents (for X-ray imaging) will include inorganic and organic iodine compounds (e.g., diatrizoate), radiopaque metals and their salts (e.g., silver, gold, platinum and the like) and other radiopaque compounds (e.g., calcium salts, barium salts such as barium sulfate, tantalum and tantalum oxide). Suitable paramagnetic contrast agents (for MR imaging) include gadolinium diethylene triaminepentaacetic acid (Gd-DTPA) and its derivatives, and other gadolinium, manganese, iron, dysprosium, copper, europium, erbium, chromium, nickel and cobalt complexes, including complexes with 1,4,7,10-tetraazacyclododecane-N,N',N",N‴-tetraacetic acid (DOTA), ethylenediaminetetraacetic acid (EDTA), 1,4,7,10-tetraazacyclododecane -N,N',N"-triacetic acid (DO3A), 1,4,7-triazacyclononane-N,N',N"-triacetic acid (NOTA), 1,4,8,11-tetraazacyclotetradecane-N,N',N",N‴-tetraacetic acid (TETA), hydroxybenzylethylene-diamine diacetic acid (HBED) and the like. Suitable superparamagnetic contrast agents (for MR imaging) include magnetites, superparamagnetic iron oxides, monocrystalline iron oxides, particularly complexed forms of each of these agents that can be attached to a negatively charged backbone. Still, other suitable imaging agents are the CT contrast agents including iodinated and noniodinated and ionic and nonionic CT contrast agents, as well as contrast agents such as spin-labels or other diagnostically effective agents. Other stains and dyes that may be present in the composition include but not limited to congo red, acid fuchsin, acridine orange, basic fuchsin, giemsa stain, methyl green, methylene blue, neutral red, orange-G, and Orcein.

### Neurotoxins

In some embodiments, the active agent is a neurotoxin. In some embodiments, a neurotoxin agent may be selected from the group consisting of botulinum toxin type A, botulinum toxin type B, botulinum toxin type C, botulinum toxin type D, botulinum toxin type E, botulinum toxin type F, abobotulinumtoxinA, BTX-A, daxibotulinumtoxinA, incobotulinumtoxinA, onabotulinumtoxinA, rimabotulinumtoxinB, syntaxin, derivatives thereof (including, but not limited to, engineered recombinant neurotoxins, including engineered recombinant botulinum toxins or fragments thereof as set forth in WO 2014/068317 and WO 199807864, pharmaceutically acceptable salts thereof, and combinations thereof.

The compositions may include one or more active agents from about 0.1 wt. % to about 25 wt. %, about 0.1 wt. % to about 20 wt. %, about 0.1 wt. % to about 15 wt. %, about 0.1 wt. % to about 10 wt. %, about 0.1 wt. % to about 8 wt. %, about 0.1 wt. % to about 5 wt. %, about 0.1 wt. % to about 4 wt. %, about 0.1 wt. % to about 3 wt. %, about 0.1 wt. % to about 3 wt. %, or about 0.1 wt. % to about 1 wt. % of the total composition. Specific examples include about 0.1 wt. %, about 0.5 wt. %, about 1 wt. %, about 2 wt. %, about 5 wt. %, about 10 wt. %, about 25 wt. %, and ranges between any two of these values. The weight percentages disclosed herein may be weight-to-weight or weight-to-volume percentages with respect to the total amount of the composition.

In some embodiments, the active agent may be present from about 1 microgram to about 100 milligrams per mL of the composition, about 1 microgram to about 10 milligrams per mL of the composition, about 1 microgram to about 5 milligrams per mL of the composition, about 1 microgram to about 1 milligram per mL of the composition, or about 1 microgram to about 100 micrograms per mL of the composition.

In some embodiments, an effective amount of an active agent, such as a neurotoxin agent in the composition is 0.1 units, 0.2 units, 0.3 units, 0.4 units, 0.5 units, 0.6 units, 0.7 units, 0.8 units, 0.8 units, 0.9 units, 1.0 unit, 2 units, 3, units, 4 units, 5 units, 6 units, 7 units, 8 units, 9 units, 10 units, 11 units, 12 units, 13 units, 14 units, 15 units, 16 units, 17 units, 18 units, 19 units, 20 units, 25 units, 30 units, 35 units, 40 units, 45 units, 50 units, 100 units, 125 units, 150 units, 175 units, 200 units, 225 units, 250 units, 275 units, 300 units, 325 units, 350 units, 375 units, 400 units, 425 units, 450 units, 475 units, 500 units and any individual amount or any ranges between any two of these values.

Particular examples of compositions encompassed by the invention include compositions containing about 0.01 wt. % to about 2.0 wt. % of one or more decoy molecules having an average molecular weight of about 2,000 Da to about 20,000 Da, and active agent such as minocycline, salicylate, lidocaine, sunblock agents, retinol, bimatoprost, steroids, clindamycin, minoxidil, niacinamide, and active agents of similar size and combinations thereof. Other examples of compositions encompassed by the invention include compositions containing about 0.5 wt. % to about 5.0 wt. % of one or more decoy molecules having an average molecular weight of about 2,000 Da to about 30,000 Da, and one or more active agent such as antibiotics, antifungal agents, biologics, antibodies, macromolecule active agents, peptide-based therapeutics, and active agents of similar size and combinations thereof. In some embodiments, the compositions may substantially not include extracellular matrix component, fragments thereof and combinations thereof (the decoy molecule) having a molecular weight above 60,000 Da.

In some embodiments, the compositions include about 0.1 wt. % to about 2.0 wt. % of one or more decoy molecules having an average molecular weight of about 2,000 Da to about 20,000 Da, and about 0. 1 wt. % to about 5 wt. % of one or more of any of the active agents disclosed herein. In some embodiments, the compositions include about 0.1 wt. % to about 2.0 wt. % of one or more decoy molecules having an average molecular weight of about 2,000 Da to about 30,000 Da, and about 0. 1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions may substantially not include extracellular matrix component, fragments thereof and combinations thereof (the decoy molecule) having a molecular weight above 60,000 Da.

In some embodiments, the compositions include about 0.01 wt. % to about 2.0 wt. % of elastin peptide fragments having an average molecular weight of about 2,000 Da to about 20,000 Da, and about 0. 1 wt. % to about 5 wt. % of one or more of any of the active agents disclosed herein. In some embodiments, the compositions include about 0.1 wt. % to about 2.0 wt. % of elastin peptide fragments having an average molecular weight of about 2,000 Da to about 30,000 Da, and about 0. 1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions include about 0.1 wt. % to about 1.5 wt. % of elastin peptide fragments having an average molecular weight of about 2,000 Da to about 20,000 Da, and about 0. 1 wt. % to about 5 wt. % of an active agent. In some embodiments, the compositions include about 0.1 wt. % to about 1 wt. % of elastin peptide fragments having an average molecular weight of about 2,000 Da to about 20,000 Da, and about 0. 1 wt. % to about 5 wt. % of an active agent. In some embodiments, the compositions include about 0.1 wt. % to about 0.5 wt. % of elastin peptide fragments having an average molecular weight of about 2,000 Da to about 20,000 Da, and about 0. 1 wt. % to about 5 wt. % of an active agent. In some embodiments, the compositions may substantially not include elastin peptide fragments having a molecular weight above 60,000 Da.

In some embodiments, the compositions include about 0.01 wt. % to about 2.0 wt. % of hyaluronic acid having an average molecular weight of about 2,000 Da to about 20,000 Da, and about 0. 1 wt. % to about 5 wt. % of one or more of any of the active agents disclosed herein. In some embodiments, the compositions include about 0.1 wt. % to about 2.0 wt. % of hyaluronic acid having an average molecular weight of about 2,000 Da to about 20,000 Da, and about 0. 1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions include about 0.1 wt. % to about 1.5 wt. % of hyaluronic acid having an average molecular weight of about 2,000 Da to about 20,000 Da, and about 0. 1 wt. % to about 5 wt. % of an active agent. In some embodiments, the compositions include about 0.1 wt. % to about 1.0 wt. % of hyaluronic acid having an average molecular weight of about 2,000 Da to about 60,000 Da, and about 0. 1 wt. % to about 5 wt. % of an active agent. In some embodiments, the compositions include about 0.1 wt. % to about 0.5 wt. % of hyaluronic acid having an average molecular weight of about 2,000 Da to about 20,000 Da, and about 0. 1 wt. % to about 5 wt. % of an active agent. In some embodiments, the compositions may substantially not include hyaluronic acid fragments having a molecular weight above 60,000 Da.

In some embodiments, the compositions include about 0.01 wt. % to about 2.0 wt. % of collagen fragments having an average molecular weight of about 2,000 Da to about 20,000 Da, and about 0. 1 wt. % to about 5 wt. % of one or more of any of the active agents disclosed herein. In some embodiments, the compositions include about 0.1 wt. % to about 2.0 wt. % of collagen fragments having an average molecular weight of about 2,000 Da to about 20,000 Da, and about 0. 1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions include about 0.1 wt. % to about 1.5 wt. % of collagen fragments having an average molecular weight of about 2,000 Da to about 30,000 Da, and about 0. 1 wt. % to about 5 wt. % of an active agent. In some embodiments, the compositions include about 0.1 wt. % to about 1.0 wt. % of collagen fragments having an average molecular weight of about 2,000 Da to about 20,000 Da, and about 0. 1 wt. % to about 5 wt. % of an active agent. In some embodiments, the compositions include about 0.1 wt. % to about 0.5 wt. % of collagen fragments having an average molecular weight of about 2,000 Da to about 30,000 Da, and about 0. 1 wt. % to about 5 wt. % of an active agent. In some embodiments, the compositions may substantially not include collagen fragments having a molecular weight above 60,000 Da.

In some embodiments, the compositions include about 0.01 wt. % to about 2.0 wt. % of fibronectin peptide fragments having an average molecular weight of about 2,000 Da to about 20,000 Da, and about 0.1 wt. % to about 5 wt. % of one or more of any of the active agents disclosed herein. In some embodiments, the compositions include about 0.1 wt. % to about 2.0 wt. % of fibronectin peptide fragments having an average molecular weight of about 2,000 Da to about 20,000 Da, and about 0.1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions include about 0.1 wt. % to about 1.5 wt. % of fibronectin peptide fragments having an average molecular weight of about 2,000 Da to about 20,000 Da, and about 0. 1 wt. % to about 5 wt. % of an active agent. In some embodiments, the compositions include about 0.1 wt. % to about 1.0 wt. % of fibronectin peptide fragments having an average molecular weight of about 2,000 Da to about 20,000 Da, and about 0. 1 wt. % to about 5 wt. % of an active agent. In some embodiments, the compositions include about 0.1 wt. % to about 0.5 wt. % of fibronectin peptide fragments having an average molecular weight of about 2,000 Da to about 20,000 Da, and about 0. 1 wt. % to about 5 wt. % of an active agent. In some embodiments, the compositions may substantially not include fibronectin peptide fragments having a molecular weight above 60,000 Da.

In some embodiments, the compositions include about 0.01 wt. % to about 2.0 wt. % of lectin fragments having an average molecular weight of about 2,000 Da to about 20,000 Da, and about 0. 1 wt. % to about 5 wt. % of one or more of any active agents disclosed herein. In some embodiments, the compositions include about 0.1 wt. % to about 2.0 wt. % of lectin fragments having an average molecular weight of about 2,000 Da to about 30,000 Da, and about 0. 1 wt. % to about 5 wt. % of an active agent selected from the group consisting of salicylate, minocycline, lidocaine, clindamycin, minoxidil, niacinamide, dilantin, retinyl propionate, cyclobenzaprine, sildenafil, anastrazole, ascorbic acid, tranexamic acid, hydroquinone, clotrimazole, renokin, tolnaftate, terbinafine, prednisone, tretinoin, sunblock agents, retinol, bimatoprost, steroids, gabapentin, sarecycline, phenytoiun, acetyl salicycluic acid, gentamycin sulfate, clobetasol propionate, kojic acid, imiquimod, antibodies such as ixekizumab, vitamins, naproxen, antioxidants, and combinations thereof. In some embodiments, the compositions include about 0.1 wt. % to about 1.5 wt. % of lectin fragments having an average molecular weight of about 2,000 Da to about 20,000 Da, and about 0. 1 wt. % to about 5 wt. % of an active agent. In some embodiments, the compositions include about 0.1 wt. % to about 1.0 wt. % of lectin fragments having an average molecular weight of about 2,000 Da to about 20,000 Da, and about 0. 1 wt. % to about 5 wt. % of an active agent. In some embodiments, the compositions include about 0.1 wt. % to about 0.5 wt. % of lectin fragments having an average molecular weight of about 2,000 Da to about 20,000 Da, and about 0. 1 wt. % to about 5 wt. % of an active agent. In some embodiments, the compositions may substantially not include lectin fragments having a molecular weight above 60,000 Da.

### Sensation Modifying Agents

In some embodiments, the active agent is a sensation modifying agent selected from the group of a cooling agent, a warming agent, a relaxing or soothing agent, a stimulating or refreshing agent, and mixtures there of. These sensation modifying agents can be administered or delivered topically along with decoy molecules. The compositions may be formulated as a paste, gel, ointment, lotion, emulsion, cream, foam, mousse, liquid, spray, suspension, dispersion, powder, or aerosol. The compositions with sensation modifying agents can be applied to any surface tissue as disclosed herein.

In some embodiments, the cooling agent is selected from but not limited to menthol; an isomer of menthol, a menthol derivative; 4-Methyl-3-(1-pyrrolidinyl)-2[SH]-furanone; WS-23, Icilin, Icilin Unilever Analog, 5-methyl-4-(1-pyrrolidinyl)-3-[2H]-furanone; 4,5-dimethyl-3-(1-pyrrolidinyl)-2[5H]-furanone; isopulegol, 3-(l-menthoxy)propane-1,2-diol, 3-(l-menthoxy)-2-methylpropane-1,2-diol, p-menthane-2,3-diol, p-menthane-3,8-diol, 6-isopropyl-9-methyl-1,4-dioxas-piro[4,5]decane-2-methanol, menthyl succinate and its alkaline earth metal salts, trimethylcyclohexanol, N-ethyl-2-isopropyl-5-methylcyclohexanecarb-oxamide, Japanese mint (*Mentha arvensis*) oil, peppermint oil, menthone, menthone glycerol ketal, menthyl lactate, 3-(l-menthoxy)ethan-1-ol, 3-(l-menthoxy)propan-1-ol, 3-(l-menthoxy)butan-1-ol, l-menthylacetic acid N-ethylamide, l-menthyl-4-hydroxypentanoate, l-menthyl-3-hydroxybutyrate, N,2,3-trimethyl-2-(1-methylethyl)-butanamide and spearmint oil.

In some embodiments, the warming agent is selected from but not limited to polyhydric alcohols, capsaicin, capsicum powder, a capsicum tincture, capsicum extract, capsaicin, hamamalis, homocapsaicin, homodihydrocapsaicin, nonanoyl vanillyl amide, nonanoic acid vanillyl ether, vanillyl alcohol alkyl ether derivatives, such as vanillyl ethyl ether, vanillyl butyl ether, vanillyl pentyl ether, and vanillyl hexyl ether, isovanillyl alcohol alkyl ethers, ethylvanillyl alcohol alkyl ethers, veratryl alcohol derivatives, substituted benzyl alcohol derivatives, substituted benzyl alcohol alkyl ethers, vanillin propylene glycol acetal, ethylvanillin propylene glycol acetal, ginger extract, ginger oil, gingeol and gingeron.

In some embodiments, the relaxing or soothing agent is selected from but not limited to herb extracts, selected from the group consisting of aloe vera, alpha bisabolol, D-panthenol, allantoin, hamamelis, chamomile, yarrow; calendula, comfrey, witch hazel and other astringents, sea weed, and oat extracts; oils, selected from the group consisting of: almond oil, avocado oil, and comfrey; and essential oils, selected from the group consisting of: cardamone, eucalyptus, *mentha piperita* (peppermint), hyssop, and rosemary; waxy or unctuous substances selected from the group consisting of: lanolin or vaselline jelly, minerals, selected from the group consisting of: zinc oxide, calamine and selenium; vitamins, selected from the group consisting of: tocopheryl acetate (vitamin E), and pharmaceutical agents selected from the group consisting of: analgesics, anesthetics, anti-inflammatory agents, and anti-histamines, and muscle relaxants; menthol, camphor, eugenol, eucalyptol, safrol, methyl salicylate, menthyl lactate, menthyl ethoxyacetate, menthone glycerinacetal, 3-1-menthoxypropane-1,2-diol, ethyl 1-menthyl carbonate, (1S,3S,4R)-p-menth-8-en-3-ol, menthyl pyrrolidone carboxylate, N-substituted-p-menthane-3-carboxamides hamamelis extract and ginger oil.

In some embodiments, the stimulating or refreshing agent is selected from but not limited to an alcohol, L-menthol, camphor, menthe oil, capsicum extract, capsaicin, benzyl nicotinate, salicylate, glycol salicylate, acetyl choline, serotonin, histamine, a prostaglandin, a neurotransmitter; a CNS stimulant, caffeine and quinine.

### Therapeutic Cells

In some embodiments, the active agent is a therapeutic cell. In some embodiments, the therapeutic cell is a stem cell, a genetically engineered mammalian cell, an antigen presenting cell, or a combination thereof.

In some embodiments, the therapeutic cell may be a stem cell. For example, the stem cell may be adult stem cells, tissue-specific stem cells, fetal stem cells, cord blood stem cells, cells derived from the umbilical cord, embryonic stem cells, induced pluripotent stem cells, pluripotent mesenchymal stem cells, multipotent mesenchymal stem cells, hematopoietic stem cells, and combinations thereof. In some embodiments, the mesenchymal stem cells (MSCs), multipotent adult progenitor cells (MAPCs) and/or other stem cells are capable of differentiating into specialized or partially specialized cell types of tissue or organ systems. In some embodiments, the MSCs, MAPCs, and/or other stem cells are genetically engineered or modified to over-express a chemokine and/or chemokine receptor, which can substantially improve the survivability and longevity of the genetically modified MSCs, MAPCs, and/or other stem cells as well as potentially improve the survivability of tissue in which the genetically modified stem cells are introduced. The over-expressed chemokine and/or chemokine receptor can mitigate apoptosis of the genetically modified stem cells when the genetically modified stem cells are introduced into a mammalian subject for therapeutic applications and/or cellular therapy.

In some embodiments, the therapeutic cell is a genetically engineered mammalian cell. The genetically engineered mammalian cell may be configured to express any endogenous or exogenous gene through plasmid constructs, viral constructs, stable integration into chromosomes, or other techniques known in the art. For example, the mammalian cell may be genetically engineered to express a tumor or a cancer antigen. In some embodiments, the tumor antigen, without limitation, includes stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor. In some embodiments, the genetically engineered mammalian cells may be skin cells.

In some embodiments, the therapeutic cell may be an antigen presenting cell, such as T cells, B cells, dendritic cells, and macrophages. In some embodiments, therapeutic cells are engineered immune cells expressing chimeric receptors, such as chimeric antigen receptors (CARs) and transgenic T cell receptors (TCRs). The chimeric receptors, e.g., CARs or transgenic TCRs, generally bind to one or more antigen expressed by, associated with, and/or specific for a disease or condition in the subject and/or cell(s) or tissue(s) thereof. Such diseases may include tumors, cancers, other proliferative diseases, autoimmune diseases or disorders, and/or infectious agents or disease.

In some embodiments, a therapeutic cell may be, for example, a fibroblast, an integument cell, an adipocyte, a pre-adipocyte, a stem cell, an epithelial cell, a retinal cell, an immune function cell, a melanocyte or other pigment cell, a hair follicle cell, a keratinocyte, a Langerhans cell, Merkel cells, mast cells, muscle cells, or a combination thereof. Also contemplated are methods of delivery of muscle cells, bone cells, pancreatic cells, cells of a mucosal membrane, chondrocytes, cells of the nervous system, hormone secreting cells, endocrine cells, intestinal cells, and germ cells.

In some embodiments, the therapeutic cells are autologous cells. Autologous cells can be isolated from tissue in a variety of ways, all which are known to one skilled in the art. In one embodiment, cells can be isolated from a biopsy material by conventional methods. The biopsy material can be extracted from any tissue of the patient relating to the tissue type of the defect or tissue regeneration. By way of example and not by limitation, suitable cells include tenocytes, myocytes, stem cells, osteocytes, chondrocytes, epithelial cells, keratinocytes, nerve cells (including, but not limited to neurocytes, astrocytes, dendritic cells, and glial cells), fibroblasts, odontocytes, synoviocytes, adipocytes, and cementocytes. In addition, precursor cells to these cell types are also useful in the present composition. In one embodiment, for example, myoblasts, which are precursors to myocytes; osteoblasts, which are precursors to osteocytes, and neuroblasts, which are precursors to neurocytes, are all useful in the present disclosure. In some embodiments, the cells and cell precursors are autologous cells and autologous cell precursors.

In some embodiments, the therapeutic cells are allogenic cells. The term "allogeneic" refers to cells, tissue, or organisms that are of different genetic constitution. The stem cells and genetically engineered mammalian cells disclosed herein may be allogenic cells.

In some embodiments, the number of therapeutic cells in the composition may be about 10² cells to about 10⁶ cells per mL of the composition, about 10³ cells to about 10⁶ cells per mL of the composition, about 10⁴ cells to about 10⁶ cells per mL of the composition, about 10⁵ cells to about 10⁶ cells per mL of the composition, about 10³ cells to about 10⁹ cells per mL of the composition, or about 10³ cells to about 10¹⁰ cells per mL of the composition.

### Compositions

The compositions of various embodiments may include any of the active agents identified above or combinations thereof in an effective amount, and one or more of any decoy molecules disclosed herein. The compositions disclosed herein are formulated for topical application, transdermal application, percutaneous application, or by microneedle injection to the skin of a subject. Without intending to be limiting, but for purposes of exemplary embodiments, it is contemplated that the composition may be a paste, gel, ointment, lotion, emulsion, cream, foam, mousse, liquid, spray, suspension, dispersion, powder, or aerosol. The composition includes one or more excipients to provide the desired form and a desired viscosity, flow or other physical or chemical characteristics for effective application, coverage and adhesion to the skin/surface tissue.

In some embodiments, the compositions described herein may further include one or more cosmetic or pharmaceutically acceptable diluents, fillers, disintegrants, binders, lubricants, surfactants, hydrophobic vehicles, water soluble vehicles, emulsifiers, buffers, humectants, moisturizers, solubilizers, preservatives, colorants, plastizers, carriers, excipients, and the like and combinations thereof. The person of ordinary skill in the art can refer to various pharmacologic references such as, for example, Modern Pharmaceutics, Banker & Rhodes, Marcel Dekker, Inc. (1979) and Goodman & Gilman's The Pharmaceutical Basis of Therapeutics, 6th Edition, MacMillan Publishing Co, New York (1980) for guidance in determining the amount of such components in the compositions of embodiments.

Excipients in the composition are selected based on the type of formulation intended. Standard excipients include gelatin, casein, lecithin, gum acacia, cholesterol, tragacanth, stearic acid, benzalkonium chloride, calcium stearate, glyceryl monostearate, cetostearyl alcohol, cetomacrogol emulsifying wax, sorbitan esters, polyoxyethylene alkyl ethers, polyoxyethylene castor oil derivatives, polyoxyethylene sorbitan fatty acid esters, polyethylene glycols, polyoxyethylene stearates, colloidol silicon dioxide, phosphates, sodium dodecyl sulfate, carboxymethylcellulose calcium, carboxymethylcellulose sodium, methylcellulose, hydroxyethylcellulose, hydroxypropylcellulose, hydroxypropylmethycellulose phthalate, noncrystalline cellulose, magnesium aluminum silicate, triethanolamine, polyvinyl alcohol, polyvinylpyrrolidone, sugars, and starches.

"Surfactants" are surface-active agents that lower surface tension and thereby increase the emulsifying, foaming, dispersing, spreading and wetting properties of a product. Suitable non-ionic surfactants include emulsifying wax, glyceryl monooleate, polyoxyethylene alkyl ethers, polyoxyethylene castor oil derivatives, polysorbate, sorbitan esters, benzyl alcohol, benzyl benzoate, cyclodextrins, glycerin monostearate, poloxamer, povidone, or combinations thereof. In one embodiment, the non-ionic surfactant is stearyl alcohol.

"Emulsifiers" are surface active substances which promote the suspension of one liquid in another and promote the formation of a stable mixture, or emulsion, of oil and water. Common emulsifiers are metallic soaps, certain animal and vegetable oils, and various polar compounds. Suitable emulsifiers include acacia, anionic emulsifying wax, calcium stearate, carbomers, cetostearyl alcohol, cetyl alcohol, cholesterol, diethanolamine, ethylene glycol palmitostearate, glycerin monostearate, glyceryl monooleate, hydroxpropyl cellulose, hypromellose, lanolin, hydrous, lanolin alcohols, lecithin, medium-chain triglycerides, methylcellulose, mineral oil and lanolin alcohols, monobasic sodium phosphate, monoethanolamine, nonionic emulsifying wax, oleic acid, poloxamer, poloxamers, polyoxyethylene alkyl ethers, polyoxyethylene castor oil derivatives, polyoxyethylene sorbitan fatty acid esters, polyoxyethylene stearates, propylene glycol alginate, self-emulsifying glyceryl monostearate, sodium citrate dehydrate, sodium lauryl sulfate, sorbitan esters, stearic acid, sunflower oil, tragacanth, triethanolamine, xanthan gum or combinations thereof. In one embodiment, the emulsifier is glycerol stearate.

In some embodiments, the compositions described herein may be formulated as a liquid. Liquid dosage forms for topical administration may include diluents such as, for example, alcohols, glycols, oils, water, and the like. Such compositions may also include wetting agents or emulsifiers. In some embodiments, the compositions of embodiments may be formulated as oil-in-water or water-in-oil emulsion. A cream can be a water-in-oil (w/o) emulsion in which an aqueous phase is dispersed in an oil phase, or an oil-in-water (o/w) emulsion in which an oil is dispersed within an aqueous base. An ointment generally refers to a more viscous oil-in-water cream. Traditional ointment bases (*i.e.* carrier) include hydrocarbons (petrolatum, beeswax, etc.) vegetable oils, fatty alcohols (cholesterol, lanoilin, wool alcohol, stearyl alcohol, etc.) or silicones. Insoluble solids such as starch, zinc oxide, calcium carbonate, or talc can also be used in ointments and creams. Gel forms of the compositions described above can be formed by the entrapment of large amounts of aqueous or aqueous-alcoholic liquids in a network of polymers or of colloidal solid particles. Such polymers or colloids (gelling or thickening agents) are typically present at concentrations of less than 10% w/w and include carboxymethyl cellulose, hydroxypropylmethyl cellulose, hydroxyethyl cellulose, methyl cellulose, sodium alginate, alginic acid, pectin, tragacanth, carrageen, agar, clays, aluminum silicate, carbomers, and the like.

An emulsion is a preparation of one liquid distributed in small globules throughout the body of a second liquid. The dispersed liquid is the discontinuous phase, and the dispersion medium is the continuous phase. When oil is the dispersed liquid and an aqueous solution is the continuous phase, it is known as an oil-in-water emulsion, whereas when water or aqueous solution is the dispersed phase and oil or oleaginous substance is the continuous phase, it is known as a water-in-oil emulsion. The oil phase may consist at least in part of a propellant, such as an HFA propellant. Either or both of the oil phase and the aqueous phase may contain one or more surfactants, emulsifiers, emulsion stabilizers, buffers, and other excipients. Preferred excipients include surfactants, especially non-ionic surfactants; emulsifying agents, especially emulsifying waxes; and liquid non-volatile nonaqueous materials, particularly glycols such as propylene glycol. The oil phase may contain other oily pharmaceutically approved excipients. For example, materials such as hydroxylated castor oil or sesame oil may be used in the oil phase as surfactants or emulsifiers.

A "lotion" is a low- to medium-viscosity liquid composition. A lotion can contain finely powdered substances that are in soluble in the dispersion medium through the use of suspending agents and dispersing agents. Alternatively, lotions can have as the dispersed phase liquid substances that are immiscible with the vehicle and are usually dispersed by means of emulsifying agents or other suitable stabilizers. In one embodiment, the lotion is in the form of an emulsion having a viscosity of between 100 and 1000 centistokes. The fluidity of lotions permits rapid and uniform application over a wide surface area. Lotions are typically intended to dry on the skin leaving a thin coat of their medicinal components on the skin's surface.

A "cream" is a viscous liquid or semi-solid emulsion of either the "oil-in-water" or "water-in-oil type". Creams may contain emulsifying agents and/or other stabilizing agents. In one embodiment, the composition is in the form of a cream having a viscosity of greater than 1000 centistokes, typically in the range of 20,000-50,000 centistokes. Creams are often time preferred over ointments as they are generally easier to spread and easier to remove.

An "ointment" is a semisolid preparation containing an ointment base and optionally one or more active agents. Examples of suitable ointment bases include hydrocarbon bases (e.g., petrolatum, white petrolatum, yellow ointment, and mineral oil); absorption bases (hydrophilic petrolatum, anhydrous lanolin, lanolin, and cold cream); water-removable bases (e.g., hydrophilic ointment), and water-soluble bases (e.g., polyethylene glycol ointments). Pastes typically differ from ointments in that they contain a larger percentage of solids. Pastes are typically more absorptive and less greasy that ointments prepared with the same components.

A "gel" is a semisolid system containing dispersions of small or large molecules in a liquid vehicle that is rendered semisolid by the action of a thickening agent or polymeric material dissolved or suspended in the liquid vehicle. The liquid may include a lipophilic component, an aqueous component or both. Some emulsions may be gels or otherwise include a gel component. Some gels, however, are not emulsions because they do not contain a homogenized blend of immiscible components. Suitable gelling agents include, but are not limited to, modified celluloses, such as hydroxypropyl cellulose and hydroxyethyl cellulose; Carbopol homopolymers and copolymers; and combinations thereof. Suitable solvents in the liquid vehicle include, but are not limited to, diglycol monoethyl ether; alklene glycols, such as propylene glycol; dimethyl isosorbide; alcohols, such as isopropyl alcohol and ethanol. The solvents are typically selected for their ability to dissolve the drug. Other additives, which improve the skin feel and/or emolliency of the composition, may also be incorporated. Examples of such additives include, but are not limited, isopropyl myristate, ethyl acetate, C12-C15 alkyl benzoates, mineral oil, squalane, cyclomethicone, capric/caprylic triglycerides, or combinations thereof.

Emollient or lubricating vehicles that help hydrate the skin can also be used. "Emollients" are an externally applied agent that softens or soothes skin and are generally known in the art and listed in compendia, such as the "Handbook of Pharmaceutical Excipients", 4th Ed., Pharmaceutical Press, 2003. These include, without limitation, almond oil, castor oil, ceratonia extract, cetostearoyl alcohol, cetyl alcohol, cetyl esters wax, cholesterol, cottonseed oil, cyclomethicone, ethylene glycol palmitostearate, glycerin, glycerin monostearate, glyceryl monooleate, isopropyl myristate, isopropyl palmitate, lanolin, lecithin, light mineral oil, medium-chain triglycerides, mineral oil and lanolin alcohols, petrolatum, petrolatum and lanolin alcohols, soybean oil, starch, stearyl alcohol, sunflower oil, xylitol, or combinations thereof. In one embodiment, the emollients are selected from the group consisting of ethylhexylstearate and ethylhexyl palmitate.

In particular embodiments, the compositions described above can be formulated as aerosols in which the composition is dissolved in a propellant such as dichlorodifluoromethane, trichlorofluoromethane, dichlorotetrafluoroethane, carbon dioxide, or other suitable gas, and a co-solvent such ethanol, acetone, hexadecyl alcohol, and the like and combinations thereof.

In certain embodiments, the compositions of various embodiments may be formulated for improving enhance the texture, appearance, color, sensation, or hydration of the skin and may additionally include additives such as vitamins, cosmetic peptides, oil control agents, and other skin care agents.

Vitamins include, for example, vitamin D, vitamin K, vitamin B (including niacinamide, nicotinic acid, C₁₋₁₈ nicotinic acid esters, and nicotinyl alcohol; B6 compounds, such as pyroxidine; and B5 compounds, such as panthenol, or "pro-B5"), vitamin A (including retinoids such as retinyl propionate, carotenoids, and other compounds), vitamin E (including tocopherol sorbate, tocopherol acetate, other esters of tocopherol), vitamin C (including ascorbyl esters of fatty acids, and ascorbic acid derivatives, for example, ascorbyl glucoside, magnesium ascorbyl phosphate, sodium ascorbyl phosphate, and ascorbyl sorbate), and all natural and/or synthetic analogs thereof, and combinations thereof. In various embodiments, the compositions may include about 0.0001 wt. % to about 50 wt. %, about 0.001 wt. % to about 10 wt. %, about 0.01 wt. % to about 5 wt. %, or about 0.1 wt. % to about 1 wt. %, or any individual concentration or range of each vitamin contained in the composition.

Peptides include di-, tri-, tetra-, penta-, and hexa-peptides, their salts, isomers, derivatives, and mixtures thereof. Examples of useful peptide derivatives include, but are not limited to, peptides derived from soy proteins, palmitoyl-lysine-threonine (pal-KT) and palmitoyl-lysine-threonine-threonine-lysine-serine (MATRIXYL^{®}) palmitoyl-glycine-glutamine-proline-arginine (RIGIN^{®}), these three being available from Sederma, France, and Cu-histidine-glycine-glycine (Cu-HGG, also known as IAMIN^{®}), and naturally occurring and synthesized derivatives thereof, and combinations thereof. In various embodiments, the compositions may include about 1 x 10⁻⁷ wt. % to about 20 wt. %, about 1 x 10⁻⁶ wt. % to about 10 wt. %, and about 1 x 10⁻⁵ wt. % to about 5 wt. %, or any individual concentration or range of each peptide contained in the composition.

Oil control agents include compounds useful for regulating the production of skin oil, or sebum, and for improving the appearance of oily skin. Examples of oil control agents include, for example, salicylic acid, dehydroacetic acid, benzoyl peroxide, vitamin B3 (for example, niacinamide), and the like, their isomers, esters, salts and derivatives, and mixtures thereof. The compositions of such embodiments may include about 0.0001 wt. % to about 15 wt. %, about 0.01 wt. % to about 10 wt. %, about 0.1 wt. % to about 5 wt. %, and about 0.2 wt. % to about 2 wt. %, or any individual concentration or range of each oil control agent contained in the composition.

Other skin care agents include retinol, steroids, sunblock, salicylate, minocycline, antifungals, peptides, antibodies, lidocaine, and the like and combinations thereof. In some embodiments, other skin care agents include N-acyl amino acid compounds includinf, for example, N-acyl phenylalanine, N-acyl tyrosine, and the like, their isomers, including their D and L isomers, salts, derivatives, and mixtures thereof. An example of a suitable N-acyl amino acid is N-undecylenoyl-L-phenylalanine is commercially available under the tradename SEPIWHITE^{®}. Other skin active agents include, but are not limited to, Lavandox, Thallasine 2, Argireline NP, Gatuline In-Tense and Gatuline Expression, Myoxinol LS 9736, Syn-ake, and Instensyl^{®}, Sesaflash^{™}, N- acetyl D-glucosamine, panthenol (for example, DL panthenol available from Alps Pharmaceutical Inc.), tocopheryl nicotinate, benzoyl peroxide, 3-hydroxy benzoic acid, flavonoids (for example, flavanone, chalcone), farnesol, phytantriol, glycolic acid, lactic acid, 4-hydroxy benzoic acid, acetyl salicylic acid, 2-hydroxybutanoic acid, 2-hydroxypentanoic acid, 2-hydroxyhexanoic acid, cis- retinoic acid, trans-retinoic acid, retinol, retinyl esters (for example, retinyl propionate), phytic acid, N-acetyl-L-cysteine, lipoic acid, tocopherol and its esters (for example, tocopheryl acetate: DL-a- tocopheryl acetate available from Eisai), azelaic acid, arachidonic acid, tetracycline, ibuprofen, naproxen, ketoprofen, hydrocortisone, acetominophen, resorcinol, phenoxyethanol, phenoxypropanol, phenoxyisopropanol, 2,4,4'-trichloro-2'-hydroxy diphenyl ether, 3,4,4'- trichlorocarbanilide, octopirox, lidocaine hydrochloride, clotrimazole, miconazole, ketoconazole, neomycin sulfate, theophylline, and mixtures thereof. Further skin care agents are disclosed in US Publication No. 2007/0020220A1.

In some embodiments, the compositions can also include skin lightening agents, such as ascorbic acid compounds, vitamin B₃ compounds, azelaic acid, butyl hydroxyanisole, gallic acid and its derivatives, glycyrrhizinic acid, hydroquinone, kojic acid, arbutin, mulberry extract, and mixtures thereof. Use of combinations of skin lightening agents is believed to be advantageous in that they may provide skin lightening benefit through different mechanisms.

In some embodiments, the compositions include sunblock agents, such as but not limited to para-aminobenzoic acid (PABA), PABA esters (glyceryl PABA, amyldimethyl PABA and octyldimethyl PABA), benzophenones (oxybenzone and sulisobenzone), cinnamates (octylmethoxy cinnamate and cinoxate), salicylates (homomethyl salicylate) anthranilates, TiO₂, avobenzone, bemotrizinol, bisoctrizole, 3-(4-methylbenzylidene)-camphor, cinoxate, diethylamino hydroxybenzoyl hexyl benzoate, dioxybenzone, drometrizole trisiloxane, ecamsule, ethylhexyl triazone, homosalate, menthyl anthranilate, octocrylene, octyl salicylate, iscotrizinol, isopentenyl-4-methoxycinnamate, octyl- dimethyl-p-aminobenzoic acid, octyl-methoxycinnamate, oxybenzone, polysilicone-15, trolamine salicylate, and ZnO. In some embodiments, any active agent disclosed herein can be combined with any of the sunblock agents disclosed herein or known in the art.

In some embodiments, the compositions can also include a pH adjusting agent or a buffering agent, which is present in the composition to neutralize and/or activate the thickening polymer in order to facilitate the formation of a composition having the desirable rheological qualities. Any base or buffer system known in the art and suitable for use in a skin contact application can be used. In one embodiment, the base can include triethanolamine, such as solutions of 10% triethanolamine (TEA), alkali metal hydroxides like sodium hydroxide (NaOH), salts of weak acids such as ammonium lactate, sodium citrate, sodium ascorbate, or mixtures thereof.

In some embodiments, the compositions can be in the form of hydrogels. Hydrogels are typically prepared by cross-linking various monomers and/or polymers to provide a three-dimensional polymer network. Non-limiting examples of polymers include, polyoxyethylene-polypropylene block copolymers, ionic poly saccharides, such as chitosan or sodium alginate, cellulose, and biodegradable polymers, such as poly-lactides (PLA) and poly-glycolides (PGA), butylene succinate (PBS), polyhydroxyalkanoate (PHA), polycaprolactone acid lactone (PCL), polyhydroxybutyrate (PHB), glycolic amyl (PHV), PHB and PHV copolymer (PHBV), and poly lactic acid (PLA)-polyethylene glycol (PEG) copolymers (PLEG).

In some embodiments, the compositions disclosed herein may be in the form of sustained release composition. Sustained (or controlled) release refers to the gradual release of active agents from the composition over a period of time. While there may be an initial burst phase, in some embodiments, it is preferred that the release display relatively linear kinetics, thereby providing a constant supply of the active agent over the release period. The release period may vary from 1 to 8 hours, depending upon the skin disorder and its intended use. The compositions may contain various biodegradable polymers to facilitate slow release, such as poly-lactides (PLA), poly-glycolides (PGA), poly butylene succinate (PBS), polyhydroxyalkanoate (PHA), polycaprolactone acid lactone (PCL), polyhydroxybutyrate (PHB), glycolic amyl (PHV), PHB and PHV copolymer (PHBV), and poly lactic acid (PLA)-polyethylene glycol (PEG) copolymers (PLEG). In some embodiments, the preferred polymer is a poloxomer, such as Pluronic^{®} 127.

In some embodiments, the composition may include nanoparticles or microparticles to further facilitate delivery. In some embodiments, both the extracellular matrix component, fragments thereof and combinations (the decoy molecule) and the active agent may be encapsulated within nanoparticles and/or microparticles or may be ionically associated with nanoparticles and/or microparticles. In some embodiments, only the extracellular matrix component, fragments thereof and combinations (the decoy molecule) may be encapsulated within nanoparticles and/or microparticles. In some embodiments, only the active agent may be encapsulated within nanoparticles and/or microparticles. In some embodiments, a fraction of the extracellular matrix component, fragments thereof and combinations (the decoy molecule) and/or the active agent may be encapsulated within nanoparticles and/or microparticles. In some embodiments, the nanoparticles or microparticles may be lipid nanoparticles or lipid microparticles. Such nanoparticles or microparticles can be prepared by forming an emulsion of an active agent, extracellular matrix component, or both dispersed or dissolved in a solvent, and this solution may be combined witht glycerol and poloxomer to form an emulsion. The emulsion can be heated, cooled, and homogenized to produce microparticles or nanoparticles. In some embodiments, the nanoparticles or microparticles may be commercially available, such as, for example, hybrid polyamidoamine (PAMAM) dendrimer hydrogel/poly (lactic-co-glycolic acid) (PLGA) nanoparticles or microparticles (HDNP), chitosan (CS) nanopartices or microparticles, thiolated chitosan nanoparticles or microparticles, calcium phosphate (CaP) nanoparticles or microparticles, poly (lactic-co-glycolic acid) copolymer (PLGA), poly (ethyleneglycol)-block-poly(-caprolactone) nanopolymeric nanoparticles or microparticles, core/shell nanoparticles or microparticles composed of, for example, a lecithin liposome as the core and pluronic F 127 diacrylate (DA-PF 127), inorganically-coated retinoic acid (atRA) nanoparticles or microparticles, poly (lactic acid) (PLA) homopolymers and PEG-block-PLA copolymer nanoparticles or microparticles, PEG-block-PPG copolymers such as Pluronic, PEGylated liposome-protamine-hyaluronic acid nanoparticles or microparticles, polylactic acid/polylactic acid-polyethylene oxide (PLA/PLA-PEO) nanoparticles or microparticles, and the like and combinations thereof. In some embodiments, the nanoparticles may have a diameter of from about 2 to about 200 nanometers, about 5 to about 50 nanometers, or about 18 to about 22 nanometers, or any range or individual value encompassed by these ranges.

In some embodiments, the composition may include liposomes to facilitate delivery. In some embodiments, both the extracellular matrix component, fragments thereof and combinations (the decoy molecule) and the active agent may be encapsulated within liposomes. In some embodiments, only the extracellular matrix component, fragments thereof and combinations (the decoy molecule) may be encapsulated within liposomes. In some embodiments, only the active agent may be encapsulated within liposomes. In some embodiments, a fraction of the extracellular matrix component, fragments thereof and combinations (the decoy molecule) and/or the active agent may be encapsulated within liposomes. Liposomes are well known and commonly used in the pharmaceutical arts, and any type of liposome can be used in the embodiments. In some embodiments, the liposomes may be phosphatidylcholine (PC) and other constituents such as cholesterol and lipid-conjugated hydrophilic polymers. In some embodiments, the liposomes may be chitosan or may be coated in chitosan (i.e., chitosomes). In some embodiments, the compositions may include colloidal lipids. Such compositions may include colloidal polar lipids formed from one or more non-ionic polyethylene glycol derivatives of castor oil and/or hydrogenated castor oil such as, for example, PEG-30 castor oil, PEG-33 castor oil, PEG-36 castor oil, PEG-40 castor oil, PEG-30 hydrogenated castor oil and PEG-40 hydrogenated castor oil, an anionic purified polysaccharide such as Gellan Gum, one or more buffering agents such as, for example, boric acid, trimethamine, and, in some embodiments, one or more aqueous lubricants and one or more colloidal aqueous lubricants. In some embodiments, the liposomes or colloidal lipids may form particles about 1 nanometers to about 50 nanometers or about 6 nanometers to about 22 nanometers. In some embodiments, the compositions may include about 0.1 w/v % to about 15 w/v % lipids.

A wide variety of methods may be used for preparing the compositions described above. Broadly speaking, the compositions may be prepared by combining together the components of the composition, as described herein, at a temperature and for a time sufficient to provide a pharmaceutically acceptable composition. In embodiments of the invention, the compositions components of the compositions are dissolved, suspended, dispersed or otherwise mixed in a selected carrier or vehicle, at an effective concentration such that the condition to be treated is relieved or ameliorated.

Further embodiments are directed to devices including the compositions described above. For example, such compositions can be coated on bandages, mixed with bioadhesives, or included in wound dressings.

### Permeation Enhancers

In some embodiments, the composition can include one or more penetration or permeation enhancers for transdermal drug delivery. Chemical permeation enhancers (CPEs) for transdermal drug delivery. CPEs are known in the art. Suitable CPEs may include, but are not limited to solvents (e.g., monohydric alcohols such as methanol, ethanol, propanol, isopropanol), fatty acids (e.g., oleic acid, caprylic acid), fatty alcohols (e.g. lauryl alcohol, myristyl alcohol, oleyl alcohol), surfactants (e.g., ionic or non-ionic detergents), fatty acid esters (e.g. isopropyl myristate, isopropyl palmitate, methylpropionate, and ethyl oleate), organic acids (e.g. salicylic acid and salicylates, citric acid and succinic acid), nitrogenous compounds (e.g. urea), bile salts and derivatives, micelles/liposomes or micelle-forming or liposome-forming components (e.g., phospholipids), sulfoxides, terpenes and terpenoids, polyols, urea and derivatives, and chelating agents. A non-limiting list of CPEs may include methanol, ethanol, propylene glycol, ethylene glycol, glycerol, butanediol, polyethylene glycol, polyethylene glycol monolaurate, diethylene glycol, monoethyl ether (transcutol), oleic acid, caprylic acid, menthol, nerol, camphor, methyl salicylate, sodium laurate, sodium lauryl sulfate, cetyltrimethylammonium bromide, benzalkonium chloride, poloxamer (231, 182, 184), Tween (20, 40, 60, 80), sodium dodecyl sulfonate (SDS), methylsulfonylmethane (MSM), benzalkonium chloride, polyoxyl 40 hydrogenated castor oil, didecyldimethylammonium bromide (DDAB), didecyltrimethylammonium bromide (DTAB), polysorbates, Na glyacolate, Na deoxycholate, EDTA, citric acid, dimethylacetamide (DMA), DMSO, dimethylformamide (DMF), dimethylsulfoxide, decylmethylsulfoxide, propylene glycol, polyethylene glycol, glycerol, 2-pyrrolidone, 1-methyl-2-pyrrolidone, ethanolamine, diethanolamine, triethanolamine urea, lecithin, terpenes, terpenoids, 1-substituted azacycloheptan-2-ones, such as 1-n-dodecylcyclazacycloheptan-2-one phospholipids, water, and mixtures thereof. More permeation enhancer(s) suitable to be used with the present invention may be known by those skilled in the art. (See e.g., Williams et al., "Penetration enhancers," Adv. Drug Deliv. Rev. 2004 Mar. 27; 56(5):603-18; and Pathan et al., "Chemical Penetration Enhancers for Transdermal Drug Delivery System," Trop. J. Pharma. Res., April 2009; 8(2): 173-179.

The permeation or penetration enhancers can be present from about 0.1 to about 30.0% w/w depending on the type of compound. For example, the penetration enhancers can be included in the formulations of embodiments in a total amount by weight of about 0.1 w/w % to about 15 w/w %, and in various embodiments, the penetration enhancers can be included in a total amount of about 2 w/w % to about 12 w/w %, about 4 w/w % to about 10 w/w %, about 4 w/w % to about 7 w/w %, about 4 w/w % to about 6 w/w %, about 4.5 w/w % to about 5.5 w/w %, about 4 w/w % to about 5 w/w %, or any range or individual concentration within these example ranges.

In some embodiments, the penetration or permeation enhancer may be a non-ionizable glycol ether. Non-ionizable glycol ethers include, for example, diethylene glycol monomethyl ether, triethylene glycol monomethyl ether, polyethylene glycol monomethyl ether, ethylene glycol monoethyl ether, diethylene glycol monoethyl ether, triethylene glycol monoethyl ether, ethylene glycol monoisopropyl ether, ethylene glycol monobutyl ether, diethylene glycol monobutyl ether, triethylene glycol monobutyl ether, ethylene glycol monoisobutyl ether, diethylene glycol monohexyl ether, ethylene glycol mono 2-ethylhexyl ether, diethylene glycol mono 2-ethylhexyl ether, ethylene glycol monoallyl ether, ethylene glycol monophenyl ether, ethylene glycol monobenzyl ether, diethylene glycol monobenzyl ether, propylene glycol monomethyl ether, dipropylene glycol monomethyl ether, tripropylene glycol monomethyl ether, dipropylene glycol monopropyl ether, propylene glycol monobutyl ether, dipropylene glycol monobutyl ether, propylene glycol monophenyl ether, ethylene glycol dimethyl ether, diethylene glycol dimethyl ether, triethylene glycol dimethyl ether, diethylene glycol diethyl ether, diethylene glycol dibutyl ether, dipropylene glycol dimethyl ether, and the like and combinations thereof. The glycol portion of these non-ionizable glycol ether include a broad range of chemicals including, but not limited to, propylene glycol, dipropylene glycol, butylene glycol, and polyethyleneglycols having general formula HOCH2(CH2OH)nCH2OH where n (number of oxyethylene groups) is 4-200. In particular embodiments, the non-ionizable glycol ether may be diethylene glycol monoethyl ether ("DEGEE" or "ethoxydiglycol" known under its trade name TRANSCUTOL^{®}, commercially available from Gattefosse, Paramus, N.J).

The non-ionizable glycol ethers can be included in the compositions of various embodiments at a concentration of about 0.01 w/w % to 50 w/w %, and in particular embodiments, the concentration of non-ionizable glycol ethers may be about 0.5 w/w % to about 10 w/w %. In various embodiments, the non-ionizable glycol ether can be included in a total amount of about 1 w/w % to about 25 w/w %, about 2 w/w % to about 20 w/w %, about 4 w/w % to about 10 w/w %, about 4 w/w % to about 8 w/w %, about 4 w/w % to about 5 w/w %, or any range or individual concentration within these example ranges.

In some embodiments, the penetration or permeation enhancer may be a peptide or protein fragment. Such peptides and protein fragments are generally termed "skin penetrating peptides" (SPPs) or cell penetrating peptides (CPPs). SPPs may stabilize these structural proteins in the skin rather than denaturing them. For example, SPPs bind to keratin proteins through hydrogen bonds and weak electrostatic interactions and may operate as binding mediators between keratin and drug molecules. SPPs may also utilize pathways between corneocytes via diffusion of drug via gaps between cells as well as through lipid bilayers, without disruption. An example of a SPP is TD-1, which is known to loosen the desmosome-induced tight junctions between corneocytes with a change in the space between cells from about 30 nm to about 466 nm in 30 minutes from topical application. The cell gaps increase and then gradually are restored in 1 hour after treatment with TD-1. Various SPPs are known in the art and numerous peptides containing 9 to 19 amino acids have been shown to exhibit skin penetrating activity. Embodiments encompass all such peptides.

In some embodiments, both the permeation enhancers and the decoy molecules help in penetration of the active agents through the surface tissue. In some embodiments, the permeation enhancers and decoy molecules act in concert, in a synergistic manner. In some embodiments, the permeation enhancers specifically help in penetration of hydrophobic active agents.

In some embodiments, the compositions are devoid of permeation enhancers.

### Transdermal Patches

In some embodiments, the compositions disclosed herein can be in the form of transdermal patches. The transdermal patches can be in any conventional form such as, for example, a strip, a gauze, a film, and the like. Patch material may be nonwoven or woven (e.g., gauze dressing). Layers may also be laminated during processing. It may be nonocclusive or occlusive, but the latter is preferred for backing layers. The patch is preferably hermetically sealed for storage (e.g., foil packaging). The patch can be held onto the skin and components of the patch can be held together using various adhesives. For example, the transdermal patch can be in the form of a band-aid type device, or it may be packaged in a small metal or plastic "cup", which is strapped onto the appropriate site using an adhesive, tape, or an outer fabric or leather strap, similar to that worn as part of a watch. The entire patch may be disposable or may be refillable.

The transdermal patch disclosed herein may be made of any polymeric material. Non-limiting examples of polymeric matrix materials that may be used in a transdermal patch are ethylene vinyl acetate (EVA) copolymer, crosslinked poly(vinyl alcohol), poly(hydroxy ethylmethacrylate), acyl substituted cellulose acetates, hydrolyzed alkylene-vinyl acetate copolymers, polyvinyl chloride, polyvinyl acetate, polyvinyl alkyl ethers, polyvinyl fluoride, polycarbonate, polyurethane, polyamide, polysulphones, styrene acrylonitrile copolymers, crosslinked poly(ethylene oxide), poly(alkylenes), poly(vinyl imidazole), poly(esters), poly(ethylene terephthalate), polyphosphazenes, chlorosulphonated polyolefines, poly-lactides (PLA), poly-glycolides (PGA), or combinations thereof.

In some embodiments, the transdermal patch can be in the form of hydrogels. In some embodiments, the compositions in the transdermal patch may further include components like binders, buffers, colorings, dessicants, diluents, humectants, preservatives, stabilizers, other excipients, adhesives, plasticizers, tackifiers, thickeners, cooling agents, or combinations thereof.

### Methods of Using the Compositions

Disclosed herein are methods for treating various disorders. The composition comprising active agents, hyaluronidase/elastase, and decoy molecules described herein are administered to a surface tissue. The administration is generally topical, transdermal, percutaneous, or microneedle injection. A "surface tissue" includes any tissue surface such as, but not limited to, skin, mucosa, eyes, ears, inside the nose, inside the mouth, lips, urethral openings, vagina, anus, tongue, frenulum of tongue, hair, teeth, bone, lacrimal glands, sinus mucosa, respiratory tract, gums, and the like. In some embodiments, the surface tissue is a skin surface or a mucosal surface. In some embodiments, mucosal surface can be eye. In some embodiments, mucosal surface can be oral cavity or vaginal cavity.

In some embodiments, the compositions administered comprise one or more active agents and a decoy molecule selected from an extracellular matrix component, fragments thereof and combinations thereof. In some embodiments, the compositions administered comprise one or more active agents and an enzyme selected from hyaluronidase, elastase, or a combination thereof. In some embodiments, the compositions administered comprise one or more active agents and a decoy molecule selected from an extracellular matrix component, fragments thereof and combinations thereof, and further comprise an enzyme selected from hyaluronidase, elastase, or a combination thereof. In some embodiments, the compositions administered comprise one or more active agents, and no decoy molecules or hyaluronidase/elastase enzymes. In some embodiments, the compositions administered comprise only decoy molecules and no active agents. In some embodiments, the compositions administered comprise an enzyme selected from hyaluronidase, elastase, or a combination thereof, and no active agents.

### Skin Disorders

Embodiments of the invention are directed to compositions for use in methods of treating a skin condition in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions comprise an active agent and hyaluronidase/elastase enzymes, and no decoy molecules. In embodiments, the skin condition is selected from acne, psoriasis, atopic dermatitis, rosacea and combinations thereof.

In some embodiments, the skin condition may be a benign neoplasm, premalignancy or malignancy. In some embodiments, the skin condition may be selected from Human Papilloma Virus induced lesions e.g. warts, common warts, palmoplantar warts, flat warts, epidermodysplasia verruciformis related warts, anogenital warts, condyloma accuminatum; Herpesvirus related lesions including those induced by HHV-1 (HSV-1), HHV-2 (HSV-2), HHV-3 (varicella-zoster virus) e.g. chicken pox, Herpes zoster, shingles; Poxvirus induced lesions e.g. molluscum contagiosum, orf,; callus, cutaneous horns, corns, acrochordons, fibroepithelial polyps, prurigo nodularis, actinic keratoses, squamous cell carcinoma, squamous cell carcinoma in situ, keratoacanthoma, basal cell carcinoma, cutaneous lymphomas and benign lymphocytic infiltrates & hyperplasias of the skin, clear cell acanthoma, large cell acanthoma, epidermolytic acanthoma, porokeratosis, hyperkeratosis, lichenoid keratosis, acanthosis, acanthosis nigricans, confluent and reticulated papillomatosis, nevi, including e.g. dermal nevi, epidermal nevi, compound nevi, ILVEN (inflammatory linear verrucous epidermal nevi), nevus sebaceous, nevus comedonicus, and the like; acne, e.g. comedonal acne, inflammatory acne, papular acne, pustular acne, cystic acne; cysts, e.g. epidermoid cysts, milia, trichilemmal cysts, follicular cysts, proliferating cysts, dermoid cysts, pilonidal cysts, apocrine cysts, eccrine cysts, sebaceous cysts, mucous cysts, myxoid cysts, ganglion cysts, synovial cysts, vellus hair cysts, steatocystoma, hidrocystoma; adnexal neoplasms e.g. trichofolliculoma, fibrofolliculoma, perifollicular fibroma, trichodiscoma, nevus sebaceous, chondroid syringoma, trichoepithelioma, trichoblastoma, desmoplastic trichoepithelioma, pilomatricoma, pilomatrical carcinoma, tricholemmoma, trichelemmal carcinoma, tumor of the follicular infundibulum, tricoadenoma, proliferating pilar tumor, sebaceous hyperplasia, sebaceous adenoma, sebaceous epithelioma, sebaceous carcinoma, syringoma, poroma, hidradenoma, apocrine hidradenoma, spiradenoma, cylindroma, eccrine nevus (eccrine hamartoma), papillary adenoma, papillary adenocarcinoma; Benign melanocytic neoplasms e.g. ephilides, café-au-lait macules, Becker's melanosis, lentigines, solar lentigines, lentigo simplex, mucosal melanocytic lesions, Mongolian spots, Nevus of Ota, blue nevus, common acquired melanocytic nevi (nevocellular nevus, "moles"), congenital nevi, nevus spilus, recurrent nevi; vascular and perivascular neoplasms and reactive hyperplasias e.g., hemangiomas, cherry angiomas, hobnail hemangiomas (targeted hemosiderotic hemangiomas), tufted angiomas, hemangioendotheliomas, angiolymphoid hyperplasia with eosinophilia (ALHE), Glomus tumors (glomangiomas), hemangiopericytomas; cutaneous neural and neuroendocrine neoplasms e.g. neuromas, Schwannomas, neurofibromas, nerve sheath tumor, nerve sheath myxoma, neurothekeoma, granular cell tumor; fibrotic and fibrohistiocytic proliferations e.g. acrochordons, fibroepithelial polyps, fibromas, fibrous papules, angiofibromas, pearly penile papules, periungual fibromas, dermatofibromas, fibrokeratomas, sclerotic or pleomorphic fibromas, connective tissue nevi; cutaneous scars, hyperplasias, keloids, rosacea, cutaneous fungal, dermatophyte & mold infections, onychomycosis, hyperpigmentation, rhytides, psoriasis, malignant melanoma, seborrheic keratosis, seborrheic keratosis variants including e.g. dermatosis papulosis nigra, inverted follicular keratosis/keratoma warty dyskeratosis/warty dyskeratoma, acrokeratosis verruciformis, stucco keratosis; or a combination thereof.

The compositions disclosed herein can include any active agent to treat a skin condition. Non-limiting examples of active agents include salicylic acid, potassium hydroxide, podophyllin, cantharidin, imiquimod, nitric acid, oral cimetidine, 5-fluorouracil, bleomycin, DNCB, imiquimod, and trichloroacetic acid, benzoyl peroxide, bleomycin, 2,4-dinitrochlorobenzene, fluorouracil, salicyclic acid, silver nitrate, zinc sulfate, zinc oxide, clindamycin hydrochloride and clindamycin phosphate, erythromycin, tetracycline, dicloxacilin, doxycycline, minocycline, bacitracin, chlortetracycline, neomycin, mupirocin, polymyxin B, cuprimyxin, furazolidone, gentamycin, lincomycin, cephalosporins, betalactam antibiotics, Iincomycin hydrochloride, tazarotene, vitamin A, retinoic acid, tretinoin, isoretinoin, adapalene, retinol, acitretin, bexarotene, retinoids; oxybutynin; vitamin D, vitamin C, vitamin B, vitamin E; sulfur; glucocorticosteroids, corticosteroids, triamcinolone, triamcinolone acetonide, betamethasone, betamethasone 1 7-valerate, betamethasone dipropionate, halcinonide, isoflupredone acetate, flumethasone, fluocinonide, mometasone, fluticasone, fluticasone propionate, prednisolone, beclomethasone, hydrocortisone, cyproterone, drospirenone, estrogen, progestogen, tacrolimus, pimecrolimus, ursolic acid, betulinic acid, moronic acid, oleanolic acid, acyclovir, valaciclovir, famciclovir, penciclovir, docosanol, perillyl alcohol, and combinations thereof.

Embodiments of the invention are directed to compositions for use in methods of improving skin texture in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules. In some embodiments, improving skin texture is selected from improving luminosity, quality and combinations thereof.

In some embodiments, a composition for use in a method of treating scars and wrinkles on a skin surface includes administering to the skin surface a composition decribed herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules.

The therapeutic compositions disclosed herein may have cosmetic purposes. The composition can be used, for example, to improve the appearance of skin, such as by reduction or removal of facial lines and wrinkles, as well as reduction or removal of stretch marks. According to one embodiment, a method of improving appearance of skin includes administering the composition in an amount sufficient to stimulate elastogenesis. Moreover, the compositions disclosed herein may tighten loose, sagging skin on the face and other parts of the body including arms, legs, chest and neck areas, or give the appearance of reducing wrinkles. Other methods of use of the compositions include stimulation of smooth muscle cells and gingival fibroblasts to produce elastin and fibrillin (oxytalan fibers), respectively, for the treatment of neointimal thickening and loosening of teeth (gingivitis), respectively. Accordingly to some embodiments, the disclosure also contemplates compositions and methods for stimulating dermal cell differentiation.

Furthermore, the compositions may be used to enhance wound healing and to prevent and treat cutaneous hypertrophic scars. Accordingly, another embodiment of the disclosure includes a method of promoting wound healing and reducing scarring comprising applying a pretreated fibroblast composition to the wound in an amount sufficient to stimulate deposition of elastin at a site of injury. In some embodiments, compositions comprising therapeutic cells that produce elastin or collagen may be used to promote wound healing or to treat scars. Wounds suitable for treatment include those resulting from trauma such as burns, abrasions and cuts, wounds resulting from surgical procedures such as incisions and skin grafting, cutaneous wounds, corneal wounds, and ulcers. Elastin synthesis at the site of injury may also lessen scarring since scar tissue is devoid of elastin, and elastin is an important component of uninjured skin. The stimulation and secretion of elastin into the matrix may also generally provide a favorable environment for the cells that participate in the healing process, further accentuating the wound healing process.

### Hair Disorders

The compositions of various embodiments disclosed herein can be used for the treating hair, hair shaft, hair follicles, hair bulbs, oil glands, and components thereof. Thus, certain embodiments include methods for administering an active agent by contacting hair, scalp, or skin containing hair or hair follicles with the compositions described above. The compositions and methods described herein can be used for administering any active agent to hair, hair shaft, hair follicles, hair bulbs, oil glands, and components thereof, including small molecule drugs, macromolecular drugs, biologics, antibodies, chimeric antibodies, peptides, antioxidants, and the like and combinations thereof.

The compositions of various embodiments may include any active agent for treatment of diseases related to hair, hair shaft, hair follicles, hair bulbs, oil glands, and components thereof, including, for example, hair loss, dandruff, seborrheic dermatitis, alopecia areata, hair disease, ringworm, tinea capitis, folliculitis, pattern hair loss, telogen effluvium, cradle cap, trichotillomania, traction alopecia, trichorrhexis nodosa, folliculitis decalvans, head lice infestation, frontal fibrosing alopecia, non-scarring hair loss, pityriasis amiantacea, dissecting cellulitis of the scalp, acne keloidalis nuchae, monilethrix, pediculosis, alopecia totalis, pseudopelade of Brocq, bubble hair deformity, hair casts, hypertrichosis, ingrown hair, monilethrix, premature greying of hair, pattern hair loss, trichorrhexis invaginata, and the like. The agents can be designed for topical, systemic, or local delivery, and non-limiting examples of active agents include a biologic, therapeutic peptides, biomimetic peptide, small molecule and macromolecular analgesic agents, steroids, and dyes and coloring agents. Particular embodiments, the active agent may be but not limited to minoxidil (Rogaine), finasteride (Propecia), spironolactone (Aldactone), cimetidine (Tagmet), triamcinolone (Kenalog, Triderm), prednisone, betamethasone (Diprolene), fluocinolone (Synalar-HP), clobetasol (Clobex, Cormax, Embeline, Temovate), anthralin (Dritho-Creme, Dritho-Scalp, Zithranol-RR), sulfazine, sulfasalazine (Azulfidine), cyclosporine (Gengraf, Neoral, Restasis, Sandimmune), and the like and combinations thereof. Further examples of active agents include dyes and hair coloring agents and/or humectants, emollients, oils, proteins, silicones, surfactants, polymers, or other hair conditioning agents. The compostions of various embodiments can include any one or more active agent listed above. For example, the compositions of some embodimetns may include a hair growth agent, a coloring agent or dye, and a conditioning agent, and in other embodiments, compositions may include one of a growth agent, dye, or conditioning agent.

The compositions of the various embodiments described above can be used to deliver adequate active agent to the hair, hair shaft, hairshaft, hair follicle, hair bulb, or oil gland to affect treatment. In particular embodiments, the decoy may allow the active agent to penetrate the epidermis and dermis to the hair follicle and hair bulb. For example, certain active agents, such as minoxidil, do not penetrate the skin layers well, limiting the amount of active agent in that contacts the hair follicle, where it is effective, without chemical carriers (e.g. dimethyl sulfoxide, DMSO) or mechanical delivery devices (e.g. microneedles). Compositions including the active agent and a decoy can provide sufficient penetration and active agent delivery to the hair follicle without using chemical carriers or mechanical delivery systems, reducing potential side effects and/or injuries to the epidermis and scalp. In addition, the decoys described aboce allow active agents to penetrate the hair shaft, thereby increasing delivery of active agents, dyes, hair conditioning agents and the like to the hair shaft. For example, improved dye penetration, coloring, and color retention can be achieved using compositions including hair dye with a decoy. Similarly, hair conditioning agents such as humectants and proteins can be combined with a decoy to produce compsitions that deliver such conditioning agents to the hair shaft.

### Other Disorders

Embodiments of the invention are directed to compositions for use in methods of improving the execution of motor tasks and/or functional performance in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules. In embodiments, the improving the execution of motor tasks and/or functional performance in a subject in need thereof includes improvement in fine motor skills of a subject, due to, for exemple, extreme or excessive temperatures (warm or cold).

Embodiments of the invention are directed to compositions for use in methods of treating Reynaud's syndrome in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules.

Embodiments of the invention are directed to compositions for use in methods of treating claudication in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules.

Embodiments of the invention are directed to compositions for use in methods of treating the signs and symptoms of peripheral vascular disease in a subject in need thereof comprising topically administering to a surface tissue of the subject a composition described herein. The composition may comprise an effective amount of an active agent and a decoy molecule selected from an extracellular matrix component, fragments thereof and combinations thereof as described herein. In some embodiments, the composition may further comprise hyaluronidase/elastase enzymes. In some embodiments, the compositions may comprise an active agent and hyaluronidase/elastase enzymes, and no decoy molecules.

In some embodiments, the compositions disclosed herein can be used to treat various conditions, such as but not limited to chronic pain, post-operative pain, urinary incontinence, neurological disorders (Alzheimer's, dementia, Parkinson's, restless leg syndrome, depression, neuropathic pain, schizophrenia, sleep disturbance, cognitive disorder), angina, coronary heart disease, COPD, nausea, motion sickness, contraceptive, hormonal therapy, arthritis, osteoarthritis, rheumatoid arthritis, inflammatory bowel disease, addiction, ADHD, anti-inflammatory conditions, skin disorders, breast cancer, erectile dysfunction, vitamin deficiency, calcium deficiency, diabetes, diabetic neuropathy, diabetic foot, post-menopause symptoms, hot flashes, hormone replacement therapy, migraine, herpes infection, gingival inflammation, renal failure, Tinnitus, tennis elbow, tendonitis, lipolysis, carpal tunnel syndrome, hypogonadism, avascular necrosis, induction of labor, peripheral neuropathic pain, spinal cord injury, oral mucositis, and hypertension.

In some embodiments, the compositions disclosed herein may be used to treat conditions related to nails, such as Beau's lines, onycholysis, onychomycosis, onychoschizia, paronychia, onychocryptosis, mycosis, yellow nail syndrome, onychorrhexis, koilonychias, subungual hematoma, leukonychia, psoriatic onychodystrophy, stipple nails, onychogryphosis, and the like.

In some embodiments, the compositions disclosed herein may be used to treat conditions related to eye, such as amblyopia, blepharitis, chalazion, conjunctivitis, corneal abrasion, dry eye, diabetic retinopathy, glaucoma, keratitis, hordeolum, uveitis, sty, and the like.

In some embodiments, the compositions disclosed herein may be used to treat conditions related to buccal or oral cavity, such as oral cancer, thrush, ulcers, gingivitis, sores, leukoplakia, smoker's palate, oral candidosis, bacterial and viral infections, and the like.

In some embodiments, the compositions disclosed herein may be used to treat conditions related to nasal cavity, such as rhinitis, nasal polyps, sinus infection, upper respiratory tract infections, and the like.

In some embodiments, the compositions disclosed herein may be used to treat vaginal diseases, such as vaginitis, vaginal discharge, gonorrhea, bacterial vaginosis, sexually transmitted diseases, atrophic vaginitis, yeast infection, genital wart, vaginal cancer, and the like.

In some embodiments, the compositions disclosed herein may be used to treat conditions related to anus, such as hemorrhoids, anal cancer, pruritus ani, anal fistula, and the like.

In some embodiments, the compositions disclosed herein may be used to treat conditions related to tongue, such as median rhomboid glossitis, atrophic glossitis, fissured tongue, geographic tongue, oral hairy leukoplakia, lichen planus, linea alba, squamous cell carcinoma, papilloma, macroglossia, and the like.

In some embodiments, the compositions disclosed herein may be used to treat sinus conditions, such as sinusitis, rhinosinusitis, acute sinusitis, subacute sinusitis, subacute rhinosinusitis, chronic sinusitis, chronic rhinosinusitis, acute exacerbation of chronic rhinosinusitis, fungal sinus disease, sinusitis with polyps, sinus tumors, and the like.

In some embodiments, the compositions disclosed herein may be used to treat respiratory tract conditions, such as lung cancer, interstitial lung disease, pulmonary embolism, chronic obstructive pulmonary disease, pneumonia, pneumothorax, pulmonary hypertension, pleural effusion, non-small cell lung cancer, asthma, pulmonary fibrosis, obstructive lung disease, respiratory disease, sarcoidosis, bronchitis, tuberculosis, idiopathic pulmonary fibrosis, cystic fibrosis, traction bronchiectasis, pneumonitis, respiratory failure, bronchiolitis, hypersensitivity pneumonitis, restrictive lung disease, usual interstitial pneumonia, lung infection, acute, respiratory distress syndrome, pleurisy, pneumoconiosis, coalworker's pneumoconiosis, hypoxemia, dermatomyositis, burning chest pain, pneumocystis pneumonia, and the like.

In some embodiments, the compositions disclosed herein may be used to treat conditions associated with lacrimal ducts, such as nasolacrimal duct obstruction, partial lacrimal duct obstruction, total lacrimal duct obstruction, dacryostenosis, dacryoadenitis, dacryocystitis, congenital dacryocystitis, ocular infection, and the like.

In some embodiments, the compositions disclosed herein may be used to treat conditions associated with inner ear, such as inner ear infection, Meniere's disease, vertigo, autoimmune inner ear disease, noise-induced hearing loss, acoustic neuroma, benign paroxysmal positional vertigo, drug-induced ototoxicity, herpes zoster oticus, purulent labyrinthitis, vestibular neuronitis, and the like.

In some embodiments, the compositions disclosed herein may be used to treat bladder conditions, such as bladder cancer, urinary tract infection, cystocele, interstitial cystitis, overactive bladder, urinary, incontinence, urinary bladder disease, urinary retention, benign prostatic hyperplasia, neurogenic bladder dysfunction, vesicoureteral reflux, stress incontinence, gastrointestinal disease, nocturnal enuresis, and the like.

In some embodiments, the compositions disclosed herein may be used to treat kidney conditions, such as acute kidney failure or injury, pyelonephritis, chronic kidney disease, polycystic kidney disease, kidney disease, glomerulonephritis, kidney pain or stone, lupus erythematosus, nephrotic syndrome, nephritis, diabetic nephropathy, IgA nephropathy, autosomal dominant polycystic, kidney disease, cystic kidney disease, renal cyst, alport syndrome, renal tubular acidosis, goodpasture syndrome, medullary sponge kidney, and the like.

In some embodiments, the compositions disclosed herein may be used to treat urinary tract conditions, such as hydronephrosis, bacteriuria, hematuria, bacterial, fungal, and yeast infections, and the like.

The compositions for use in methods of such embodiments can be used for treating nearly any condition. For example, the methods of embodiments can be used for treatment of a variety of skin conditions including acne, local pain relief, local fungal or bacterial infections, skin cancer, abscesses, cellulitis, texture, appearance, sensation, hydration and the like. In other embodiments, the methods may be used for administration of various cosmetic therapies for improving, for example, skin thickness, elasticity, resiliency, smoothness, tone, texture, brightness, clarity, contour, firmness, tautness, suppleness, discoloration, skin lesions, and the like and combinations thereof. In other embodiments, the methods may be used in conjunction with various cosmetic therapies. The methods of further embodiments can be used for enhancing the color or strength of, for example, hair or teeth. In still other embodiments, the methods of the invention can be used for administering active agents for treating numerous systemic conditions in which transdermal delivery of the active agent is preferred, for example, chronic pain relief, cancer, motion sickness, chronic illnesses, and the like and combinations thereof.

Embodiments of the invention are directed to compositions for use in methods of treating, reducing or improving the look of frown lines (e.g., glabellar lines), wrinkles or crow's feet lines in a subject in need thereof comprising topically administering to a surface tissue of the subject a composition comprising an effective amount of an active agent and an extracellular matrix component, fragments thereof and combinations thereof as described herein. In some embodiments, the active agent is a neurotoxin. In some embodiments, the frown lines, wrinkles or crow's feet lines may be fine, moderate or severe or a combination thereof.

Embodiments of the invention are directed to compositions for use in methods of treating the symptoms of excessive sweating in a subject in need thereof comprising topically administering to a surface tissue of the subject a composition comprising an effective amount of an active agent and an extracellular matrix component, fragments thereof and combinations thereof as described herein. In some embodiments, the active agent is a neurotoxin. In some embodiments, sweating is severe underarm sweating (severe primary axillary hyperhidrosis). In some embodiments, sweating is underarm sweating, hand sweating, foot sweating self-perceivied or externally-perceived excessive sweating and combinations thereof. In some embodiments, the subject is 18 years and older. In some embodiments, the subject has not responded to other medicines used on the skin (topical).

Embodiments of the invention are directed to compositions for use in methods of treating migraines in a subject in need thereof comprising topically administering to a surface tissue of the subject a composition comprising an effective amount of an active agent and an extracellular matrix component, fragments thereof and combinations thereof as described herein. In some embodiments, the active agent is a neurotoxin. In embodiments, migraines may be episodic migraines. In some embodiments, migraines may be chronic migraines.

Embodiments of the invention are directed to compositions for use in methods of treating a condition in a subject in need thereof comprising topically administering to a surface tissue of the subject a composition comprising an effective amount of an active agent and an extracellular matrix component, fragments thereof and combinations thereof as described herein. In embodiments, the condition may be selected from overactive bladder symptoms incontinence, to prevent headaches with chronic migraine, increased muscle stiffness, abnormal head position and neck pain that happens with cervical dystonia (CD), strabismus, or blepharospasm. In embodiments, the condition may be a skin condition. In embodiments, the condition may be Reynaud's syndrome, excessive sweating, improving skin texture, claudication, or peripheral vascular disease. In embodiments, the condition may be selected from overactive bladder symptoms incontinence, to prevent headaches with chronic migraine, increased muscle stiffness, abnormal head position and neck pain that happens with cervical dystonia (CD), strabismus, or blepharospasm, headaches, erectile dysfunction, depression, plantar ficitis, skin texture and appearance, sweating, hyperhidrosis and temporary improvement in the appearance of (i) moderate to severe glabellar lines associated with corrugator and/or procerus muscle activity, (ii) moderate to severe lateral canthal lines associated with orbicularis oculi activity, or (iii) moderate to severe forehead lines associated with frontalis muscle activity. In embodiments, the condition may be selected from overactive bladder symptoms (such as a strong need to urinate with leaking or wetting accidents (urge urinary incontinence), a strong need to urinate right away (urgency), and urinating often (frequency)) in adults 18 years and older when another type of medicine (anticholinergic) does not work well enough or cannot be taken; to treat leakage of urine (incontinence) in adults 18 years and older with overactive bladder due to neurologic disease who still have leakage or cannot tolerate the side effects after trying an anticholinergic medication; to prevent headaches in adults with chronic migraine who have 15 or more days each month with headache lasting 4 or more hours each day in people 18 years or older; to treat increased muscle stiffness in elbow, wrist, finger, thumb, ankle, and toe muscles in people 18 years and older with upper and lower limb spasticity; or to treat the abnormal head position and neck pain that happens with cervical dystonia (CD) in people 16 years and older; to treat certain types of eye muscle problems (strabismus) or abnormal spasm of the eyelids (blepharospasm) in people 12 years and older, erectile dysfunction, depression, plantar ficitis, skin texture and appearance, overactive bladder (OAB) with symptoms of urge urinary incontinence, urgency, and frequency, in adults who have an inadequate response to or are intolerant of an anticholinergic medication, urinary incontinence due to detrusor overactivity associated with a neurologic condition (e.g., spinal cord injury (SCI), multiple sclerosis (MS)) in adults who have an inadequate response to or are intolerant of an anticholinergic medication, prophylaxis of headaches in adult patients with chronic migraine (≥15 days per month with headache lasting 4 hours a day or longer), spasticity in adult patients, severe axillary hyperhidrosis that is inadequately managed by topical agents in adult patients, blepharospasm associated with dystonia in patients ≥12 years of age, and temporary improvement in the appearance of (i) moderate to severe glabellar lines associated with corrugator and/or procerus muscle activity, (ii) moderate to severe lateral canthal lines associated with orbicularis oculi activity, or (iii) moderate to severe forehead lines associated with frontalis muscle activity.

### Cancer Therapy

In some embodiments, a composition for use in a method of eliciting immune response in a subject includes administering to a tissue surface a composition comprising a plurality of therapeutic cells expressing an antigen and a decoy molecule selected from an extracellular matrix component, fragments thereof and combinations thereof as described herein. In some embodiments, the antigen may be a tumor antigen. The tumor antigen, without limitation, includes stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor.

In some embodiments, a composition for use in a method of treating cancer in a subject includes administering to a tissue surface a composition comprising a plurality of therapeutic cells expressing an antigen and a decoy molecule selected from an extracellular matrix component, fragments thereof and combinations thereof as described herein. In some embodiments, the antigen may be a tumor antigen. The tumor antigen, without limitation, includes stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor.

In some embodiments, the compositions for use in methods disclosed herein can be used in combination with any adoptive cell transfer (ACT) therapy. ACT is a very effective form of immunotherapy and involves the transfer of immune cells with antitumor activity into cancer patients. ACT involves the identification, in vitro, of lymphocytes with antitumor activity, the in vitro expansion of these cells to large numbers and their infusion into the cancer-bearing host. Lymphocytes used for adoptive transfer can be derived from the stroma of resected tumors (tumor infiltrating lymphocytes or TILs). They can also be derived or from blood if they are genetically engineered to express antitumor T cell receptors (TCRs) or chimeric antigen receptors (CARs), enriched with mixed lymphocyte tumor cell cultures (MLTCs), or cloned using autologous antigen presenting cells and tumor derived peptides. ACT in which the lymphocytes originate from the cancer-bearing host to be infused is termed autologous ACT.

### Non-Invasive Topical Diagnostics

The compositions disclosed herein may be used for non-invasive topical diagnostics and imaging. In some embodiments, the method comprises applying a transdermal patch comprising a composition that includes one or more active agents and a decoy molecule a decoy molecule selected from an extracellular matrix component, fragments thereof and combinations thereof as described herein. In some embodiments, the composition may comprise hyaluronidase/elastase enzymes in place of decoy molecules. It is believed that the decoy molecules or hyaluronidase/elastase enzymes temporarily disrupt cell-cell (i.e. intercellular) and cell-scaffold attachment thereby allowing interstitial fluid, sweat, lymphatic fluid, serum, blood, and other body fluids to diffuse and reach the surface of a tissue, such as skin surface. The diffused fluid is absorbed or collected by the transdermal patch and the fluid is analyzed for analytes, antigens, pH, metabolotes, electrolytes, and the like. Such methods may be used to collect and diagnose body fluids noninvasively.

In some embodiments, the transdermal patch includes a liquid absorbent pad. The liquid absorbent pad may be fabricated from any convenient material, where suitable materials of interest include, but are not limited to cellulosic materials, polymeric materials, etc. In some instances, the absorbent pad material is made up of hydrophilic materials. In other embodiments, absorbent patches are selected from the group consisting of guazes, cellulosic pads, agarose gels, acrylamide gels. In some embodiments, the transdermal patch are in the form of hydrogels disclosed herein.

In some embodiments, the absorbent pad is covered on its upper surface by an occlusive layer, with the lower surface coated with an adhesive to affix the pad to the skin. The pad is disclosed as being a somewhat absorbent material capable of functioning as a reservoir, and is formed of a microcellular polyester or polyether cellular urethane foam layer, cotton, non-woven or similar cloth-like material capable of retaining, but yet dispensing, a liquid carrier.

The analytes that may be detected include but not limited to heavy metals such as lead, cadmium, lithium, copper, iron, and mercury; organic compounds; biological analytes and/or metabolites, such as oxygen, glucose, lactate, galactose, ethanol, glutamate, fructose, creatine, creatinine, bilurubin, urea, uric acid, albumin; electrolytes such as ammonia, calcium, carbon dioxide, chloride, lithium, magnesium, phosphorus, potassium, sodium; toxins; lipids such as cholesterol, triglycerides; hormones such as insulin, 11-deoxycortisol, 17-hydroxyprogesterone, androstenedione, DHEA sulfate, dimeric Inhibin A, estradiol, FSH, hCG, hCG, luteinizing hormone (LH), PAPP-A, progesterone, prolactin, SHBG, testosterone; therapeutic and pharmacologic agents; drugs of abuse such as cocaine, caffeine, alcohols, and acetominophen; recreational drugs; amino acids; blood gases; enzymes such as pancreatic and liver enzymes; antibiotics; cytokines; proteins; biomarkers; cardiac markers; inflammatory disease markers such as C-reactive protein; tumor markers; bacterial and viral antigens; and other biologically relevant molecules.

Other non-limiting examples of analytes that may be detected include ferric ammonium citrate, bees wax, digitonin, p-aminoacetophenone, dichloroquinone chlorimide, dichlorophenol, and butanoic acid.

Following extraction, detection and quantitation of the analyte may be carried out by any standard chemical, physical, enzymatic and/or optical means. The byproducts are then being detected using electrochemical, biochemical, optical, fluorescence, absorbance, reflectance, Raman, magnetic, mass spectrometry, IR spectroscopy measurement methods and combinations thereof.

In some embodiments, the compositions for use in methods disclosed herein can be used to detect antigens noninvasively. In some embodiments, the method involves applying a transdermal patch comprising a composition that includes an antibody or its fragments and a decoy molecule a decoy molecule selected from an extracellular matrix component, fragments thereof and combinations thereof as described herein. In some embodiments, the composition may comprise hyaluronidase/elastase enzymes in place of decoy molecules. The presence of decoy molecule or hyaluronidase/elastase enzymes cause rearrangement of tissues that the composition contacts by temporarily disrupting cell-cell (i.e. intercellular) and cell-scaffold attachment thereby allowing the antibody and antibody fragments to penetrate and pass through the tissue noninvasively and bind to the antigen. The bound antibody can be detected by imaging. The antibody or the antibody fragment may be any antibody described herein, such as but not limited to antibodies and its fragments that recognize a tumor antigen, a cancer antigen, an allergen, a bacterial antigen, a viral antigen, a drug, a hormone, a plant lectin, an endotoxin, and combinations thereof.

The transdermal patch can be applied to any tissue surface, such as but not limited to, skin, mucosa, eyes, ears, inside the nose, inside the mouth, lips, urethral openings, vagina, anus, tongue, frenulum of tongue, hair, teeth, bone, lacrimal glands, sinus mucosa, respiratory tract, gums, and the like. In some embodiments, the tissue surface is a skin surface or a mucosal surface. In some embodiments, mucosal surface can be eye. In some embodiments, mucosal surface can be oral cavity, buccal cavity, or vaginal cavity.

In some embodiments, the compositions for use in a method further involves detecting the antibody or the antibody fragment by imaging. The antibody or its fragment may be labelled by any of the detecting moiety disclosed herein. In some embodiments, imaging comprises detecting the antibody or the antibody fragment by SPECT (Single Photon Emission Computed Tomography), PET (Positron Emission Tomography), gamma camera imaging, rectilinear scanning, autoradiography, magnetic resonance imaging, fluorescence imaging, ultrasound, and combinations thereof.

In some embodiments, the compositions for use in methods disclosed herein can be used to measure the pH of body fluids noninvasively. In other embodiments, the methods disclosed herein can be used to monitor tissue drug levels for efficacy. In other embodiments, the methods can be used to detect coagulation parameters, such as prothrombin time (PT), activated partial thromboplastin time (aPTT), and thrombin time. In some embodiments, the methods can be used for testing various allergies. The body fluids isolated can be tested for antibodies against allergens, such as pollen, pollen, mold, pet dander, dust mites, and foods.

In some embodiments, the compositions for use in methods disclosed herein may enhance sensitivity and range of noninvasive monitoring of metabolites and electrolytes, for example in conjunction with a wearable sensor.

In some embodiments, the compositions for use in methods disclosed herein may be used to diagnose sezary syndrome, mycosis fungoides, skin lesions, infectious agents, hepatitis, bacterial infection, viral infection, fungal infection, pulmonary embolus, and tuberculosis.

In some embodiments, the compositions for use in methods can be used to image melanoma cells post excision, to monitor microinvasion. In other embodiments, methods and compositions can be used in conjunction with colonoscopy. For example, imaging of colon cancer using fluorescent tagged antibodies can performed after colon wash with composition comprising extracellular matrix components. Such methods may cause increased permeation of tagged antibodies leading to enhanced signal in the presence of colon cancer antigens.

In some embodiments, the compositions for use in methods disclosed herein have pediatric applications due to noninvasive procedures.

The compositions for use in methods of such embodiments may include a variety of additional steps including, for example, cleaning the surface tissue at the site of applying and the like. For example, prior to administering the transdermal patch the tissue surface is ablated by electromagnetic radiation, laser, dermal abrasion, chemical peel, ultrasound, heating, cooling, or by a needle. For example, skin preparation wipe may applied to the skin prior to application of the transdermal patch. It is typically applied to the target skin area by massaging, wiping, padding, rubbing or any other methods to clean the target skin site and to increase porosity. The wipe may also contain routine skin permeation enhancers known in the art. The wipe can be formed of a paper, cotton or textile based substrate soaked in agents containing water, phosphate buffered saline, lactic acid, soap, surfactant or any other chemicals, solvents or their mixtures which can be used to clean the target skin area after any skin pretreatment procedure, such as SonoPrep^{®} Ultrasonic Skin Permeation System (Sontra Medical). Preferably, the agents are inorganic or organic solvents such as water, ethanol, isopropanol or a combination thereof. An exemplary formulation of the agent contains 30-95% of isopropanol in water and the wipe material is gauze.

### Administration

The active agents and decoy molecules are a mixture of two components dissolved, suspended, dispersed or otherwise mixed in a carrier or vehicle. In some embodiments, the compositions comprising active agents and the compositions comprising decoy molecules or hyaluronidase/elastase enzymes can be administered concurrently or sequentially. Some embodiments may include the step of co-administering an active agent and a decoy molecule to a surface tissue. For example, such methods may include the step of applying a composition or formulation comprising an active agent and a composition or formulation comprising decoy molecule to a surface tissue of a subject. In other embodiments, the composition of decoy molecule may be applied to the surface tissue before topical administration of the composition of active agent. For example, a wipe containing a composition comprising one or more decoy molecules may be used for applying a decoy molecule to surface tissue followed by a step of topically administering a composition of an active agent to the surface tissue. In yet other embodiments, the composition of active agent may be applied to a surface tissue followed by applying a composition of decoy molecule to the surface tissue.

The compositions of embodiments described above may enhance the strength of known topical active agent thereby reducing the necessary dosage required to achieve a therapeutically effective amount. For example, in some embodiments, the strength of a composition containing an active agent and a decoy molecule may be about equal to about 80% or 90% greater than the active agent delivered in a standard topical formulation. In other embodiments, the strength of a composition containing an active agent and a decoy molecule may be about equal to about 75% greater, about 1.0% to about 80% greater, about 1.0% to about 75% greater, about 1.0% to about 50% greater, about 1.0% to about 25% greater, about 2.0% to about 80% greater, about 2.0% to about 75% greater, about 2.0% to about 50% greater, about 2.0% to about 25% greater, about 5.0% to about 50% greater, about 5.0% to about 25% greater than the active agent delivered in a standard topical formulation. Thus, the compositions described herein may provide therapeutic equivalence of known topically administered active agents with that an administered dose that is equal to or at least about 75% less than a standard dose, equal to or about 50% less than a standard dose, equal to or about 25% less than a standard dose, equal to or about 10% less than a standard dose, about 1.0% to about 75% less than a standard dose, about 1.0% to about 50% less than a standard dose, about 1.0% to about 25% less than a standard dose, about 1.0% to about 10% less than a standard dose, about 2.0% to about 75% less than a standard dose, about 2.0% to about 50% less than a standard dose, about 2.0% to about 25% less than a standard dose, about 2.0% to about 10% less than a standard dose, or any range or individual value encompassed by these example ranges.

In some embodiments, the methods disclosed herein may deliver at least 10% more active agent, at least 15% more active agent, at least 20% more active agent, at least 30% more active agent, at least 40% more active agent, at least 50% more active agent, at least 60% more active agent, at least 70% more active agent, at least 80% more active agent, at least 100% more active agent, at least 150% more active agent, at least 200% more active agent, at least 500% more active agent, or at least 700% more active agent to a subject when administered along with a decoy molecule, than when the active agent is administered without a decoy molecule. In some embodiments, the amount of active agent that is delivered may be from about 10% to 30% more, about 10% to 50% more, about 10% to 70% more, about 10% to 100% more, about 10% to 150% more, about 10% to 200% more, about 10% to 500% more, or about 10% to 1000% more when administered along with a decoy molecule, than when the active agent is administered without a decoy molecule.

The compositions disclosed herein may deliver the active agent more efficiently. That is, the effective amount of the active agent delivered at the site of administration is much more when compared to the delivery of the active agent without decoy molecules. As shown in Examples 1-15 and 17-20, the decoy molecules surprisingly help the active agents to penetrate the tissue more effectively, even when the active agents are used at low concentrations.

In some embodiments, administration of the composition is by topical application, transdermal, percutaneous, or microneedle injection. Administration can also be, for example, intravenous, intraperitoneal, subdermal, subcutaneous, intradermal, transcutaneous, intramuscular, oral, intra-joint, parenteral, intranasal, or by inhalation. Suitable sites of administration thus include, but are not limited to, the skin, bronchium, gastrointestinal tract, eye, buccal cavity, and ear. In some embodiments, the compositions disclosed herein can be administered to any solid tissue via a needle. Such tissues include liver tissue, lung tissue, tissues of the GI tract, muscle tissue, nervous tissue, bone, buccal tissue, and the like.

Compositions described herein can be administered transdermally, i.e., administered across the surface of the body and the inner linings of bodily passages including epithelial and mucosal tissues. In some embodiments, the compositions described herein may be administered in the form of lotions, creams, foams, patches, suspensions, solutions, and suppositories (rectal and vaginal). In some embodiments, transdermal administration can be accomplished through the use of a transdermal patch containing the composition and a carrier that is inert to the composition, non-toxic to the skin, and combine with the composition to allow the therapeutic cells to penetrate below dermis and epidermis. The carrier can take any number of forms such as paste, creams, ointments, pastes, gels, and occlusive devices. The creams and ointments can be viscous liquid or semisolid emulsions of either the oil-in-water or water-in-oil type. Pastes comprised of absorptive powders dispersed in petroleum or hydrophilic petroleum containing the compound can also be suitable. A variety of occlusive devices can be used, such as a semi-permeable membrane covering a reservoir containing the composition with or without a carrier, or a matrix containing the composition. Other occlusive devices are known in the literature.

The composition disclosed herein can also be administered as a fine-mist spray comprising, nanoencapsulated and non nanoencapsulated therapeutic cells and extracellular matrix component, and suspended in a carrier for topical application on wounds and burns in humans and animals. To this end, therapeutic cells will be used, incorporated as a suspension in appropriate carriers to be applied by means of a fine mist atomizer (of the "GMSP Pre-compressed Fine Mist Spray Pump" type).

The methods disclosed herein can be used to deliver therapeutic cells topically such that the cells penetrate the epithelial layer and are distributed uniformly in the underlying tissue. The distribution of the therapeutic cells may vary depending on the concentration of the cells and extracellular matrix component in the composition. In some embodiments, the therapeutic cells may be distributed unevenly, with some regions having higher concentration of cells than others. In some embodiments, the penetration of the cells through the epidermal layer and into the underlying tissue can be controlled by modulating the concentration of the extracellular matrix content in the composition. For example, a relatively low concentration of extracellular matrix component may allow for transport of the therapeutic cells partially across the epidermis, whereas a higher concentration of extracellular matrix component may allow for transport of therapeutic cells fully across the epidermis to the basement membrane underlying tissues layers, for example, dermis, and subcutis, when the composition is administered topically.

Methods disclosed herein may allow therapeutic cells to penetrate the tissue and repopulate and achieve the desired function. For example, topical administration of stem cells may result in penetration of the cells into the tissue, differentiation, and repopulation of the differentiated cells sufficient to reverse the underlying disorder. In other embodiments, the administered antigen presenting cells may recruit immune cells to the site of administration and may help to promote an effective immune response against a bacterial or viral infection, or against a tumor cell.

In some embodiments, the composition is administered by microneedle injection. Microneedle is a hollow needle having an exposed height of between about 0 and 1 mm and a total length of between about 0.3 mm to about 2.5 mm. Preferably, the microneedle is a hollow needle having a length of less than about 2.5 mm. Most preferably, the microneedle is a hollow needle having a length of less than about 1.7 mm. The composition comprising therapeutic cells and extracellular matrix component are delivered into the skin to a depth of at least about 0.3 mm and no more than about 2.5 mm by the microneedle.

The compositions for use in methods of such embodiments may include a variety of additional steps including, for example, cleaning the surface tissue at the site of applying and the like. In such embodiments, the composition can be applied to the surface tissue one or more times each day, and applying can be carried out for a period of at least 1 month, 2 months, 3 months, 4 months, 6 months, 8 months or 12 months.

In such embodiments, the composition can be applied to the surface tissue one or more times each day, and applying can be carried out for a period of at least 1 month, 2 months, 3 months, 4 months, 6 months, 8 months or 12 months. In some embodiments, the composition may be administered once, as needed, once daily, twice daily, three times a day, once a week, twice a week, every other week, every other day, or the like. A dosing cycle may include administration for about 1 week, about 2 weeks, about 3 weeks, about 4 weeks, about 5 weeks, about 6 weeks, about 7 weeks, about 8 weeks, about 9 weeks, or about 10 weeks. After this cycle, a subsequent cycle may begin approximately 1, 2, 3, 4, 5, 6, 7, 8, 9, 10, 11, or 12 weeks later. The treatment regime may include 1, 2, 3, 4, 5, or 6 cycles, each cycle being spaced apart by approximately 1, 2, 3, 4, 5, 6, 7, 8, 9, 10, 11, or 12 weeks.

The methods disclosed herein may include a variety of additional steps including, for example, cleaning the surface tissue at the site of applying and the like. For example, prior to administering the composition the tissue surface is ablated by electromagnetic radiation, laser, dermal abrasion, chemical peel, ultrasound, heating, cooling, or by a needle.

Abrasion of the outer layer or epidermis of the skin (dermal abrasion) is desirable to smooth or blend scars, blemishes, or other skin conditions that may be caused by, for example, acne, sun exposure, and aging. Standard techniques used to abrade the skin have generally been separated into two fields referred to as dermabrasion and microdermabrasion. Both techniques remove portions of the epidermis called the stratum corneum, which the body interprets as a mild injury. The body then replaces the lost skin cells, resulting in a new outer layer of skin. Additionally, despite the mild edema and erythema associated with the procedures, the skin looks and feels smoother because of the new outer layer of skin.

Microdermabrasion refers generally to a procedure in which the surface of the skin is removed due to mechanical rubbing by a handpiece emitting a stream of sand or grit. For example, a handpiece can be used to direct an air flow containing tiny crystals of aluminum oxide, sodium chloride, or sodium bicarbonate. The momentum of the grit tends to wear away two to three cell layers of the skin with each pass of the handpiece. Alternatively, new "crystal-free" microdermabrasion techniques utilize a diamond-tipped handpiece without a stream of grit.

In some embodiments, prior to administering the composition the tissue surface is ablated with electromagnetic radiation, for instance using a so-called fractional laser treatment. By way of example, such methods employ electromagnetic radiation (EMR) having one or more wavelengths of between approximately 1,850 to 100,000 nanometers and with pulse widths of between approximately 1 femtosecond (1×10-15 s) to 10 milliseconds (10×10-3 s) with fluence in the range of from approximately 1 J/cm2 to 300 J/cm2. In other examples, the tissue is ablated with electromagnetic radiation having one or more wavelengths of between approximately 2,200 to 5,000 nanometers. In still other examples, the tissue is ablated with electromagnetic radiation having one or more wavelengths of between approximately 190 to 320 nanometers with fluence in the range of from 1 J/cm2 to 300 J/cm2. Optionally, conditions selected for ablating portions of the tissue minimize the coagulation zone of tissue damage, for instance by keeping the coagulation zone to a relatively small diameter surrounding the ablated void.

Electromagnetic radiation (EMR), particularly in the form of laser light or other optical radiation, has been used in a variety of cosmetic and medical applications, including uses in dermatology, dentistry, ophthalmology, gynecology, otorhinolaryngology and internal medicine. For most dermatological applications, EMR treatment can be performed with a device that delivers the EMR to the surface of the targeted tissue(s). EMR treatment is typically designed to (a) deliver one or more particular wavelengths (or a particular continuous range of wavelengths) of energy to a tissue to induce a particular chemical reaction, (b) deliver energy to a tissue to cause an increase in temperature, or (c) deliver energy to a tissue to damage or destroy cellular or extracellular structures, such as for skin remodeling. Examples of devices that have been used to treat the skin during cosmetic procedures such as skin rejuvenation include the Palomar^{®} LuxIR, the Palomar^{®} 1540, 1440 and 2940 Fractional Handpieces, the Reliant Fraxel^{®} SR Laser and similar devices by Lumenis, Alma Lasers, Sciton and many others.

In some embodiments, the compositions for use in methods and compositions disclosed herein can be used in combination with photodynamic therapy. Photodynamic therapy is a minimally invasive two-step medical procedure that uses photoactivatable drugs called photosensitizers to treat a range of diseases. First, a photosensitizer is administered and, once it has permeated the target tissue, the photosensitizer is then activated by exposure to a dose of electromagnetic (usually light) radiation at a particular wavelength. The compositions disclosed herein may contain a photosensitizer.

In some embodiments, any suitable photosensitizing agent or mixture of agents may be used herein. Generally, these will absorb radiation in the range of from about 380 nm to about 900 nm. As used herein, "photosensitizer" or "photosensitizing agent" preferably means a chemical compound which, when contacted by radiation of a certain wavelength, forms singlet oxygen or thermal energy. Non-limiting examples of photosensitizers include aminolevulinic acid esters, porphyrins, porphyrin derivatives, bacteriochlorins, isobacteriochlorins, phthalocyanine, naphthalocyanines, pyropheophorbides, sapphyrins, texaphyrins, tetrahydrochlorins, purpurins, porphycenes, phenothiaziniums, and metal complexes such as, but not limited to, tin, aluminum, zinc, lutetium, and tin ethyl etiopurpurin (SnET2), and combinations thereof.

### Aerosol Delivery

In some embodiments, the compositions disclosed herein can be delivered intranasally. In some embodiments, the compositions can be an aerosol formulation. In this context, the term "aerosol formulation" may refer to an aqueous composition, a dry powder composition, or a propellant-based composition. An aerosol formulation may be delivered to a subject in different ways, for example nasally or perorally, or by inhalation.

In some embodiments, the composition may be an aqueous solution formulation adapted for pulmonary delivery via a nebulizer, including jet, vibrating mesh, and static mesh or orifice nebulizers.

In some embodiments, the composition may be a dry powder comprising micronized particles, the particles having diameters from 0.1 to 10 microns and a mean diameter of between about 0.5 to 4.5 microns, about 1 to 4 microns, about 1 to 3.5 microns, about 1.5 to 3.5 microns, or about 2 to 3 microns. The dry powder formulation is suitable for use in either a dry powder inhaler device (DPI) or a pressurized metered dose inhaler (pMDI).

In some embodiments, the compositions disclosed herein may be in the form of propellant-based formulation which may also be referred to generically herein as "a pMDI formulation". A pMDI formulation is suitable for delivery by a device such as a pressurized metered dose inhaler (pMDI). In some embodiments, the compositions further comprise a propellant. Suitable propellants are known in the art and include, for example, halogen-substituted hydrocarbons, for example fluorine-substituted methanes, ethanes, propanes, butanes, cyclopropanes or cyclobutanes, particularly 1,1,1,2-tetrafluoroethane (HFA134a) and 1,1,1,2,3,3,3-heptafluoropropane (HFA227), or mixtures thereof.

### EXAMPLES

Although the present invention has been described in considerable detail with reference to certain preferred embodiments thereof, other versions are possible. Various aspects of the present invention will be illustrated with reference to the following non-limiting examples.

### EXAMPLE 1

### Hylauronic Acid and Biomimetic Peptides

Compositions containing of a mixture of peptides that promote hair growth were prepared. The peptides, sold under the tradename Renokin^{®}, include decapeptide-10, oligopeptide-54 (CG-Nokkin), decapeptide -18, acetyl decapeptide-3, and oligopeptide-42. The peptide compositions were prepared by mixing the peptides in saline along with a decoy molecule of hyaluronic acid with a molecular weight of 10,000 daltons, 20,000 daltons, 40,000 daltons (comparative), 60,000 daltons (comparative), or 100,000 daltons (comparative). Control formulations were comprised of the peptides alone and of saline alone.

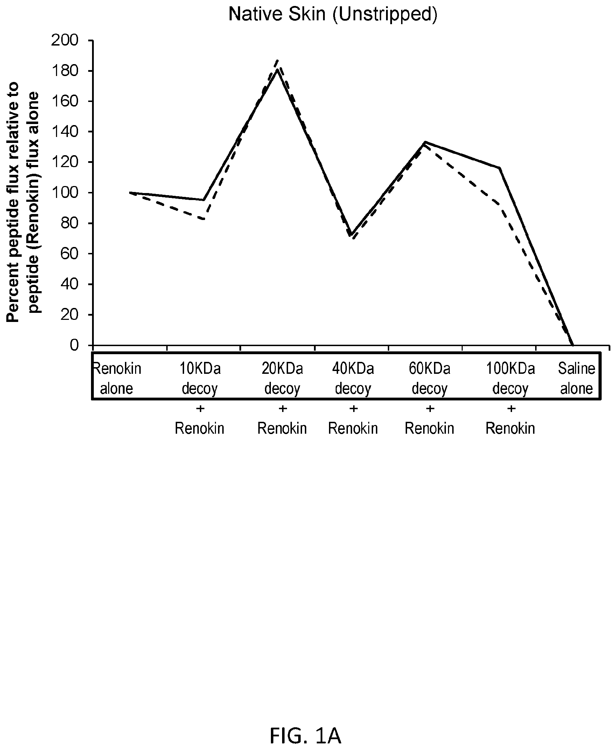

FIG. 1A shows the results for the studies conducted using skin with intact stratum corneum. This demonstrates partially passive binding, receptor mediated enhancement patterns are present and bimodal specific enhancement is present; nonspecific water enhancement would increase as size increases so the enhanced penetration effect is specific. Addition of progressively larger molecular weights reverses the benefit even with dead skin present.

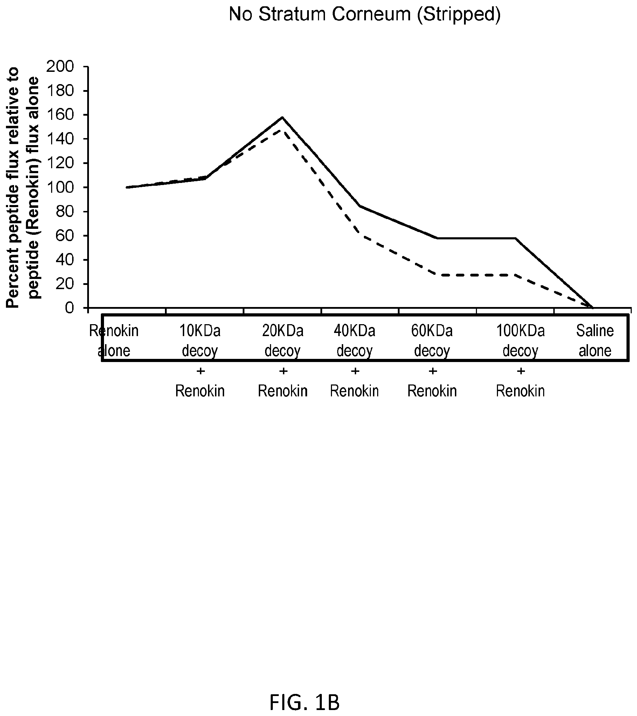

FIG. 1B shows the results for the studies conducted using skin with stratum corneum stripped off using the tape stripping method. This demonstrates active binding, receptor mediated enhancement pattern across viable skin layers without stratum corneum (i.e. without water enhancement effect at all) and specific enhancement present based on MW; larger MW not only abolishes enhancement but retards penetration across cells in the viable skin layers which present the barrier to deep epidermal and dermal penetration.

The percent of peptide flux relative to flux of peptide from the composition of peptide alone is shown for each of the test compositions. Each composition was tested twice, the first study indicated by the solid line, and the second study by the dashed line. Hyaluronic acid with a molecular weight up to 300,000 Da is known to be able to penetrate skin. The data in FIGS. 1A-1B show that delivery of the peptides using a hyaluronic acid molecule of less than about 40,000 Da is via a delivery path different than that for a hyaluronic acid molecule of greater than 40,000 Da, and that neither delivery path is purely related to a hydration effect. When stratum corneum is present on the skin (FIG. 1A), a peak in peptide delivery is observed from compositions with a hyaluronic acid of 20,000 Da and 60,000 Da. When stratum corneum is stripped from the skin (FIG. 1B), the peak achieved using a 60,000 Da hyaluronic acid decoy molecule is not observed, demonstrating that peptide delivery is not due to a hydration effect alone since enhancement of skin penetration due to hydration of the skin would increase with increasing decoy molecular weight. Further, since it is known that 100,000 Da hyaluronic acid penetrates the stratum corneum (Essendoubi, 2016), if the delivery observed from the present compositions was due to hydration it would be expected to observe peptide delivery from compositions with a 100,000 Da hyaluronic acid decoy molecule across skin with and without stratum corneum. FIG. 1B shows that the composition with 100,000 Da hyaluronic acid decoy molecule provided less delivery of peptide than did compositions with molecular weight hyaluronic acid. The compositions with a decoy molecule of 40,000 Da and less enhanced delivery of the peptides, relative to delivery from compositions with no decoy molecule (FIG. 1A).

### EXAMPLE 2

### Hylauronic Acid and Salicylate

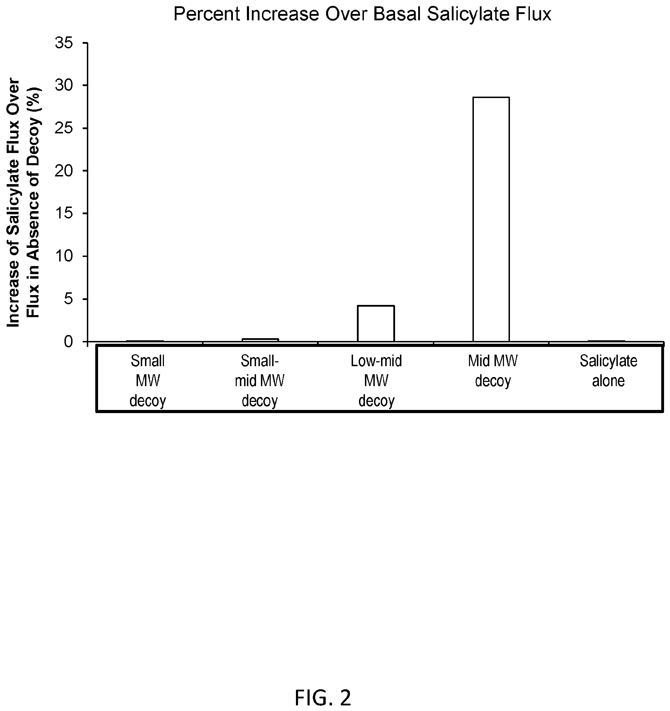

Compositions were prepared containing 1% salicylate and 1% of decoy molecule of hyaluronic acid with four molecular weights: small (5,000 Da to 10,000 Da), small to mid (10,000 Da to 20,000 Da), low to mid (20,000 Da to 30,000 Da), and mid (30,000 Da to 40,000 Da) (comparative). A control formulation containing salicylate alone was also prepared. The compositions were placed in Franz diffusion cells with skin separating the compartments of the diffusion cell. The concentration of salicylate in the receiver side of the diffusion cells was measured after a fixed time and the results are shown in FIG. 2.

The composition with the 10,000 Da to 20,000 Da decoy of hyaluronic acid achieved a 27% higher flux of salicylate compared to the flux of salicylate from the composition of salicylate alone. The 20,000 Da to 30,000 Da decoy molecule increased salicylate skin flux about 5% compared to the flux of salicylate from the composition of salicylate alone.

### EXAMPLE 3

### Hylauronic Acid and a Steriod

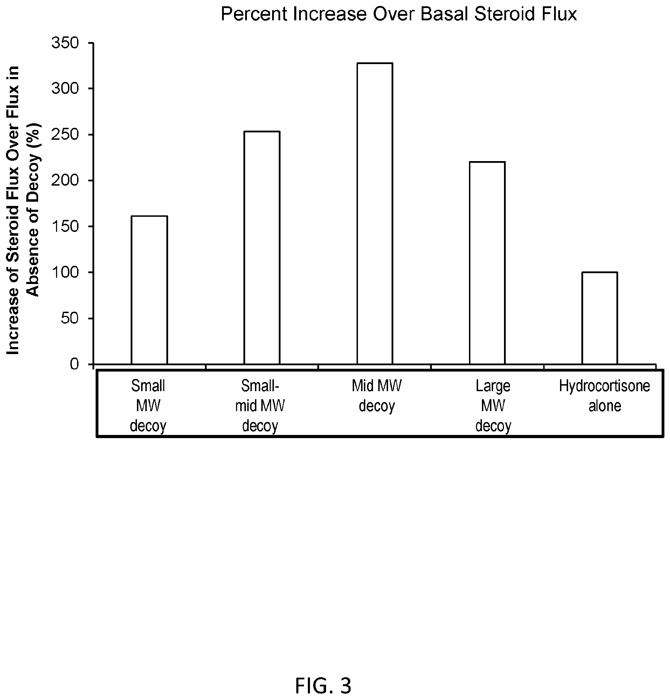

Compositions were prepared containing 1% hydrocortisone and 1% of decoy molecule of hyaluronic acid with four molecular weights: small (5,000 Da to 10,000 Da), small to mid (10,000 Da to 20,000 Da), low to mid (20,000 Da to 30,000 Da), and mid (30,000 Da to 40,000 Da) (comparative). A control formulation containing hydrocortisone alone was also prepared. The compositions were placed in Franz diffusion cells with skin separating the compartments of the diffusion cell. The concentration of salicylate in the receiver side of the diffusion cells was measured after a fixed time and the results are shown in FIG. 3.

The compositions with the hyaluronic acid decoy molecules increased delivery of hydrocortisone across the skin, with the mid-sized decoy of 20,000 Da to 30,000 Da giving a 325% increase in hydrocortisone flux compared to flux of hydrocortisone from a composition lacking the decoy molecule. The small-to-mid-sized decoy molecule with a molecular weight of about 10,000 Da to 20,000 Da increased salicylate skin flux about 250% compared to flux of hydrocortisone from a composition with no decoy molecule.

### EXAMPLE 4

### Elastin and Lidocaine

Delivery of lidocaine across skin was evaluated using compositions containing an elastin decoy molecule. Compositions containing of 1 wt. % lidocaine and 0.5 wt % of a decoy of elastin in saline were prepared with three molecular weights: very very small (2,000 Da to 5,000 Da), very small (5,000 Da to 10,000 Da), and small (10,000 Da to 20,000 Da).

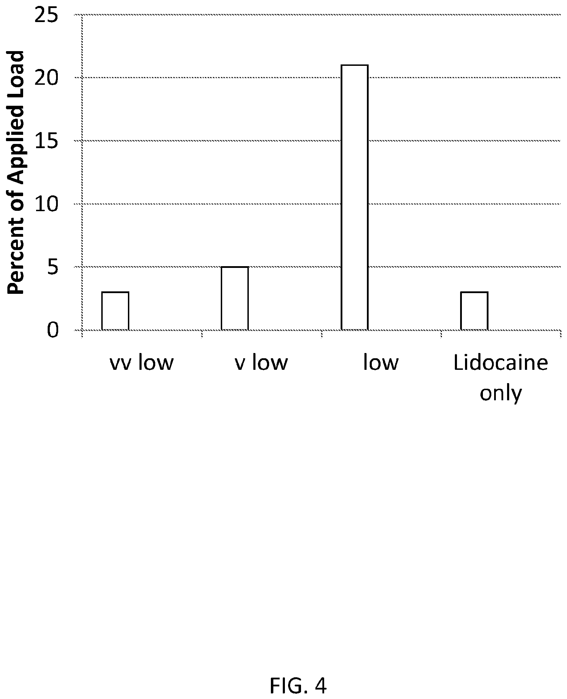

Viable porcine skin was obtained and used to produce mid-dermal grafts (0.045-0.055 units). The grafts were positioned in transcutaneous flux devices. Flow in the devices was maintained at the lowest setting and all receptor fluid was collected for each replicate (n=8 for each of the test formulation and the control formulation). Flux was continued for 12-20 hours with samples applied and left on donor skin surfaces. The skin for each cell (each chamber) was washed, then homogenized. The clarified homogenate solution and the flow through samples were assayed for lidocaine content using spectroscopy. After a 12-20 hour permeation period, the concentration of lidocaine in the skin was determined. The results are shown in FIG. 4 as the percent of applied lidocaine.

The lidocaine formulation with no decoy molecule achieved 3% penetration. Addition of an elastin decoy molecule having a small molecular weight (10,000 Da to 20,000 Da) enhanced skin penetration by about 7 fold (significant improvement in penetration, p=0.0001).

### EXAMPLE 5

### Hylauronic Acid and Minocycline

Oral minocycline HCl is highly effective but limited by ototoxicity and emerging resistance. Majority of physicians would use topical minocycline versus oral. However, topical application is currently less effective than oral because minocycline does not effectively cross skin. As a result, higher concentrations must be used and these discolor skin and textiles.

Delivery of minocycline into porcine skin *in vitro* was measured and compared to delivery of minocycline from a composition of minocycline in saline (i.e, with no decoy molecule). Compositions were prepared containing of 1 wt. % minocycline and 1% of decoy molecule of hyaluronic acid with three molecular weights: 10,000 Da mean, 20,000 Da mean, and 30,000 Da mean. A control formulation containing 1 wt % minocycline in saline was also prepared

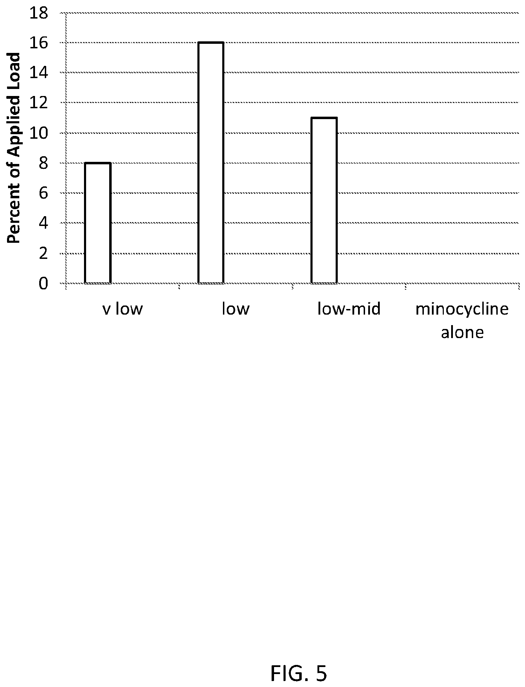

FIG. 5 shows the results of the study, where the amount of minocycline in tissue, µg minocycline/g tissue, delivered into the porcine skin grafts from the topical formulations of minocycline and sodium hyaluronate is shown by the bars with dashed fill and from the topical formulation of minocycline without sodium hyaluronate by the bar with open fill. Although minocycline had low native penetration, the polysaccharide-based decoys enhanced penetration significantly (p=0.0004). These results confirm that a decoy-mediated strategy can afford high penetration of a topical minocycline. A decoy molecule with a low molecular weight increased the very low basal penetration of minocycline to levels that can achieve higher tissue concentrations than oral while avoiding discoloration and systemic side effects. A topical composition containing minocycline with a decoy molecule can be used for treating or ameloriating skin structure infections or disorders, such as cellulitis.

### EXAMPLE 6

### Compositions For Protection of Skin from UVA/UVB Rays

Current chemical agents used for sunblock have poor compliance due to thick bases, incompatibility with cosmetics, and short duration. By enhancing function of existing agents, it becomes possible to develop a more effective sunblock, a sunblock which is resistant to rubbing off, and/or a more desirable formulation feel and use with other products (to induce better compliance).

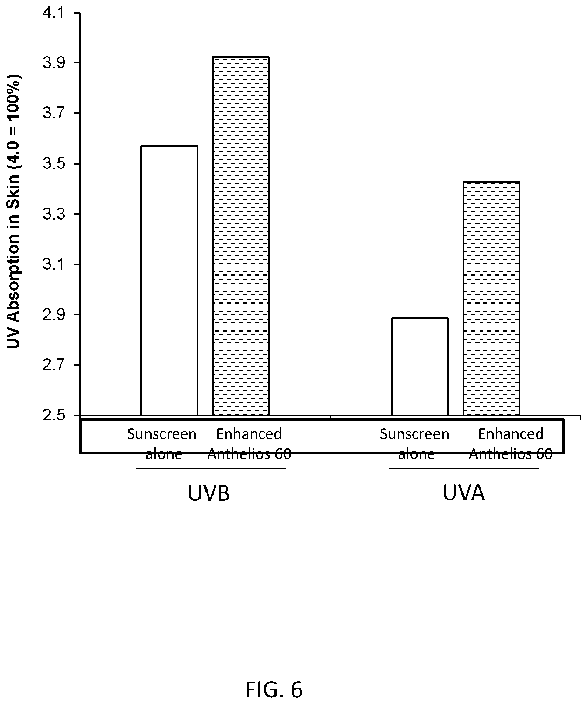

In this study, compositions for protection of skin from UV-A and/or UV-B exposure were prepared and tested. Groups include A) Laroche Posay Anthelios 60 Sunblock spiked with 1:10 saline (n=10 replicates), or B) Laroche Posay Anthelios 60 Sunblock spiked with 1:10 1% sodium hyaluronate of molecular weight 10,000 ("enhanced Anthelios 60") in saline (n=10 replicates) in donor cells. Flow was maintained at the lowest setting and all receptor fluid was collected for each replicate. Flux was continued for 12-20 hours with samples applied and left on donor surfaces. The skin for each cell (each chamber) was washed, then then punch biopsied, placed into 96 well plates and employed in full range UV spectra. UV absorbance per group was determined by wavelength for each group and UVA and UVB values determined from the appropriate wavelengths. Results are shown in FIGS. 6, 7A-7B, and 8.

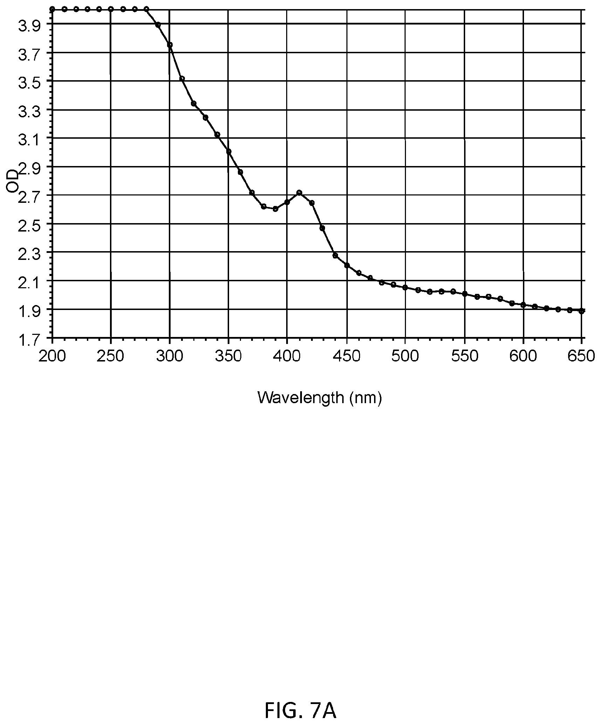

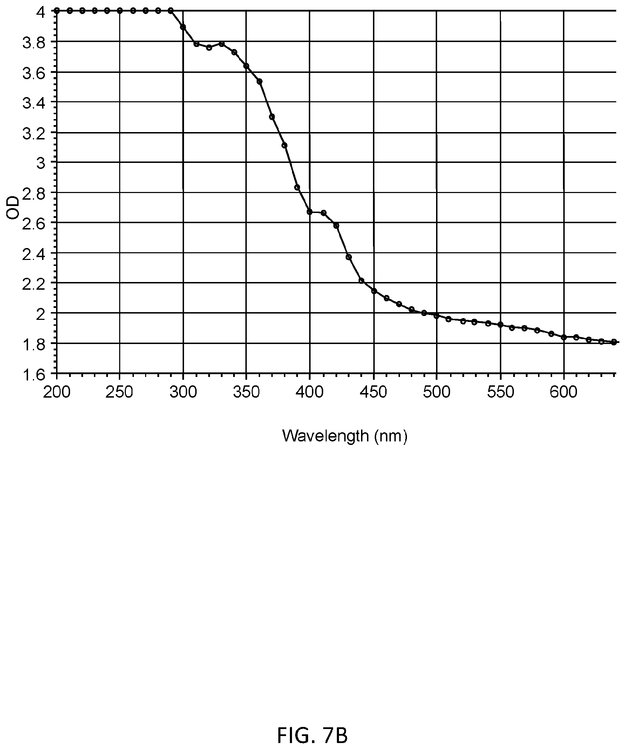

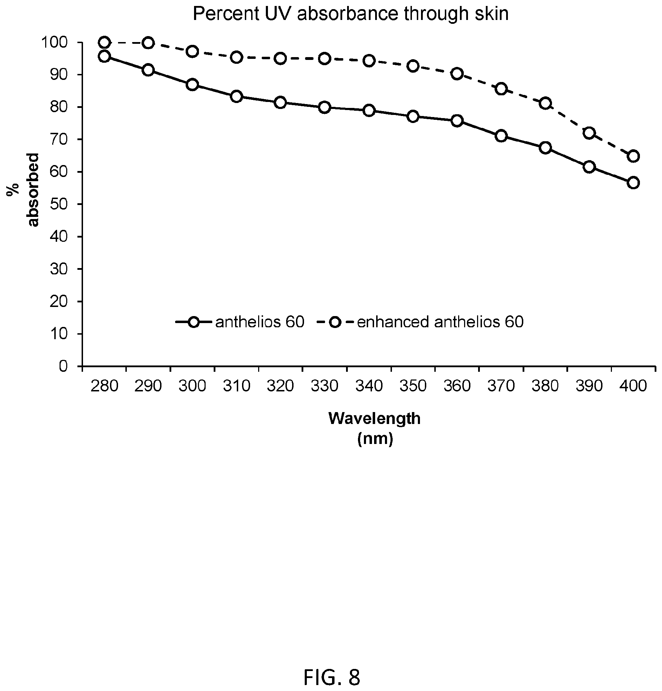

Addition of an enhancer which has no UV absorbance itself, increased the performance of a commercially available mix of UV blocking agents statistically significantly across both UVA (P=0.001) and UVB (P=0.001) as depicted in FIG. 6. Individual wavelength results by group are shown in FIG. 8 and one representative spectrum from each group is presented as FIGS. 7A and 7B.

The compositions with and without decoy molecule were tested to determine UV absorption in skin. FIG. 6 is a bar graph (4.0 corresponds to 100%) showing the absorption of UVA and UVB in skin, where the bars with dashed fill correspond with the sunscreen compositions with a decoy molecule and the solid white bars are sunscreen alone.

FIGS. 7A-7B are graphs of UV absorption as a function of wavelength, in nm, for commercially available sunscreen (Anthelios 60) (FIG. 7A) and for the commercially available sunscreen (Anthelios 60) with a decoy molecule, enhanced Anthelios 60 (FIG. 7B).

FIG. 8 is a graph showing the percent UV absorbance through skin as a function of wavelength, in nm, for commercially available sunscreen (Anthelios 60) (solid line) and for the commercially available sunscreen (Anthelios 60) with a decoy molecule, enhanced Anthelios 60 (dashed line).

### EXAMPLE 7

### Hyaluronic acid and Gabapentin

Delivery of gabapentin with hyaluronic acid into skin *in vitro* was measured using porcine skin grafts, and compared to delivery of gabapentin from a composition of gabapentin in saline (with no decoy molecule). Groups consisted of A) 1% gabapentin in saline (n=8 replicates), B) 1% gabapentin plus 1% sodium hyaluronate decoy of 3,000 Da in saline (n=8 replicates) or C) saline alone (n=8 replicates) in donor cells.

Viable porcine skin was processed to produce mid-dermal grafts (0.045-0.055 units) and the grafts were positioned in transcutaneous flux devices. Flow in the devices was maintained at the lowest setting and all receptor fluid was collected for each replicate (n=8 for each of the test formulation and control formulations). Flux was continued for 12-20 hours with samples applied and left on donor skin surfaces. The skin for each cell (each chamber) was washed, then employed in an assay of gabapentin content within the skin sample using a UPLC-mass spectrometer method. Briefly, tissues were incubated overnight in 0.5 mL of 50% acetonitrile in deionized water at 55° C with agitation. Calibration standards and tissue extraction solvent samples were diluted 10x in deionized water before analysis. Diluted standards and samples were analyzed at 1 µL injection volumes. Concentrations were reported as µg/g of gabapentin in tissue.

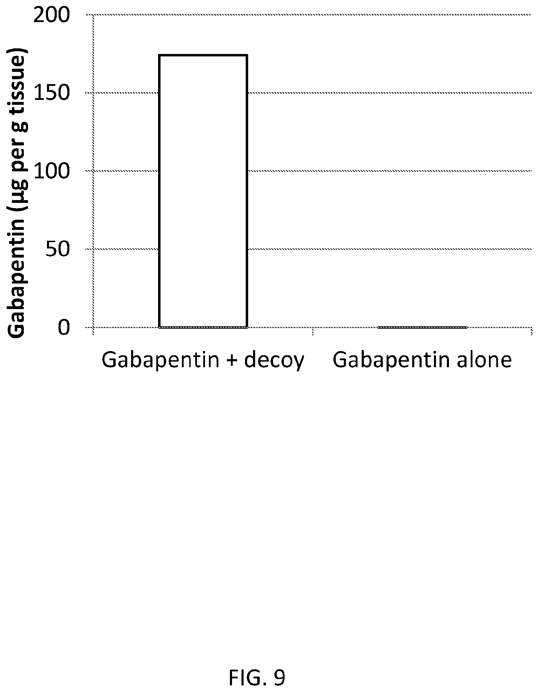

FIG. 9 shows the results of the study, where the amount of gabapentin in tissue, µg gabapentin/g tissue, delivered into the porcine skin grafts from the topical formulation of gabapentin and sodium hyaluronate and the formulation of gabapentin without sodium hyaluronate are shown. Gabapentin alone did not yield significant penetration above saline (p=0.99) but gabapentin in the presence of the decoy achieved significant penetration versus both saline (p=0.018) and gabapentin alone (p=0.013). Specifically, gabapentin alone yielded tissue levels of 0.09 µg of gabapentin per gram of tissue while gabapentin with the addition of a decoy molecule yielded tissue levels of 174.01 µg of gabapentin per gram of tissue. Thus, the addition of a decoy molecule yielded a 1,900 fold increase in delivery of the agent to the skin, and a statistically significant increased penetration of gabapentin topically.

### EXAMPLE 8

### Hyaluronic acid and Palmitoyl-lysine-threonine-threonine-lysine-serine

A topical composition containing a cosmetic agent, palmitoyl-lysine-threonine-threonine-lysine-serine (pal-KTTKS) and sodium hyaluronate (3,000 Da) as a decoy molecule were prepared. Groups consisted of A) 1% Pal-KTTKS spiked into Olay ProX (n=8 replicates), or B) 1% Pal-KTTKS spiked into Olay ProX plus 1% sodium hyaluronate decoy of 3,000 Da in saline (n=8 replicates).

Viable porcine skin was processed to produce mid-dermal grafts (0.045-0.055 units) and the grafts were positioned in transcutaneous flux devices. Flow in the devices was maintained at the lowest setting and all receptor fluid was collected for each replicate. Flux was continued for 12-20 hours with samples applied and left on donor skin surfaces. The skin for each cell (each chamber) was washed, then homogenized. The clarified homogenate solution and the flow through samples were then employed in an assay of pal-KTTKS content within the skin sample using a UPLC-mass spectrometer method.

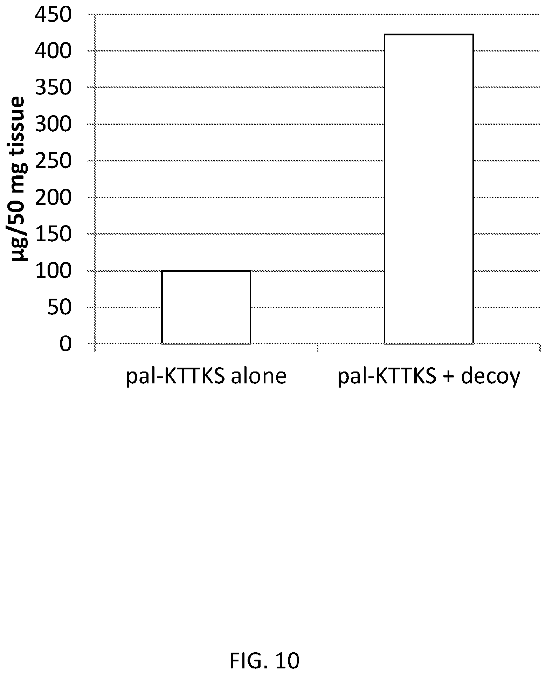

FIG. 10 shows the results of the study, where the amount of pal-KTTKS in the tissue (µg pal-KTTKS/50 mg tissue) delivered from the topical formulation of pal-KTTKS and sodium hyaluronate decoy and the topical formulation of pal-KTTKS without sodium hyaluronate are indicated. A formulation of pal-KTTKS alone (with no decoy molecule) after the 12-20 hour permeation period yielded about 100 µg pal-KTTKS/50 mg tissue. Addition of a decoy molecule improved permeation of the agent into the skin, with nearly 450 µg pal-KTTKS/50 mg tissue. Thus, the addition of a decoy molecule to the topical composition yielded a nearly 422% increased flux without optimization (P<0.01) in delivery of the agent to the skin. Thus, without any additional formulation change, a polysaccharide decoy provided substantial and significant enhancement in penetration of the most widely recognized peptidyl skincare active.

### EXAMPLE 9

### Ocular Delivery of FITC-dextran from Compositions Containing a Decoy

Intact fresh, viable porcine eyes were obtained with full orbit uninjured. Eyes were bathed to midline (lens down) in treatment solution overnight while suspended superiorly via ligature of the optic nerve. Compositions were prepared as follows: A) 5,000 Da FITC-dextran in saline (n=2 replicates), B) 5,000 Da FITC-dextran in 1% sodium hyaluronate of 3,000 Da in saline (n=2 replicates), C) 5,000 Da FTIC-dextran in 0.5% short elastin in saline (n=2 replicates), and D) saline alone.

Eyes were thoroughly washed 5 times in saline then snap frozen and analyzed with a reflectance confocal imaging system (Vivascope 1500) to noninvasively image and visualize penetration of the FITC-dextran. The confocal microscopy showed that though almost no gross signal was present within the lens, both polysaccharide and peptidyl decoy molecules provided for visible penetration of the FITC-dextran marker (drug model) to the aqueous humor, including the anterior and posterior chamber and ciliary body; to the structural elements including zonule and sclera; and to the vitreous humor including bathing the retina. Saline controls showed no granular fluorescence and no drug (marker) penetration since no FITC-dextran was present.

This experiment confirms that a 5,000 Da drug marker penetrated into the eye when combined with both classes of decoy. A similar experiment using both dextran and antibody markers at 150,000 MW confirmed penetration with both classes of decoys; though differing magnitudes of flux for 150,000 versus 5,000 MW, both exhibited penetration when applied topically to intact eyes.

### EXAMPLE 10

### Delivery of FITC-dextran to the Nail Unit from Compositions Containing a Decoy

A mixture of 1% 5,000 Da FITC-dextran and 1% 10,000 Da sodium hyaluronate was added to commercially available nail base at a 1:10 dilution. The material was applied to a toenail and allowed to stand for 3 hours. Confocal imaging was employed as before to view penetration of FITC-dextran into the nail plate. Images were acquired at 7 micron steps.

Very high levels of signal were present on the nail surface as expected. High levels of the 5,000 Da FITC-dextran conjugate were observed penetrating into the deepest layers of the nail plate as visualized by granular fluorescence patterns. Most antifungal and nutritional components of interest for the nail could thus be delivered through addition of a small decoy fragment.

### EXAMPLE 11

### Mucosal Delivery of Salicylate from Compositions Containing a Decoy Molecule

The compositions are contemplated for delivery of an agent to mucosal tissue, and a study was conducted using viable porcine buccal tissue to evaluate mucosal penetration of salicylate from compositions with an elastin decoy molecule. The following compositions were prepared for testing: A) 1% sodium salicylate in saline (n=4 replicates), or B) 1% sodium salicylate plus 0.5% short elastin fragment decoy (decoy) in saline (n=4 replicates).

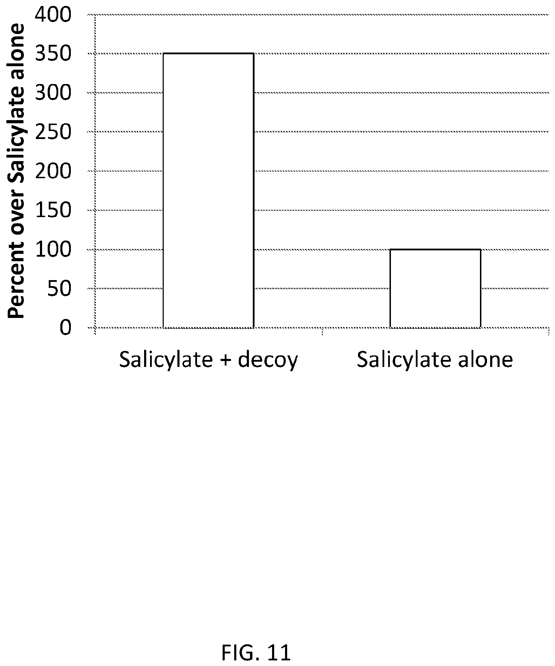

Viable porcine buccal tissue was obtained and grafts were produced. The grafts were placed in transcutaneous flux devices to measure mucosal penetration. Flow in the devices was maintained at the lowest setting and all receptor fluid was collected for each replicate (n=8 for each of the test formulation and control formulations). Flux was continued for 12-20 hours with samples applied and left on donor mucosal tissues. After the 12-20 hour test period tissue from each cell was washed, then homogenized. The clarified homogenate solution and the flow through samples were then employed in an assay of salicylate content via absorbance. The skin penetration of salicylate from a composition with an elastin decoy and from a composition with no decoy is shown in FIG. 11.

These results show that the addition of a decoy molecule to the composition achieved a 350% increase in mucosal penetration of salicylate.

### EXAMPLE 12

### Delivery of Antibody from Compositions Containing a Decoy Molecule

Compositions were prepared consisting of: A) 25µl of an alkaline phosphatase conjugated IgG antibody in saline (n=8 replicates), B) 25 µl of an alkaline phosphatase conjugated IgG antibody plus 1% sodium hyaluronate of 3,000 Da in saline (n=8 replicates), C) 25 µl of an alkaline phosphatase conjugated IgG antibody plus 1% sodium hyaluronate of 5,000 Da in saline (n=8 replicates), or D) 25µl of an alkaline phosphatase conjugated IgG antibody plus 1% sodium hyaluronate of 10,000 Da in saline (n=8 replicates) in donor cells.

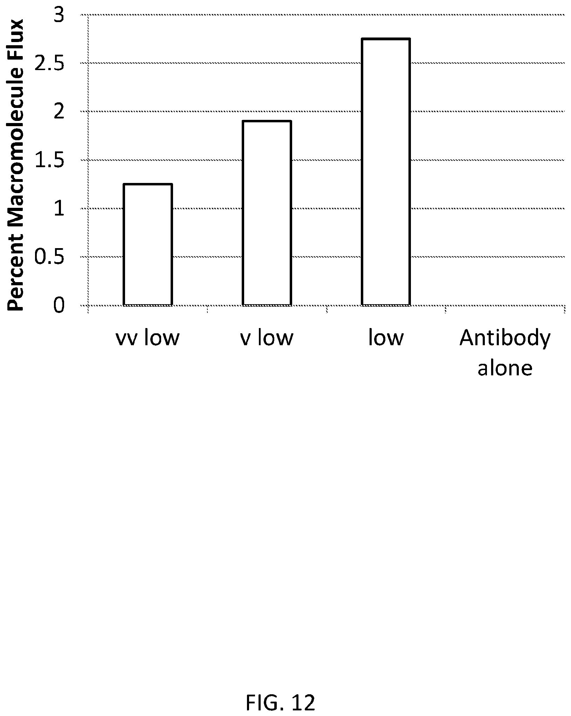

Viable porcine skin was processed to produce mid-dermal grafts (0.045-0.055 units) and the grafts were positioned in transcutaneous flux devices. Flow was maintained at the lowest setting and all receptor fluid was collected for each replicate. Flux was continued for 12-20 hours with samples applied and left on donor surfaces. The skin for each cell (each chamber) was washed and the flow through samples were then employed in an assay of alkaline phosphatase content via absorbance. The results are depicted in FIG. 12.

Antibody alone did not exhibit significant penetration as measured by alkaline phosphatase activity in flow through, while decoy-mediated penetration achieved over 2% penetration of the applied load. A statistically significant increase in penetration (P=0.003) was thus achieved simply by the addition of an decoy molecule. This approach thus affords a high percent penetration which enables development of a topical macromolecule therapeutic. Given that this antibody is 150-160 KD as tagged, delivery of virtually any derivatized antibody or antibody fragment is feasible as is delivery of similar molecules like botulinum toxins and derivatives or chimeras thereof.

### EXAMPLE 13

### Functional Antioxidant Capacity

Decoys of both hyaluronic acid (HA) and elastin (E6) afford increased penetration of a proprietary mixture of antioxidants from several different formulations. The same antioxidant blend was applied to skin with several different vehicle and decoy combinations as detailed below. Increased resistance to excess functional oxidative stress resulted.

Diffusion Chambers-Viable porcine skin was dermatomed to mid-dermal thickness, then punch biopsies were performed at n=6 per intended condition. A modified 6-block diffusion cell rig was prepared and set for a flow of 0.022ml/min. The formulations (200µl each) were applied to the top (donor) surface and massaged. The receptor fluid was collected for 12 hours for each cell for these experiments, then the skin was removed, cleaned, and snap-frozen for future cold homogenization in saline.

Formulations Applied to Porcine Skin
- Saline
- Formulation 1
- Formulation 1 + 1% HA
- Formulation 1 + 0.5% E6 (VGVAPG)
- Formulation 2
- Formulation 2 + 1% HA
- Formulation 2 + 0.5% E6
- Formulation 3
- Formulation 3 + 1% HA
- Formulation 3 + 0.5% E6
- Formulation 3 + 1% HA + 0.5% E6

Invitrogen Amplex Red Kit (Cat#A22188): The Amplex^{®} Red reagent (10-acetyl-3,7-dihydroxyphenoxazine) in the presence of HRP reacts with H₂O₂ in a 1:1 stoichiometry to produce the red-fluorescent oxidation product, resorufin. We employ the kit as a baseline measurement of reactive oxygen species (as the kit was designed) to ensure no aberrant ROS baseline values were present. We then deliberately introduce oxidative stress and watch how each flow-through sample responds. Kit directions were followed for solution prep and reaction setup.

Reactions were incubated at 30° C for 30 minutes, protected from light and mixed for 5 seconds every 5 minutes (in plate reader). Measure absorbance at 260 nm (reference value to ensure normality) and 560 nm (resorufin) and record values as Baseline (pre-stress). Absorbance was selected instead of fluorescence to allow faster reads post-spike (approximately 1 minute per cycle). For each point, subtract the value derived from average of zero-H₂O₂ control wells (n=2).

Add 20 uL spike of 0.1mM H₂O₂ stock to each well rapidly then measure absorbance at 260 nm and 560 nm and record values as Stress time zero. Measure dynamic cycles continuously through 5 cycles (approximately 5 minutes) then again at 10 min and 15 min. The multiple reads are to ensure the peak value and linear range can be assessed since resorufin can itself undergo a second oxidation to a non-absorbant/fluorescent state due to the excess H₂O₂ from the spike.

Formulation 1 formulation achieved a mean of 5.15% antioxidant capacity over normal skin controls (saline-treated). Though not statistically significant (p>0.2), the antioxidant capacity of Formulation 1-treated skin was consistently greater than that of saline-treated skin.

All subsequent formulation comparisons were made relative to the Formulation 1 formulation as a reference antioxidant capacity. In this way, the increase in capacity versus current base could be assessed without direct measurement of individual species.

HA Formulations: HA increased the antioxidant capacity of receptor fluid for each base, but there were notable differences from formulation to formulation:

| Formulation | Native + HA | Sans1 + HA | Sans2 + HA |
|---|---|---|---|
| Antioxidant capacity versus Native | 200-210% | 414% but rapidly declining to appx 100% | 316% to 360% |
| Significance | P>0.2 | *P=0.019 | *P=0.029; *P=0.039 |

Overall, the highest significant antioxidant capacity increases were observed when HA was added to the Formulation 3 base.

E6 afforded consistent increases in antioxidant capacity versus Formulation 1 skin though none achieved p<0.05 (most p<0.08) due to small sample size and lower increases. Since the sans bases were designed around HA behavior rather than E6, there were not significant differences in E6 enhancement from formulation to formulation. Unlike previously observed for other actives in other formulation bases, E6 did not attain as high an increase in antioxidant capacity as observed for HA.

| Formulation | Native + E6 | Sans1 + E6 | Sans2 + E6 |
|---|---|---|---|
| Antioxidant capacity versus Native | 162% | 165% | 135% |

### EXAMPLE 14

### FTIC-dextran Confocal Skin Analysis

Real time confocal microscopy imaging was performed on human subjects using a VivaScope^{®} 1500 to visualize penetration of various sized FITC-dextran conjugates up to 150,000 Da across hair bearing skin (dorsal forearm) and non-hair bearing skin (volar forearm). Groups were prepared in saline and consisted of 1% 5,000 Da FITC-dextran or 1% 5,000 Da FITC-dextran plus 1% sodium hyaluronate having average molecular weights of 5,000 Da to 20,000 Da in saline. Similar results were obtained using 0.5% elastin fragments (E6) having molecular weights of 10,000 Da to 20,000 Da in place of HA decoy.

### EXAMPLE 15

### Summary

It will be appreciated that Examples 1-6, 11, 12 illustrate hyaluronic acid as a decoy molecule exemplary of the decoy molecules contemplated herein. As described above, decoy molecules of collagen and elastin are contemplated, where the molecular weight of the decoy molecule can be selected to tailor the delivery of the agent of interest across the skin. The table below summarizes the effect of the decoy molecule (using the hyaluronic acid as exemplary) on the transdermal delivery of a small molecule compound (e.g, one with a molecular weight of less than about 850 Da), on the transdermal delivery of a macromolecule compound (e.g, a peptide or a protein), on the extent of penetration of the decoy molecule into skin upon topical application, and on the enhancement of water content in skin by the decoy molecule, on a scale using + symbols to reflect extent of the effect. As seen, there is a disconnect between skin penetration of the decoy molecule, hydration of skin due to the decoy molecule and the delivery of the compound into the skin, indicating that the enhanced skin delivery is not due to hydration or presence of the decoy, but to an activity of the decoy molecule in the skin.

| Decoy Molecule - Hyaluronic Acid | Delivery of a Small Molecule Compound | Delivery of a Macromolecule Compound | Hyaluronic Acid Skin Penetration | Water Content Enhancement in Skin |
|---|---|---|---|---|
| disaccharide (400 Da) (comparative) | 0 | 0 | +++++ | +++ |
| degraded 5000 Da | - | - | ++++ | +++ |
| 3000 Da | +++ | + | ++++ | +++ |
| 5000 Da | + | + | +++ | ++ |
| 10,000Da | +++ | ++ | ++ | + |
| 20,000 Da | ++++ | +++++ | ++ | + |
| 100,000 Da degraded (comparative) | ++++ | +++ | +++ | + |
| 100,000 Da (comparative) | 0 | 0 | +/- | +/- |

### EXAMPLE 16

The following compounds will be made and tested for increased flux compared to compositions containing no decoy molecule:

### Collagen and Vitamin C

Compositions containing vitamin C and a decoy molecule of collagen with three molecular weights designated A1, B1, C1 in saline will be prepared. A control formulation comprised of vitamin C in saline will also be tested. The compositions will be placed in Franz diffusion cells with skin separating the compartments of the diffusion cell. The concentration of vitamin C in the receiver side of the diffusion cells will be measured after a fixed time.

### Collagen and Diclofenac

Compositions containing diclofenac and a decoy molecule of collagen with three molecular weights of 5,000 Da, 25,000 Da and 50,000 Da (comparative) in saline will be prepared. A control formulation comprised of diclofenac in saline will also be tested. The compositions will be placed in Franz diffusion cells with skin separating the compartments of the diffusion cell. The concentration of diclofenac in the receiver side of the diffusion cells will be measured after a fixed time.

### Elastin and Niacinamide

Compositions containing niacinamide and a decoy molecule of elastin with three molecular weights 5,000 Da, 25,000 Da and 50,000 Da (comparative) in saline will be prepared. A control formulation comprised of niacinamide in saline will also be tested. The compositions will be placed in Franz diffusion cells with skin separating the compartments of the diffusion cell. The concentration of niacinamide in the receiver side of the diffusion cells is measured after a fixed time.

### Elastin and Naproxen

Compositions containing naproxen and a decoy molecule of elastin with three molecular weights 5,000 Da, 25,000 Da, and 50,000 Da (comparative) in saline will be prepared. A control formulation containing naproxen in saline will also be tested. The compositions will be placed in Franz diffusion cells with skin separating the compartments of the diffusion cell. The concentration of naproxen in the receiver side of the diffusion cells will be measured after a fixed time.

### Topical administration of bimatoprost for hair growth

Compositions will be prepared containing 0.01% bimatoprost and a 0.5% of a decoy molecule of elastin fragments with molecular weight ranges: small (5,000 Da to 10,000 Da), small to mid (10,000 Da to 20,000 Da), low to mid (20,000 Da to 30,000 Da), and mid (30,000 Da to 40,000 Da) (comparative). Control formulations containing 0.01% bimatoprost alone and saline alone will also be prepared. The compositions will be applied to subjects who have recently completed a cycle of chemotherapy approximately 21 days prior and experienced near total scalp hair loss. Subjects treated with compositions containing the decoys are expected to achieve faster rates of hair growth at 1, 2, and 4 weeks relative to comparable controls. Additionally, length, thickness, and density of hair are expected to be greater in subjects treated with compositions containing the decoys.

### Decoy-enhanced color treatment for hair shafts

Compositions containing a commercially available hair dye formulations will be spiked with 1% of decoy molecule of hyaluronic acid with low to mid molecular weight (20,000 Da to 30,000 Da) will be prepared and compared to the dye alone. The compositions will be applied to half of scalp hair shafts each (intra-subject control) and will be removed after 30 minutes. The depth of color will be assessed after rinsing, after one week, and after 4 weeks. The hair shafts treated with the composition containing the decoys are expected to demonstrate greater richness, depth, and persistence of color.

### Bimatoprost for dry eye

Compositions will be prepared containing 0.01% bimatoprost and a 0.5% of a decoy molecule of elastin fragments with molecular weight ranges: small (5,000 Da to 10,000 Da), small to mid (10,000 Da to 20,000 Da), low to mid (20,000 Da to 30,000 Da), and mid (30,000 Da to 40,000 Da) (comparative). Control formulations containing 0.01% bimatoprost alone and saline alone will also be prepared. The compositions will be applied to subject's eye surface as eye drops. Subjects treated with compositions containing the decoys are expected to recover from dry eye relative to comparable controls.

### EXAMPLE 17

### TOPICAL PEPTIDYL DECOY MEDIATED ENHANCEMENT OF SALICYLATE DELIVERY THROUGH MUCOSAL SURFACES

Porcine buccal tissue was harvested and cleaned of additional tissue. Punch biopsies (n=4 each) were obtained and employed in a transcutaneous flux rig per relevant protocols. Groups consisted of (1) 0.5% elastin peptide E6 + 1% salicylate in normal saline; (2) 1% salicylate in normal saline; and (3) Saline alone. Flux was continued for 12-20 hours with samples applied and left on donor surfaces. Flow through samples were analyzed for salicylate content via microplate reader (Molecular Devices Spectramax M3). The addition of the peptidyl decoy E6 afforded a 351.8% increase in salicylate penetration through buccal tissue to the flow through. Although salicylate alone showed penetration through the tissue, the overall penetration was not statistically significant over saline alone. Addition of the decoy to salicylate afforded statistically significant increases however (p=0.036).

### EXAMPLE 18

### Topical application of sildenafil

Replicates of mid-dermal grafts were employed in a transcutaneous flux rig per relevant protocols. Groups consisted of (1) 1% oligo- and polysaccharide decoy (hyaluronic acid chains of a very short length) + 1% sildenafil in normal saline (n=10); (21% sildenafil in normal saline (n=10); and (3) Saline alone (n=2). Flux was continued for 12-20 hours with samples applied and left on donor surfaces. The skin for each cell (each chamber) was washed, then homogenized; the clarified homogenate solution was assayed for sildenafil concentration and read in a 96 well microplate assay via a Molecular Devices Spectramax M3. Sildenafil alone was not detectable and not significant versus saline alone; sildenafil+HA penetrated at 13.8% flux to achieve a tissue concentration of 96.6 ug per graft. The results were highly statistically significant for superior penetration of sildenafil with decoy versus sildenafil alone (p=0.0003). Given 41% bioavailability of oral sildenafil and assuming uniform tissue distribution, the currently approved therapeutic dose of sildenafil only achieves a tissue concentration of 0.137 ug sildenafil per g of tissue in an average 75 kg male. Thus, the addition of the decoy would afford a topical that could potentially reduce the total and systemic exposure (risks and side effects) and achieve even higher efficacy than oral administration.

### EXAMPLE 19

### Topical enhancement of hair shaft penetration with saccharidyl decoys

Single donor hair shafts were obtained, washed with saline, and divided into 7 cm segments. Each segment (n=5 per group) were incubated for 5 minutes in the respective treatment solution; groups consisted of (1) 1.0% oligo- and polysaccharide decoy (hyaluronic acid chains of a very short length) + 1% FITC-dextran (MW3,000-5,000) in normal saline; (2) 1% FITC-dextran (MW3,000-5,000) in normal saline; and (3) Saline alone. Hair shafts were washed 5 times with an excess of saline then were divided into 1 cm segments (seven segments each per well) on a 96 well plate. Absorbance of FITC was then measured via microplate reader (Molecular Devices Spectramax M3). Although the dextran alone adhered significantly to the outer layers of the hair shaft to attain increased absorbance at 495 to 518 nm nm versus saline, the addition of short oligo- and polysaccharide decoys afforded a 234% rate of loading due to increased penetration into the shaft itself during the brief 5 minute treatment period. Microscopy confirmed the results.

### EXAMPLE 20

### Topical enhancement of bone and periosteal penetration via saccharidyl/peptidyl decoys.

Porcine bones were obtained and carefully cleaned of all soft tissue outside of the periosteum. Each bone had periosteum reflected in one portion to expose bone directly and the adjacent area retained intact periosteum in order to evaluate bone penetration and penetration of periosteum. The areas at the margins of the zone to be tested had a petroleum jelly applied to create a reservoir. 2 ml of test article was applied to each test area on the bone encompassing both periosteum-intact and exposed bone surfaces. Groups consisted of (1) 1.0% oligo- and polysaccharide decoy (hyaluronic acid chains of a very short length) + 1% FITC-dextran (MW3,000-5,000) in normal saline; (2) 1% FITC-dextran (MW3,000-5,000) in normal saline alone; and (3) 1.0% elastin fragment decoy + 1% FITC-dextran (MW3,000-5,000) in normal saline. Bones were allowed to incubate overnight then were washed with a large excess of saline five times before UV exposure, photography, and image analysis. All groups displayed some binding and retention of FITC-dextran to periosteum though the groups with decoys showed much higher and more uniform levels. In contrast, both groups with decoys showed high penetration and retention of FITC-dextran into the cortex of bone while the group without decoy was essentially undetectable.

### EXAMPLE 21

### Larger molecular weight decoys abolishes Flux

Replicates of mid-dermal grafts (grafts and punches for flux rig per Illustris sops), were employed in a transcutaneous flux rig per relevant protocols. Groups consisted of (1) 1% oligo- and polysaccharide decoy -hyaluronic acid chains of under 20K MW + 1% sodium salicylate in normal saline (n=6); (2) 0.9% oligo- and polysaccharide decoy -hyaluronic acid chains of under 40K MW + 0.1% hyaluronic acid chains of over 150K MW + 1% sodium salicylate in normal saline (n=6); (3) + 1% sodium salicylate in normal saline (n=6); and (4) normal saline alone (n=6). Flux was continued for 12-20 hours with samples applied and left on donor surfaces. The skin for each cell (each chamber) was washed, then homogenized; the clarified homogenate solution was assayed for sodium salicylate concentration and read in a 96 well microplate assay via a Molecular Devices Spectramax M3. Sodium salicylate had OD290 of 0.0165AU above saline so exhibited low level detectable penetration alone. Sodium salicylate with low MW alone as decoy (no large decoy) attained OD values of 0.1075AU (increase of 651.5%). Interestingly, the addition of a small portion of high MW HA to the low MW HA while holding total HA constant abolished ALL benefit of the decoy; the decoy with a trace of high MW HA was not significantly higher better than sodium salicylate alone and in fact was numerically closer to saline (-0.01AU lower but not statistically significant). Thus, the addition of a trace of high MW HA completely abolished the benefit of the low MW HA decoy. This result explained prior observations that low purity small MW HA (which was contaminated with high MW HA) did not demonstrate the benefits observed with a pure small MW HA.

### EXAMPLE 22

### Larger HA abolishes Flux - Addition of high MW "impurity" to pure low MW composition

Replicates of mid-dermal grafts (grafts and punches for flux rig per Illustris sops), were employed in a transcutaneous flux rig per relevant protocols. Groups consisted of (1) 1% oligo- and polysaccharide decoy -hyaluronic acid chains of under 20K MW (confirmed by UPLC to not contain detectable MW>50K) + 1% FITC-dextran (MW 150K) in normal saline (n=6); (2) 1% oligo- and polysaccharide decoy -hyaluronic acid chains of under 20K MW + 1% FITC-dextran (MW 150K) + 0.1% hyaluronic acid chains of 60K MW "added as a contaminant" (n=6); (3) 1% oligo- and polysaccharide decoy - hyaluronic acid chains of under 20K MW + 1% FITC-dextran (MW 150K) + 0.1% hyaluronic acid chains of 100K MW "added as a contaminant" (n=6); and (4) 1% FITC-dextran (MW 150K) in normal saline (n=6). Flux was continued for 12-20 hours with samples applied and left on donor surfaces. The skin for each cell (each chamber) was washed, then homogenized; the clarified homogenate solution was assayed for FITC concentration by fluorescence as read in a 96 well microplate via a Molecular Devices Spectramax M3. Results are presented in Table below.

| | Low MW decoy | Low MW decoy + 0.1% 60K decoy | Low MW decoy + 0.1% 100K decoy |
|---|---|---|---|
| Detectable Flux | 18.25% flux | 0.76% flux | 1.70% flux |
| (over saline/background) | (significant) | (same as background) | (same as background) |
| P vs saline | P=0.002 | P>0.05 (P=0.87) | P>0.05 (P=0.63) |

Consistent with prior literature, MW 150KD molecule in saline was at background levels so 1% FITC-dextran (MW 150K) in saline gave no detectable flux. In contrast 1% FITC-dextran (MW 150K) with a pure low MW alone as decoy (no large decoy) afforded detectable, statistically significant flux of over 18%. Interestingly, the addition of a relatively small portion of high MW HA to the low MW HA abolished ALL significant benefits of the decoy; the decoy with a trace of high MW HA was not significantly higher better than saline or background alone and was numerically close to background levels. Thus, the addition of a trace of high MW HA to an otherwise functional decoy mixture completely abolished the benefit of the low MW HA decoy statistically. This result explained prior observations that low purity small MW HA (which was contaminated with high MW HA) did not demonstrate the benefits observed with a pure small MW HA.

### EXAMPLE 23

### Larger HA abolishes Flux - Removal of high MW "impurity" from a broad MW population with a low mean MW

In order to replicate real-world manufacturing conditions and further elucidate the observation in example 1, a low mean MW HA was produced by degrading a very large MW HA (MW>1,800,000; from Habier) such that a broad, flat range of MWs was achieved with a mean of under 20K. Specifically, 18 mg (appx 300 units per mg) hyaluronidase (from Sigma) was added to 1L of a 3% hyaluronic acid solution and shaken gently for 72 hours at 37 degrees Celsius. The product was heat-killed to destroy and residual hyaluronidase, ethanol precipitated and vacuum dried. The product was tested via UPLC and found to be a broad range of MWs with mean of 18K. Samples of this low mean HA MW mixture were prepared as is; additional samples were first processed via MW cutoff filter of 50K (Amicon filter) such that the additional samples had ONLY 50K and under MW. The filtration did not change the calculated mean MW but truncated the curve such that the low MW HA no longer contained any detectable high MW impurities.

Replicates of mid-dermal grafts (grafts and punches for flux rig per Illustris sops), were employed in a transcutaneous flux rig per relevant protocols. Groups consisted of (1) purified mixed 1% oligo- and polysaccharide decoy -hyaluronic acid chains of under 18K MW (confirmed by UPLC to not contain detectable MW>50K) + 1% FITC-dextran (MW 150K) in normal saline (n=6); (2) unpurified 1% oligo- and polysaccharide decoy -hyaluronic acid chains of under 20K MW (which contained process contaminant/impurities of MW>50K) + 1% FITC-dextran (MW 150K) + 0.1% hyaluronic acid chains of 60K MW "added as a contaminant" (n=6); and (3) 1% FITC-dextran (MW 150K) in normal saline (n=6). Flux was continued for 12-20 hours with samples applied and left on donor surfaces. The skin for each cell (each chamber) was washed, then homogenized; the clarified homogenate solution was assayed for FITC concentration by fluorescence as read in a 96 well microplate via a Molecular Devices Spectramax M3. Results are presented in Table below.

| | Low MW decoy after filtration | Unpurified Low MW decoy (with high MW contamination) |
|---|---|---|
| Detectable Flux | 20.11% flux | 4.80% flux |
| (over saline/background) | (significant) | (same as background) |
| P vs saline | P=0.03 | P>0.05 (P=0.78) |

### EXAMPLE 24

### Proof of Concept 150,000 Da Facial Application

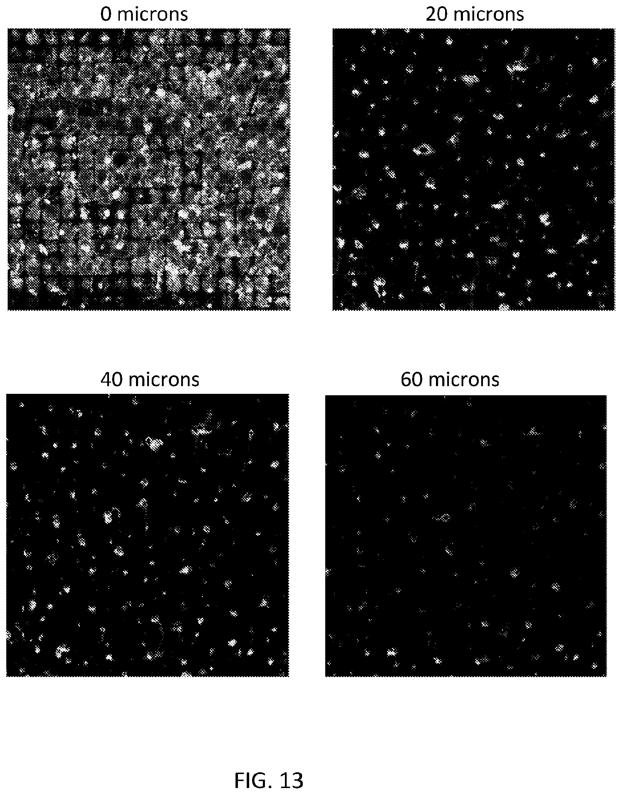

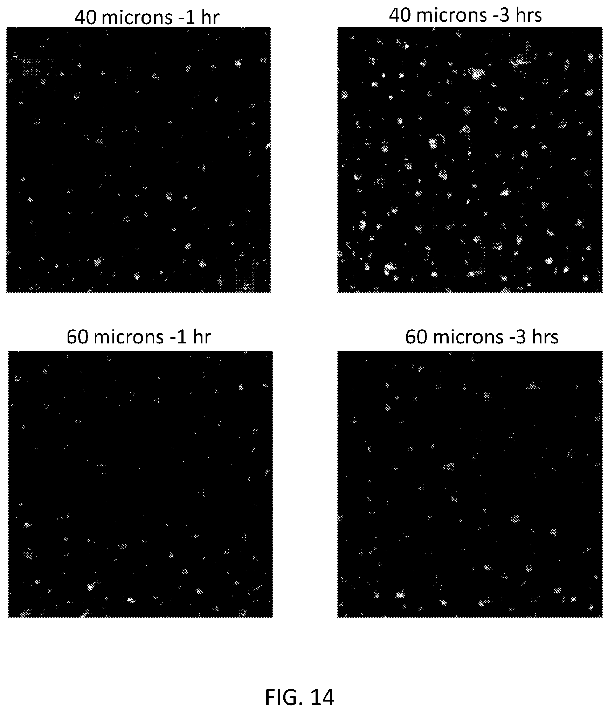

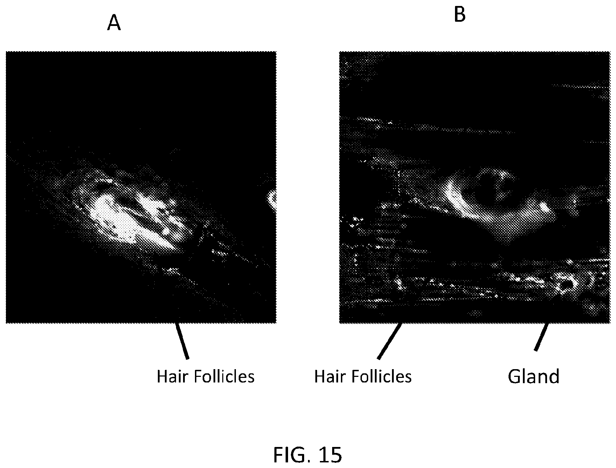

Methods. Real time confocal microscopy imaging was performed on human subjects using a VivaScope^{®} 3000 to visualize penetration of 150,000 Da FITC-dextran conjugate (mimicking large molecular weight molecules, such as botulinum toxin A) across glabellar and lateral canthus regions. 100 µL aliquots of 1% FITC-Dextran (150,000 Da average molecular weight) + 1% polysacccharide decoy in normal saline were applied to the indicated areas and massaged gently. direct sunlight was avoided and allowed to dwell for 1 hour. As per routine confocal methods, the area was prepped by cleansing gently with alcohol prep pads and then afixing confocal ring directly to the skin to be imaged. The ultrasound gel was applied and confocal probe was affixed. After imaging, gel was cleansed with excess normal saline and then test sample (FITC-Dextran with no decoy molecules) reapplied as above, allowed to dwell for an additional 2 hours as above before repeating imaging protocol. Confocal imaging was performed after 3 hrs of application at various depths- 0, 20, 40, and 60 microns. Results as shown in FIG. 13 demonstrate penetration of FITC-Dextran in the presence of decoy molecules in the glabellar region. Test sample did not exhibit any penetration. The penetration of FITC-Dextran increased over time, as shown in FIG. 14. FIG. 15 shows penetration of FITC-dextran in the presence of various decoy molecules ILS-20 (A) and ILS-3 (B) in the lateral canthus region after 3 hrs. ILS-20 is hyaluronic acid fragments of an average molecule weight of about 20,000 Da and ILS-3 is hyaluronic acid fragments of an average molecular weight of about 8,500 Da.

### EXAMPLE 25

### Topical Axillary Application of Botulinum Toxin A

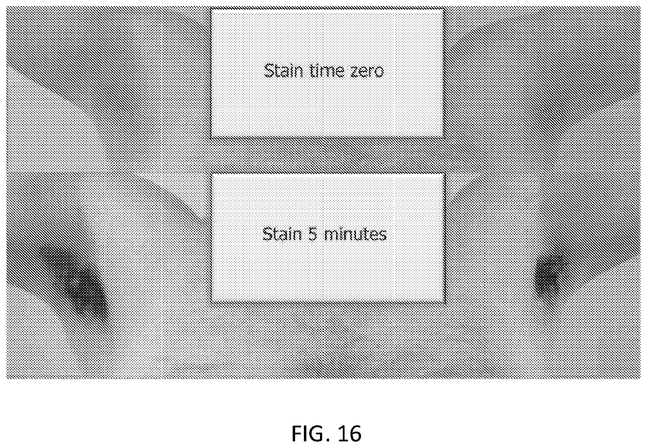

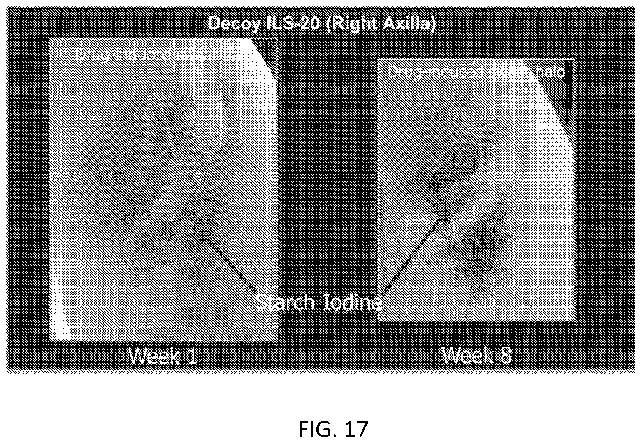

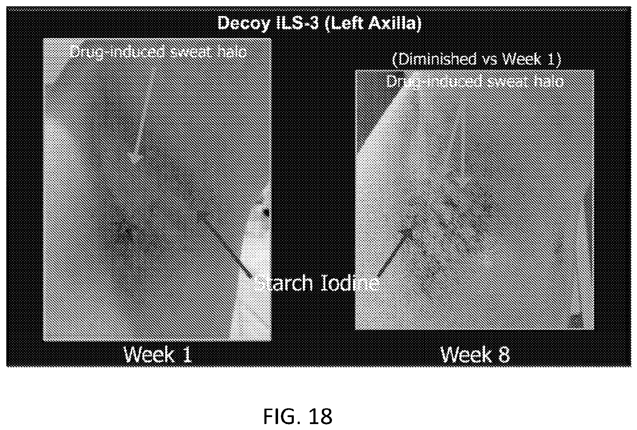

Method. Cleaned target areas with soap and water with thorough rinsing. Reconstituted 2 vials of 100U Botox^{®} with 100uL aliquots each of 1% FITC-Dextran (150,000D avg MW) + 1% polysaccharide decoy in NS (either ILS-20 or ILS-3). ILS-20 is hyaluronic acid fragments of an average molecule weight of about 20,000 Da and ILS-3 is hyaluronic acid fragments of an average molecular weight of about 8,500 Da. Recovered as much volume as possible with a syringe (did not remove cap; appx 80uL). Applied to the mid axillary line at the axillary fold and massaged gently to each axilla in a normohydrotic male subject. Covered with Tegaderm dressing for 1 hour to minimize loss or spread of test article. Removed dressing and washed gently with NS. On day 10, employed starch iodine testing per routine methods after standardized exercise routine to induce sweating. Results are shown in FIG. 16-18.

### EXAMPLE 26

A suspension of pluripotent stem cells will be injected into a solid tissue that has some decrements in function. The suspension includes fragments of elastin which allow improved permeation of cells throughout the tissue versus injections without the elastin fragments. After several weeks, the tissue is repopulated with differentiated cells sufficient to reverse the prior underlying disorder and achieve the desired tissue functions.

### EXAMPLE 27

A hydrogel containing engineered cells and hyaluronic acid fragments of a very short length is applied to a wound with some areas of dermal exposure. The hyaluronic acid chains afford more rapid permeation of the cells throughout the wound. The result is faster penetration, more uniform distribution, and higher retention than if the hyaluronic acid fragments were not present. As a result, the wound heals faster and achieve normal function with minimal scarring.

### EXAMPLE 28

A microneedle is applied to skin which has a topical suspension of engineered autologous cells on the surface. This suspension contains low MW fragments of elastin and hyaluronic acid at a concentration of 0.5% and 1.0% respectively. Due to the presence of these oligo/polypeptide and oligo/polysaccharide fragments, the cells are deposited more rapidly, distributed more uniformly throughout the skin, and retained at a higher rate than if the fragments had not been present. This results in skin showing reduced signs of atrophy and aging.

### EXAMPLE 29

A suspension of engineered autologous T cells is injected into a solid tissue that has contains residual cancer cells. This suspension includes fragments of elastin which will allow improved permeation of cells throughout the tissue versus injections without the elastin fragments. After several weeks, the tissue will no longer contain any viable tumor cells and the subject will be cancer free.

### EXAMPLE 30

### Diagnostic penetration of antibodies across intact skin

A subject with a high sugar diet and environmental pollution exposure will apply a hydrogel containing an oligo- and polysaccharide fragments (hyaluronic acid chains of a very short length) at 1% and mixture of full length fluorescent-tagged antibodies (of MW up to 160 Kd) to various advanced glycation end products at an optimal concentration and will allow the gel to stand for 3-6 hours. The treated area will then be cleaned and photographed with UV light or filters to visualize intensity and granularity of the antibodies via optical imaging. The photographs will be quantitatively evaluated for stippling above background which will reflect presence of defined antigens. The subject will subsequently repeat to monitor impact of subsequent interventions and lifestyle alterations.

### EXAMPLE 31

### Diagnostic penetration of antibodies across tissue barriers post excisional biopsy

A subject with Fitzpatrick type 2 skin and prior history of excision of precancerous lesions will present with a lesion highly suspicious for melanoma. An excisional biopsy with margins will be performed. The site of excision will be incubated with a solution containing an oligo- and polysaccharide fragments (hyaluronic acid chains of a very short length) and tagged antibody for melanoma antigen(s) for 30 minutes, followed by rinsing and visualization of tags. Areas with evidence of tagged antibody will undergo further resection such that all residual micro-invasions have been removed. Several years later, the subject will have the same approach applied to a new lesion in conjunction with Moh's surgery to ensure removal of cellular micro-invasions.

### EXAMPLE 32

### Interstitial Diagnostic Patch

A hydrogel containing a mixture of peptidyl and saccharidyl fragments of extracellular matrix will be applied to the shoulder of a subject. A discrete multidomain binding resin patch which allows individual assessment of hydrophobic, ionic, small molecule or protein components of serum or interstitial fluid will be applied subsequently and allowed to remain overnight. The patch will subsequently be examined to evaluate interstitial and serum markers of interest.

### EXAMPLE 33

### Emergency Diagnostics

A patch containing peptidyl and/or saccharidyl fragments of extracellular matrix plus a d-dimer-reactive compound which generates color in the presence of d-dimers will be applied to a subject in suspicion of pulmonary embolus while in transport to medical care from a rural area. The presence of d-dimers will inform and guide immediate and subsequent emergent care.

### EXAMPLE 34

### Hyaluronidase and Lidocaine

Delivery of lidocaine across skin will be evaluated using compositions containing hyaluronidase enzyme and decoy molecules. Compositions containing of 1 wt. % lidocaine, 0.5 wt % of decoy molecules (elastin fragments) of 5,000 Da to 10,000 Da, and 0.5 wt. % hyaluronidase will be prepared. Viable porcine skin will be obtained and used to produce mid-dermal grafts. The grafts will be positioned in transcutaneous flux devices. Flux will be continued for 12-20 hours and samples will be applied and left on donor skin surfaces. The skin from each cell (each chamber) will be washed and homogenized. The clarified homogenate solution and the flow through samples will be assayed for lidocaine content using spectroscopy.

### EXAMPLE 35

### Elastase and Minocycline

Delivery of minocycline across skin will be evaluated using compositions containing elastase enzyme, as described in Example 1. Delivery of minocycline into porcine skin in vitro will be measured and compared to delivery of minocycline from a composition of minocycline in saline (i.e, with no elastase and decoy molecules). Compositions will be prepared containing of 1 wt. % minocycline, 0.5 wt % of decoy molecules (hyaluronic acid fragments) of 5,000 Da to 10,000 Da., and 1 wt.% elastase. A topical composition containing minocycline with elastase can be used for treating or ameloriating skin structure infections or disorders, such as cellulitis.

### EXAMPLE 36

### Elastase and Vitamin C

Compositions containing vitamin C, decoy molecules (hyaluronic acid fragments) of 5,000 Da to 10,000 Da, and elastase enzyme will be prepared. A control formulation comprised of vitamin C in saline will also be tested. The compositions will be placed in Franz diffusion cells with skin separating the compartments of the diffusion cell. The concentration of vitamin C in the receiver side of the diffusion cells will be measured after a fixed time. The process will be repeated with compositions containing vitamin C and elastase, but without decoy molecules.

### EXAMPLE 37

### Topical administration of bimatoprost for hair growth

Compositions will be prepared containing 0.01 wt.% bimatoprost, about 0.5 wt.% of hyaluronidase and elastase combination, and 0.5 wt % of decoy molecules (hyaluronic acid fragments) of 5,000 Da to 10,000 Da. Control formulations containing 0.01 wt.% bimatoprost alone and saline alone will also be prepared. The compositions will be applied to subjects who have recently completed a cycle of chemotherapy approximately 21 days prior and experienced near total scalp hair loss. Subjects treated with compositions containing hyaluronidase/elastase are expected to achieve faster rates of hair growth at 1, 2, and 4 weeks relative to comparable controls. Additionally, length, thickness, and density of hair are expected to be greater in subjects treated with compositions containing hyaluronidase/elastase and decoy molecules.

### EXAMPLE 38

### Bimatoprost for dry eye

Compositions will be prepared containing 0.01 wt.% bimatoprost, 0.5 wt % of decoy molecules (hyaluronic acid fragments) of 5,000 Da to 10,000 Da., and a 0.5 wt.% of hyaluronidase. Control formulations containing 0.01% bimatoprost alone and saline alone will also be prepared. The compositions will be applied to subject's eye surface as eye drops. Subjects treated with compositions containing hyaluronidase are expected to recover from dry eye relative to comparable controls.

## Claims

1. A composition comprising:
a mixture of two components dissolved, suspended, dispersed or otherwise mixed in a carrier or vehicle, the first component consisting of a decoy molecule consisting of an extracellular matrix (ECM) component and/or fragment thereof, and the second component comprised of one or more active agents,
wherein the extracellular matrix component or fragment thereof has an average molecular weight of about 2,000 daltons to about 30,000 daltons and is present in the composition at between about 0.001 wt. % to about 10 wt. %.

2. The composition of claim 1, wherein the extracellular matrix component is selected from the group consisting of hyaluronic acid, collagen, fibronectin, elastin, lectin, and fragments thereof and combinations thereof.

3. The composition of claim 1 or claim 2, wherein the ECM component or fragment thereof comprises hyaluronic acid or elastin.

4. The composition of claim 1-3, wherein the composition comprises between about 0.1 wt. % to about 5 wt. % extracellular matrix component or fragment thereof.

5. The composition of claims 1-4, wherein the active agent is selected from the group consisting of small molecule drugs, macromolecular drugs, biologics, antibodies, chimeric antibodies, antibody fragments, diagnostic antibodies, antigens, peptides, adjuvants, antioxidants, cosmetic ingredients, therapeutic cells, diagnostic agents, radioactive tracers, contrast agents, neurotoxins, sensation modifying agents, and combinations thereof.

6. The composition of claims 1-5, wherein the composition further comprises about 0.1 wt. % to about 10 wt. % of an enzyme selected from the group consisting of hyaluronidase, elastase, or a combination thereof.

7. The composition of claims 1-6, wherein the composition further comprises pharmaceutically acceptable diluents, fillers, disintegrants, binders, lubricants, surfactants, hydrophobic vehicles, water soluble vehicles, emulsifiers, buffers, humectants, moisturizers, solubilizers, preservatives, colorants, plasticizers, carriers, excipients, and combinations thereof.

8. The composition of claims 1-7, wherein the composition is a paste, a gel, an ointment, a lotion, an emulsion, a cream, a foam, a mousse, a liquid, a spray, a suspension, a dispersion, a powder, or an aerosol.

9. A composition for use in therapy, comprising:
a mixture of two components dissolved, suspended, dispersed or otherwise mixed in a carrier or vehicle, the first component consisting of a decoy molecule consisting of an ECM component and/or fragment thereof and the second component comprising one or more active agents,
wherein the extracellular matrix component or fragment thereof has an average molecular weight of about 2,000 daltons to about 30,000 daltons and is present in the composition at between about 0.001 wt. % to about 10 wt. %.

10. The composition for use according to claim 9, wherein:
(a) the extracellular matrix component is selected from the group consisting of hyaluronicacid, collagen, fibronectin, elastin, lectin, and combinations thereof; and
(b) the composition comprises about 0.1 wt. % to about 25 wt. % of the active agent wherein the active agent is selected from the group consisting of small molecule drugs, macromolecular drugs, biologics, antibodies, chimeric antibodies, antibody fragments, diagnostic antibodies, antigens, peptides, adjuvants, antioxidants, cosmetic ingredients, therapeutic cells, diagnostic agents, radioactive tracers, contrast agents, neurotoxins, sensationmodifying agents, and combinations thereof;
(c) wherein the dosage of the active agent that is required to achieve a therapeutically effective amount is reduced.

11. A composition comprising:
a plurality of therapeutic cells expressing a tumor antigen; and
a decoy molecule consisting of an ECM component and/or a fragment thereof present in the composition at between about 0.1 wt. % to about 10 wt. %,
wherein said composition is for separate or simultaneous use in the treatment of cancer, and wherein the ECM component and/or fragment thereof has an average molecular weight of about 2,000 daltons to about 30,000 daltons.

12. A composition comprising:
a plurality of therapeutic cells expressing a tumor antigen; and
a decoy molecule consisting of an ECM component and/or fragments thereof having an average molecular weight of about 2,000 daltons to about 30,000 daltons
wherein the ECM component and/or fragments thereof are present in the composition at between about 0.1 wt.% to about 10 wt.% and
wherein said composition for use as claimed in claim 11, and
wherein the tumor antigen is selected from stomach tumor, colon tumor, prostate tumor, cervical tumor, skin tumor, uterine tumor, ovarian tumor, pancreatic tumor, kidney tumor, liver tumor, head and neck tumor, squamous cell tumor, gastrointestinal tumor, breast tumor, lung tumor, and brain tumor.

13. A composition comprising:
a mixture of two components dissolved, suspended, dispersed or otherwise mixed in a carrier or vehicle, the first component consisting of a decoy molecule consisting of an ECM component and/or a fragment thereof, and the second component comprising a neurotoxin agent;
wherein the decoy molecule is present in the composition at between about 0.001 wt.% to about 10 wt.% and has an average molecularweight of about 2,000 daltons to about 30,000 daltons, and
wherein the composition is for separate or simultaneous use in the treatment of symptoms of excessive sweating.

14. A composition comprising:
a mixture of two components dissolved, suspended, dispersed or otherwise mixed in a carrier or vehicle, the first component consisting of a decoy molecule consisting of an ECM component and/or a fragment thereof and the second component comprising a neurotoxic agent,
wherein the decoy molecule is present in the composition at between about 0.001 wt.% to about 10 wt.%, and
wherein the composition is for use as claimed in 13, and
wherein said neurotoxin agent is selected from the group consisting of botulinum toxin type A, botulinum toxin type B, botulinum toxin type C, botulinum toxin type D, botulinum toxin type E,botulinum toxin type F, abobotulinumtoxinA, BTX-A, daxibotulinumtoxinA, incobotulinumtoxinA, onabotulinumtoxinA, rimabotulinumtoxinB, syntaxin derivatives thereof, pharmaceutically acceptable salts thereof, and combinations thereof.

15. A transdermal patch comprising:
a mixture of two components dissolved, suspended, dispersed or otherwise mixed in a carrier or vehicle, the first component consisting of a decoy molecule consisting of an ECM component and/or a fragment thereof and the second component comprising an antibody or an antibody fragment,
wherein the decoy molecule (i) present in the composition at between about 0.001 wt.% to about 10 wt. % and has an average molecular weight of about 2,000 daltons to about 30,000 daltons,
optionally wherein an antigen recognized by the antibody or antibody fragment is a tumor antigen, a cancer antigen, an allergen, a bacterial antigen, a viral antigen, a drug, a hormone, a plant lectin, an endotoxin, or a combination thereof.

## Patentansprüche

1. Zusammensetzung, umfassend:
eine Mischung aus zwei Komponenten, die in einem Träger oder Vehikel gelöst, suspendiert, dispergiert oder anderweitig gemischt sind, wobei die erste Komponente aus einem Ködermolekül besteht, das aus einer Komponente der extrazellulären Matrix (ECM) und/oder einem Fragment davon besteht, und die zweite Komponente einen oder mehrere Wirkstoffe umfasst,
wobei die Komponente der extrazellulären Matrix oder das Fragment davon ein durchschnittliches Molekulargewicht von etwa 2.000 Dalton bis etwa 30.000 Dalton aufweist und in der Zusammensetzung zwischen etwa 0,001 Gew.- % bis etwa 10 Gew.- % vorliegt.

2. Zusammensetzung nach Anspruch 1, wobei die Komponente der extrazellulären Matrix aus der Gruppe ausgewählt ist, die aus Hyaluronsäure, Kollagen, Fibronectin, Elastin, Lectin und Fragmenten davon und Kombinationen davon besteht.

3. Zusammensetzung nach Anspruch 1 oder Anspruch 2, wobei die ECM-Komponente oder das Fragment davon Hyaluronsäure oder Elastin umfasst.

4. Zusammensetzung nach Anspruch 1-3, wobei die Zusammensetzung zwischen etwa 0,1 Gew.-% bis etwa 5 Gew.-% der Komponente der extrazellulären Matrix oder des Fragments davon umfasst.

5. Zusammensetzung nach den Ansprüchen 1-4, wobei der Wirkstoff aus der Gruppe ausgewählt ist, die aus niedermolekularen Arzneimitteln, makromolekularen Arzneimitteln, Biologika, Antikörpern, chimären Antikörpern, Antikörperfragmenten, diagnostischen Antikörpern, Antigenen, Peptiden, Adjuvanzien, Antioxidantien, kosmetischen Inhaltsstoffen, therapeutischen Zellen, diagnostischen Mitteln, radioaktiven Tracern, Kontrastmitteln, Neurotoxinen, wahrnehmungsmodifizierenden Mitteln und Kombinationen davon besteht.

6. Zusammensetzung nach den Ansprüchen 1-5, wobei die Zusammensetzung ferner etwa 0,1 Gew.-% bis etwa 10 Gew.-% eines Enzyms umfasst, das aus der Gruppe ausgewählt ist, die aus Hyaluronidase, Elastase oder einer Kombination davon besteht.

7. Zusammensetzung nach den Ansprüchen 1-6, wobei die Zusammensetzung ferner pharmazeutisch unbedenkliche Verdünnungsmittel, Füllstoffe, Sprengmittel, Bindemittel, Schmiermittel, Tenside, hydrophobe Vehikel, wasserlösliche Vehikel, Emulgatoren, Puffer, Feuchthaltemittel, Befeuchtungsmittel, Lösungsvermittler, Konservierungsmittel, Farbstoffe, Weichmacher, Träger, Hilfsstoffe und Kombinationen davon umfasst.

8. Zusammensetzung nach den Ansprüchen 1-7, wobei die Zusammensetzung eine Paste, ein Gel, eine Salbe, eine Lotion, eine Emulsion, eine Creme, ein Schaum, eine Mousse, eine Flüssigkeit, ein Spray, eine Suspension, eine Dispersion, ein Pulver oder ein Aerosol ist.

9. Zusammensetzung zur Verwendung in der Therapie, umfassend:
eine Mischung aus zwei Komponenten, die in einem Träger oder Vehikel gelöst, suspendiert, dispergiert oder anderweitig gemischt sind, wobei die erste Komponente aus einem Ködermolekül besteht, das aus einer ECM-Komponente und/oder einem Fragment davon besteht, und die zweite Komponente einen oder mehrere Wirkstoffe umfasst,
wobei die Komponente der extrazellulären Matrix oder das Fragment davon ein durchschnittliches Molekulargewicht von etwa 2.000 Dalton bis etwa 30.000 Dalton aufweist und in der Zusammensetzung mit zwischen etwa 0,001 Gew.- % bis etwa 10 Gew.- % vorliegt.

10. Zusammensetzung zur Verwendung nach Anspruch 9, wobei:
(a) die Komponente der extrazellulären Matrix aus der Gruppe ausgewählt ist, die aus Hyaluronsäure, Kollagen, Fibronectin, Elastin, Lectin und Kombinationen davon besteht; und
(b) die Zusammensetzung etwa 0,1 Gew.-% bis etwa 25 Gew.-% des Wirkstoffs umfasst, wobei der Wirkstoff aus der Gruppe ausgewählt ist, die aus niedermolekularen Arzneimitteln, makromolekularen Arzneimitteln, Biologika, Antikörpern, chimären Antikörpern, Antikörperfragmenten, diagnostischen Antikörpern, Antigenen, Peptiden, Adjuvanzien, Antioxidantien, kosmetischen Inhaltsstoffen, therapeutischen Zellen, diagnostischen Mitteln, radioaktiven Tracern, Kontrastmitteln, Neurotoxinen, wahrnehmungsmodifizierenden Mitteln und Kombinationen davon besteht;
(c) wobei die Dosierung des Wirkstoffs, die erforderlich ist, um eine therapeutisch wirksame Menge zu erreichen, reduziert ist.

11. Zusammensetzung, umfassend:
eine Vielzahl von therapeutischen Zellen, die ein Tumorantigen exprimieren; und
ein Ködermolekül, das aus einer ECM-Komponente und/oder einem Fragment davon besteht, die/das in der Zusammensetzung mit zwischen etwa 0,1 Gew.-% bis etwa 10 Gew.-% vorliegt,
wobei die Zusammensetzung zur separaten oder gleichzeitigen Verwendung bei der Behandlung von Krebs bestimmt ist und wobei die ECM-Komponente und/oder das Fragment davon ein durchschnittliches Molekulargewicht von etwa 2.000 Dalton bis etwa 30.000 Dalton aufweist.

12. Zusammensetzung, umfassend:
eine Vielzahl von therapeutischen Zellen, die ein Tumorantigen exprimieren; und
ein Ködermolekül, das aus einer ECM-Komponente und/oder Fragmenten davon besteht, die ein durchschnittliches Molekulargewicht von etwa 2.000 Dalton bis etwa 30.000 Dalton aufweist/aufweisen,
wobei die ECM-Komponente und/oder Fragmente davon in der Zusammensetzung mit zwischen etwa 0,1 Gew.-% bis etwa 10 Gew.-% vorliegen und
wobei die Zusammensetzung zur Verwendung nach Anspruch 11 und
wobei das Tumorantigen ausgewählt ist aus Magentumor, Dickdarmtumor, Prostatatumor, Zervixtumor, Hauttumor, Gebärmuttertumor, Eierstocktumor, Bauchspeicheldrüsentumor, Nierentumor, Lebertumor, Kopf- und Halstumor, Plattenepithelzelltumor, Magen-Darm-Tumor, Brusttumor, Lungentumor und Hirntumor.

13. Zusammensetzung, umfassend:
eine Mischung aus zwei Komponenten, die in einem Träger oder Vehikel gelöst, suspendiert, dispergiert oder anderweitig gemischt sind, wobei die erste Komponente aus einem Ködermolekül besteht, das aus einer ECM-Komponente und/oder einem Fragment davon besteht, und die zweite Komponente ein Neurotoxinmittel umfasst;
wobei das Ködermolekül in der Zusammensetzung mit zwischen etwa 0,001 Gew.-% bis etwa 10 Gew.-% vorliegt und ein durchschnittliches Molekulargewicht von etwa 2.000 Dalton bis etwa 30.000 Dalton aufweist, und
wobei die Zusammensetzung zur separaten oder gleichzeitigen Verwendung bei der Behandlung von Symptomen übermäßigen Schwitzens bestimmt ist.

14. Zusammensetzung, umfassend:
eine Mischung aus zwei Komponenten, die in einem Träger oder Vehikel gelöst, suspendiert, dispergiert oder anderweitig gemischt sind, wobei die erste Komponente aus einem Ködermolekül besteht, das aus einer ECM-Komponente und/oder einem Fragment davon besteht, und die zweite Komponente ein neurotoxisches Mittel umfasst,
wobei das Ködermolekül in der Zusammensetzung mit zwischen etwa 0,001 Gew.-% bis etwa 10 Gew.-% vorliegt, und
wobei die Zusammensetzung zur Verwendung nach 13 bestimmt ist, und
wobei das Neurotoxinmittel aus der Gruppe ausgewählt ist, die aus Botulinumtoxin Typ A, Botulinumtoxin Typ B, Botulinumtoxin Typ C, Botulinumtoxin Typ D, Botulinumtoxin Typ E, Botulinumtoxin Typ F, AbobotulinumtoxinA, BTX-A, DaxibotulinumtoxinA, IncobotulinumtoxinA, OnabotulinumtoxinA, RimabotulinumtoxinB, Syntaxinderivaten davon, pharmazeutisch unbedenklichen Salzen davon und Kombinationen davon besteht.

15. Transdermales Pflaster, umfassend:
eine Mischung aus zwei Komponenten, die in einem Träger oder Vehikel gelöst, suspendiert, dispergiert oder anderweitig gemischt sind, wobei die erste Komponente aus einem Ködermolekül besteht, das aus einer ECM-Komponente und/oder einem Fragment davon besteht, und die zweite Komponente einen Antikörper oder ein Antikörperfragment umfasst,
wobei das Ködermolekül (i) in der Zusammensetzung mit zwischen etwa 0,001 Gew.-% bis etwa 10 Gew.-% vorliegt und ein durchschnittliches Molekulargewicht von etwa 2.000 Dalton bis etwa 30.000 Dalton aufweist,
wobei optional ein durch den Antikörper oder das Antikörperfragment erkanntes Antigen ein Tumorantigen, ein Krebsantigen, ein Allergen, ein bakterielles Antigen, ein virales Antigen, ein Arzneimittel, ein Hormon, ein pflanzliches Lectin, ein Endotoxin oder eine Kombination davon ist.

## Revendications

1. Composition comprenant :
un mélange de deux composants dissous, en suspension, dispersés ou autrement mélangés dans un support ou un véhicule, le premier composant étant constitué d'une molécule leurre constituée d'un composant de matrice extracellulaire (MEC) et/ou d'un fragment de celui-ci, et le second composant étant constitué d'un ou plusieurs agents actifs,
dans laquelle le composant de matrice extracellulaire ou un fragment de celui-ci présente un poids moléculaire moyen d'environ 2 000 daltons à environ 30 000 daltons et est présent dans la composition à raison d'environ 0,001 % en poids à environ 10 % en poids.

2. Composition de la revendication 1, dans laquelle le composant de matrice extracellulaire est choisi dans le groupe constitué par l'acide hyaluronique, le collagène, la fibronectine, l'élastine, la lectine et des fragments de ceux-ci et des combinaisons de ceux-ci.

3. Composition de la revendication 1 ou la revendication 2, dans laquelle le composant de MEC ou un fragment de celui-ci comprend de l'acide hyaluronique ou de l'élastine.

4. Composition des revendications 1 à 3, dans laquelle la composition comprend entre environ 0,1 % en poids à environ 5 % en poids de composant de matrice extracellulaire ou de fragment de celui-ci.

5. Composition des revendications 1 à 4, dans laquelle l'agent actif est choisi dans le groupe constitué par des médicaments à petites molécules, des médicaments macromoléculaires, des produits biologiques, des anticorps, des anticorps chimériques, des fragments d'anticorps, des anticorps diagnostiques, des antigènes, des peptides, des adjuvants, des antioxydants, des ingrédients cosmétiques, des cellules thérapeutiques, des agents diagnostiques, des traceurs radioactifs, des agents de contraste, des neurotoxines, des agents modifiant les sensations et des combinaisons de ceux-ci.

6. Composition des revendications 1 à 5, dans laquelle la composition comprend en outre environ 0,1 % en poids à environ 10 % en poids d'une enzyme choisie dans le groupe constitué par l'hyaluronidase, l'élastase ou une combinaison de celles-ci.

7. Composition des revendications 1 à 6, dans laquelle la composition comprend en outre des diluants, des charges, des délitants, des liants, des lubrifiants, des tensioactifs, des véhicules hydrophobes, des véhicules hydrosolubles, des émulsifiants, des tampons, des humectants, des hydratants, des solubilisants, des conservateurs, des colorants, des plastifiants, des supports, des excipients acceptables pharmaceutiquement et des combinaisons de ceux-ci.

8. Composition des revendications 1 à 7, dans laquelle la composition est une pâte, un gel, une pommade, une lotion, une émulsion, une crème, une mousse (foam), une mousse (mousse), un liquide, un spray, une suspension, une dispersion, une poudre ou un aérosol.

9. Composition destinée à être utilisée en thérapie, comprenant :
un mélange de deux composants dissous, en suspension, dispersés ou autrement mélangés dans un support ou un véhicule, le premier composant étant constitué d'une molécule leurre constituée d'un composant de MEC et/ou d'un fragment de celui-ci et le second composant comprenant un ou plusieurs agents actifs,
dans laquelle le composant de matrice extracellulaire ou un fragment de celui-ci présente un poids moléculaire moyen d'environ 2 000 daltons à environ 30 000 daltons et est présent dans la composition à raison d'environ 0,001 % en poids à environ 10 % en poids.

10. Composition destinée à être utilisée selon la revendication 9, dans laquelle :
(a) le composant de matrice extracellulaire est choisi dans le groupe constitué par l'acide hyaluronique, le collagène, la fibronectine, l'élastine, la lectine et des combinaisons de ceux-ci ; et
(b) la composition comprend environ 0,1 % en poids à environ 25 % en poids de l'agent actif, dans laquelle l'agent actif est choisi dans le groupe constitué par des médicaments à petites molécules, des médicaments macromoléculaires, des produits biologiques, des anticorps, des anticorps chimériques, des fragments d'anticorps, des anticorps diagnostiques, des antigènes, des peptides, des adjuvants, des antioxydants, des ingrédients cosmétiques, des cellules thérapeutiques, des agents diagnostiques, des traceurs radioactifs, des agents de contraste, des neurotoxines, des agents modifiant les sensations et des combinaisons de ceux-ci ;
(c) dans laquelle la dose de l'agent actif nécessaire pour obtenir une quantité thérapeutiquement efficace est réduite.

11. Composition comprenant :
une pluralité de cellules thérapeutiques exprimant un antigène tumoral ; et
une molécule leurre constituée d'un composant de MEC et/ou d'un fragment de celui-ci présent dans la composition à raison d'environ 0,1 % en poids à environ 10 % en poids,
dans laquelle ladite composition est destinée à une utilisation séparée ou simultanée dans le traitement d'un cancer, et dans laquelle le composant de MEC et/ou un fragment de celui-ci présente un poids moléculaire moyen d'environ 2 000 daltons à environ 30 000 daltons.

12. Composition comprenant :
une pluralité de cellules thérapeutiques exprimant un antigène tumoral ; et
une molécule leurre constituée d'un composant de MEC et/ou de fragments de celui-ci présentant un poids moléculaire moyen d'environ 2 000 daltons à environ 30 000 daltons
dans laquelle le composant de MEC et/ou des fragments de celui-ci sont présents dans la composition à raison d'environ 0,1 % en poids à environ 10 % en poids, et
dans laquelle ladite composition est destinée à être utilisée selon la revendication 11, et
dans laquelle l'antigène tumoral est choisi parmi une tumeur de l'estomac, une tumeur du côlon, une tumeur de la prostate, une tumeur du col de l'utérus, une tumeur de la peau, une tumeur de l'utérus, une tumeur des ovaires, une tumeur du pancréas, une tumeur des reins, une tumeur du foie, une tumeur de la tête et du cou, une tumeur des cellules squameuses, une tumeur gastro-intestinale, une tumeur mammaire, une tumeur pulmonaire et une tumeur cérébrale.

13. Composition comprenant :
un mélange de deux composants dissous, en suspension, dispersés ou autrement mélangés dans un support ou un véhicule, le premier composant étant constitué d'une molécule leurre constituée d'un composant de MEC et/ou d'un fragment de celui-ci, et le second composant comprenant un agent neurotoxique ;
dans laquelle la molécule leurre est présente dans la composition à raison d'environ 0,001 % en poids à environ 10 % en poids et présente un poids moléculaire moyen d'environ 2 000 daltons à environ 30 000 daltons, et
dans laquelle la composition est destinée à une utilisation séparée ou simultanée dans le traitement des symptômes de transpiration excessive.

14. Composition comprenant :
un mélange de deux composants dissous, en suspension, dispersés ou autrement mélangés dans un support ou un véhicule, le premier composant étant constitué d'une molécule leurre constituée d'un composant de MEC et/ou d'un fragment de celui-ci et le second composant comprenant un agent neurotoxique,
dans laquelle la molécule leurre est présente dans la composition à raison d'environ 0,001 % en poids à environ 10 % en poids, et
dans laquelle la composition est destinée à être utilisée selon la revendication 13, et
dans laquelle l'agent neurotoxique est choisi dans le groupe constitué par la toxine botulique de type A, la toxine botulique de type B, la toxine botulique de type C, la toxine botulique de type D, la toxine botulique de type E, la toxine botulique de type F, l'abobotulinumtoxinA, BTX-A, la daxibotulinumtoxinA, l'incobotulinumtoxinA, l'onabotulinumtoxinA, la rimabotulinumtoxinB, des dérivés de syntaxine de ceux-ci, des sels acceptables pharmaceutiquement de ceux-ci et des combinaisons de ceux-ci.

15. Timbre transdermique comprenant :
un mélange de deux composants dissous, en suspension, dispersés ou autrement mélangés dans un support ou un véhicule, le premier composant étant constitué d'une molécule leurre constituée d'un composant de MEC et/ou d'un fragment de celui-ci et le second composant comprenant un anticorps ou un fragment d'anticorps,
dans lequel la molécule leurre (i) est présente dans la composition à raison d'environ 0,001 % en poids à environ 10 % en poids et présente un poids moléculaire moyen d'environ 2 000 daltons à environ 30 000 daltons,
éventuellement dans lequel un antigène reconnu par l'anticorps ou un fragment d'anticorps est un antigène tumoral, un antigène de cancer, un allergène, un antigène bactérien, un antigène viral, un médicament, une hormone, une lectine végétale, une endotoxine ou une combinaison de ceux-ci.
